# EUROPÄISCHE PATENTSCHRIFT

(11) **EP 2 400 961 B1**
(45) Veröffentlichungstag und Bekanntmachung des Hinweises auf die Patenterteilung: **22.11.2017**
(21) Anmeldenummer: 10704367.1
(22) Anmeldetag: 18.02.2010
(51) Int. Cl.: A61K 31/196, A61K 31/44, A61K 31/519, A61K 45/06, A61P 1/00, A61P 11/00, A61P 17/00, A61P 19/00, A61P 25/00, A61P 29/00, A61P 35/00, A61K 31/136, A61K 31/55

(54) **ARZNEIMITTELKOMBINATIONEN ENTHALTEND PDE4-INHIBITOREN UND NSAIDS**
MEDICINE COMBINATIONS CONTAINING PDE4 INHIBITORS AND NSAIDS
COMBINAISONS DE MÉDICAMENTS CONTENANT DES INHIBITEURS PDE4 ET NSAID

(30) Priorität: 27.02.2009 EP 09153853; 22.07.2009 EP 09166127
(43) Veröffentlichungstag der Anmeldung: 04.01.2012
(73) Patentinhaber: Boehringer Ingelheim International GmbH, 55216 Ingelheim am Rhein (DE)
(72) Erfinder: NICKOLAUS, Peter, 55216 Ingelheim Am Rhein (DE); GOEGGEL, Rolf, 55216 Ingelheim Am Rhein (DE); PETER, Daniel, 55216 Ingelheim Am Rhein (DE)
(74) Vertreter: Simon, Elke Anna Maria
(86) Internationale Anmeldenummer: PCT/EP2010/052077
(87) Internationale Veröffentlichungsnummer: WO 2010/097332

(56) Entgegenhaltungen:
- WO-A-03/024489
- WO-A-2006/111549
- WO-A-2007/118793
- WO-A-2009/050242
- WO-A-2009/053268
- SAULIUS PASKAUSKAS ET AL: "Blockade of leukocyte haptokinesis and haptotaxis by ketoprofen, diclofenac and SC-560", BMC IMMUNOLOGY, BIOMED CENTRAL, LONDON, GB, vol. 12, no. 1, 64, 12 November 2011 (2011-11-12), pages 1-9, XP021111664, ISSN: 1471-2172, DOI: 10.1186/1471-2172-12-64
- D Spina: "REVIEW PDE4 inhibitors: current status", British Journal of Pharmacology, vol. 155, 1 January 2008 (2008-01-01), pages 308-315, XP055254077,
- "6 Unwanted Side Effects" In: "Handbook of Experimental Pharmacology", 1 January 2011 (2011-01-01), XP055254088, vol. 204, pages 391-392,

## Beschreibung

Die vorliegende Erfindung betrifft neue Arzneimittelkombinationen, die neben einem oder mehreren PDE4-Inhibitoren wenigstens ein NSAID (= non-steroidal anti-inflammatory drug) **(2)** umfassen, Verfahren zu deren Herstellung sowie deren Verwendung zur Therapie von insbesondere Atemwegserkrankungen wie beispielsweise COPD, chronische Sinusitis und Asthma.

Die Erfindung betrifft Arzneimittelkombinationen, die neben einem oder mehreren, bevorzugt einem PDE4-Inhibitor der allgemeinen Formel **1** 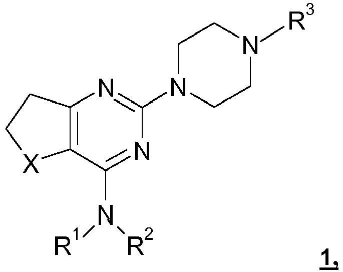
worin **X** SO ist und worin
R³ für einen gegebenenfalls substituierten, mono- oder bicyclischen, ungesättigten, teilweise gesättigten oder gesättigten Heterocyclus oder für ein gegebenenfalls substituiertes, mono-oder bicyclisches Heteroaryl steht
und worin R¹ und R² die in Anspruch 1 genannten Bedeutungen haben,
   wenigstens ein NSAID **(2)** (= non-steroidal anti-inflammatory drug) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfassen, deren Herstellung und deren Verwendung zur Therapie von Atemwegserkrankungen.

### STAND DER TECHNIK

WO 2003/024489 offenbart Kombinationen von PDE4-Inhibitoren, insbesondere Roflumilast, mit NSAIDs, insbesondere Diclofenac. Dennoch sind die in WO 2003/024489 genannten PDE4-Inhibitoren strukturell völlig unterschiedlich zu den erfindungsgemäßen Dihydrothienopyrimidinen der Formel 1.

WO 2006/111549 offenbart Dihydrothienopyrimidine als PDE4-Inhibitoren, sowie Kombinationen derer mit anderen Wirkstoffen wie beispielsweise MRP4-Inhibitoren wie Diclofenac.

Die EP 07118911.2 offenbart Heterocyclus-substituierte Piperazino Dihydrothienopyrimidine der Formel 1 als PDE4-Inhibitoren, deren Herstellung sowie deren Verwendung zur Therapie von Atemwegserkrankungen.

Weiterhin ist bekannt, dass viele PDE4-Inhibitoren der "1. Generation" wie beispielsweise Rolipram zu unerwünschten Nebenwirkungen führen. Es war folglich Aufgabe der vorliegenden Erfindung, ein Arzneimittel bzw. eine Arzneimittelkombination enthaltend einen PDE4-Inhibitor bereitzustellen, welches über ein geringes Nebenwirkungsprofil verfügt.

### BESCHREIBUNG DER ERFINDUNG

Überraschenderweise konnte nun gefunden werden, dass Arzneimittelkombinationen die neben einem PDE4-Inhibitor - insbesondere neben den als PDE4-Inhibitoren bekannten Dihydrothienopyrimidinsulfoxiden der Formel **1**, in denen R³ für einen mono- oder bicyclischen, ungesättigten, teilweise gesättigten oder gesättigten Heterocyclus oder für ein mono- oder bicyclisches Heteroaryl steht (1) - auch wenigstens ein NSAID **(2)** enthalten, ein deutlich verringertes PDE4-vermitteltes Nebenwirkungsprofil aufweisen gegenüber einem Arzneimittel, das den entsprechenden PDE4-Inhibitor bzw. das entsprechende Dihydrothienopyrimidinsulfoxid der Formel **1** alleine enthält. Infolgedessen kann die Dosierung des entsprechenden Dihydrothienopyrimidinsulfoxids der Formel **1** (als PDE4-Inhibitor) deutlich höher ausfallen, wodurch sich dessen Wirksamkeit zur Therapie von beispielsweise Atemwegserkrankungen wie insbesondere COPD, chronischer Sinusitis und Asthma bei gleichzeitig niedrig bleibendem Nebenwirkungsprofil erhöht. Dies bedeutet demnach, dass sich das therapeutische Fenster für den verwendeten PDE4-Inhibitor vergrößert.

Die vorliegende Erfindung betrifft daher eine neuartige Arzneimittelkombination, die neben einem oder mehreren PDE4-Inhibitoren wenigstens ein NSAID (=non-steroidal anti-Inflammatory drug) **(2)** gemäß den Ansprüchen umfasst.

Die vorliegende Erfindung betrifft Arzneimittelkombinationen, die neben einer Verbindung der allgemeinen Formel **1** als PDE4-Inhibitor 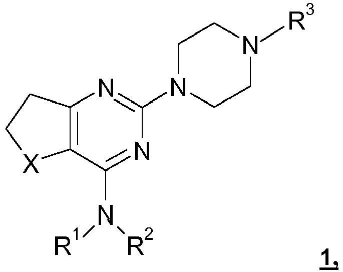 worin
- **X**: SO,
- **R¹**: H
- **R²**: H ist oder ein Rest ausgewählt aus der Gruppe bestehend aus C₁₋₁₀-Alkyl und C₂₋₆-Alkenyl ist, der gegebenenfalls durch einen oder mehrere Reste ausgewählt aus Halogen und C₁₋₃-Fluoroalkyl substituiert sein kann oder der gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, C₆₋₁₀-Aryl, einem Het, einem Hetaryl, einem mono- oder bicyclischem C₃₋₁₀-Cycloalkyl, CH₂-NR²²R²³ und NR^{2.2}R^{2.3} substituiert sein kann, welcher wiederum gegebenenfalls durch einen oder mehrere Reste ausgewählt aus der Gruppe bestehend aus OH, Halogen, OR^{2.1}, Oxo, CF₃, CHF₂, CH₂F, C₁₋₆-Alkyl, C₁₋₆-Alkanol, C₆₋₁₀-Aryl, COOR^{2.1}, CH₂-NR^{2.2}R^{2.3} und NR^{2.2}R^{2.3} substituiert sein kann,
wobei **R^{2.1}** H ist oder ein Rest ausgewählt ist aus der Gruppe bestehend aus C₁₋₆-Alkyl, C₁₋₆-Alkanol, C₁₋₃-Haloalkyl, mono- oder bicyclisches C₃₋₁₀-Cycloalkyl, C₆₋₁₀-Aryl-C₁₋₆-alkylen, mono- oder bicyclisches Hetaryl-C₁₋₆-alkylen, Het-C₁₋₆-alkylen, C₃₋₁₀-Cycloalkyl-C₁₋₆-alkylen, ein mono- oder bicyclisches C₆₋₁₀-Aryl, ein Hetaryl und ein Het,
der gegebenenfalls durch einen oder mehrere Reste ausgewählt aus der Gruppe bestehend aus OH, O-(C₁₋₃-Alkyl), Halogen, C₁₋₆-Alkyl und C₆₋₁₀-Aryl substituiert sein kann,
wobei **R^{2.2}** und **R^{2.3}** unabhängig voneinander H sind oder ein Rest ausgewählt sind aus der Gruppe bestehend aus C₁₋₆-Alkyl, mono- oder bicyclisches C₃₋₁₀ Cycloalkyl, C₆₋₁₀-Aryl-C₁₋₆-alkylen, Hetaryl-C₁₋₆-alkylen, mono- oder bicyclisches C₆₋₁₀-Aryl, Het, Hetaryl, CO-NH₂, CO-NHCH₃, CO-N(CH₃)₂, SO₂-(C₁-C₂-Alkyl), CO-R^{2.1} und COOR^{2.1},
der gegebenenfalls durch einen oder mehrere Reste ausgewählt aus der Gruppe bestehend aus OH, Halogen, C₁₋₆-Alkyl, C₆₋₁₀-Aryl und COOR^{2.1} substituiert sein kann,
wobei
- **Het**: ein drei- bis elfgliedriger, mono- oder bicyclischer, gesättigter oder teilweise gesättigter, gegebenenfalls annellierter oder gegebenenfalls überbrückter Heterocyclus ist, der 1, 2, 3 oder 4 Heteroatome unabhängig voneinander ausgewählt aus der Gruppe bestehend aus N, S oder O enthält, und wobei
- **Hetaryl**: ein fünf- bis elfgliedriger, mono- oder bicyclisches, gegebenenfalls annelliertes Heteroaryl ist, das 1, 2, 3 oder 4 Heteroatome unabhängig voneinander ausgewählt aus der Gruppe bestehend aus N, S oder O enthält,
und wobei
- **Cycloalkyl**: gesättigt oder teilweise gesättigt sein kann,
oder
- **R²**: ein mono- oder polycyclisches C₃₋₁₀ Cycloalkyl, das gegebenenfalls einfach oder mehrfach über C₁₋₃-Alkylgruppen verbrückt sein kann und das gegebenenfalls mit einem Rest ausgewählt aus der Gruppe bestehend aus verzweigtes oder unverzweigtes C₁₋₆-Alkanol, C₁₋₃-Fluoroalkyl, C₁₋₃-alkylen-OR^{2.1}, OR^{2.1}, Color^{2.1}, SO₂-NR^{2.2}R^{2.3}, Het, C₆₋₁₀-Aryl, C₁₋₆-Alkyl, C₆₋₁₀-Aryl-C₁₋₆-alkylen, Hetaryl-C₁₋₆-alkylen, mono- oder bicyclisches C₃₋₁₀ Cycloalkyl und NR^{2.2}R^{2.3} substituiert sein kann, der gegebenenfalls durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder
- **R²**: ein mono- oder polycyclisches C₆₋₁₀-Aryl, das gegebenenfalls durch OH, SH oder Halogen oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₁₀-Cycloalkyl, Het, -C₁₋₆-Alkyl, C₁₋₃-Fluoroalkyl, C₆₋₁₀-Aryl-C₁₋₆-alkylen, Het-C₁₋₆-alkylen, Hetaryl-C₁₋₆-alkylen, C₆₋₁₀-Aryl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, CF₃, CHF₂, CH₂F, Oxo, Halogen, CF₃, CHF₂, CH₂F, C₁₋₆-Alkyl, C₆₋₁₀-Aryl und NR^{2.2}R^{2.3} substituiert sein kann, oder
- **R²**: ein Rest ausgewählt aus einer Gruppe bestehend aus Het und Hetaryl, welcher gegebenenfalls durch einen oder mehrere Reste ausgewählt aus der Gruppe Halogen, OH, Oxo, CF₃, CHF₂ und CH₂F oder durch einen oder mehrere Reste ausgewählt aus der Gruppe OR^{2.1}, C₁₋₃-alkylen-OR^{2.1}, SR^{2.1},SO-R^{2.1} und
SO₂-R^{2.1}, COOR^{2.1}, COR^{2.1}, C₁₋₆-Alkanol, C₃₋₁₀-Cycloalkyl, C₆₋₁₀-Aryl, C₁₋₆-Alkyl, C₆₋₁₀-Aryl-C₁₋₆-alkylen, Hetaryl-C₁₋₆-alkylen, Het,
Hetaryl, C₁₋₃-alkylen-OR^{2.1} und NR^{2.2}R^{2.3} substituiert sein kann, der gegebenenfalls wiederum durch einen oder mehrere Reste ausgewählt aus der Gruppe bestehend aus OH, OR^{2.1}, Oxo, Halogen, CF₃, CHF₂, CH₂F, C₁₋₆-Alkyl, C₆₋₁₀-Aryl und NR^{2.2}R^{2.3} substituiert sein kann,
oder worin
- **NR¹R²**: gemeinsam einen heterocyclischen vier- bis siebengliedrigen Ring bedeutet, der gegebenenfalls überbrückt sein kann, der 1, 2 oder 3 Heteroatome ausgewählt aus der Gruppe bestehend aus N, O und S enthält und der gegebenenfalls durch einen oder mehrere Reste ausgewählt aus der Gruppe bestehend aus OH, OR^{2.1}, C₁₋₃-alkylen-O^{R.1}, Oxo, Halogen, C₁₋₆-Alkyl, C₆₋₁₀-Aryl, COOR^{2.1}, CH₂-NR^{2.2}-COO-R^{2.}1, CH₂-NR^{2.2}-CO-R^{2.}1, CH₂-NR^{2.2}-CO-CH₂-NR^{2.2}R^{2.3}, CH₂-NR^{2.2}-SO₂-C₁₋₃-Alkyl, CH₂-NR^{2.2}-SO₂-NR^{2.2}R^{2.3}, CH₂-NR^{2.2}-CO-NR^{2.2}R^{2.3}, CO-NR^{2.2}R^{2.3}, CH₂-NR^{2.2}R^{2.3} und NR^{2.2}R^{2.3} substituiert sein kann,
und worin
- **R³**: ein Rest ausgewählt aus der Gruppe bestehend aus einem Het und einem Hetaryl ist, der gegebenenfalls durch einen oder mehrere Reste ausgewählt aus der Gruppe bestehend aus
Halogen, C₁₋₃-Fluoroalkyl, CN, OH, Oxo, -C₁₋₆-Alkyl, -O-R^{2.1}, -COOR^{2.1}, SO-R^{2.1}, SO₂-R^{2.1}, C₆₋₁₀-Aryl, C₁₋₃-alkylen-C₆₋₁₀-Aryl, -C₁₋₃-alkylen-NR^{2.2}R^{2.3}, -NR^{2.2}R^{2.3}, einem C₃₋₁₀-Cycloalkyl, einem C₁₋₃-alkylen-C₃₋₁₀-Cycloalkyl, einem Het, einem Hetaryl, C₁₋₃-alkylen-Hetaryl, und C₁₋₃-alkylen-Het substituiert sein kann, die wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁₋₃-Alkyl) und O-(C₁₋₃-Alkyl) substituiert sein können,
bedeuten, wenigstens ein NSAID (= non-steroidal anti-inflammatory drug) **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten. Anspruchsgemäß sind Arzneimittelkombinationen, die neben einer Verbindung der allgemeinen Formel **1** als PDE4-Inhibitor, worin
- **X**: SO,
- **R¹**: H
- **R²**: H ist oder C₁₋₆-Alkyl ist, das gegebenenfalls durch einen oder mehrere Reste ausgewählt aus F, CF₃, CHF₂ oder CH₂F substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, Phenyl, einem Het, einem Hetaryl, einem monocyclischem C₃₋₇-Cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, F, Cl, Br, CF₃, CHF₂, CH₂F, OR^{2.1}, Oxo, Methyl, Ethyl, Propyl, Isopropyl, C₁₋₂-Alkanol, Phenyl, COOR^{2.1}, CH₂-NR^{2.2}R^{2.3} und NR^{2.2}R^{2.3} substituiert sein kann,
wobei **R^{2.1}** H ist oder ein Rest ausgewählt ist aus der Gruppe bestehend aus Methyl, Ethyl, Propyl, Isopropyl, monocyclisches C₃₋₇ Cycloalkyl, Phenyl-C₁₋₂-alkylen, Hetaryl-C₁₋₂-alkylen,
Het-C₁₋₂-alkylen, C₃₋₇-Cycloalkyl-C₁₋₂-alkylen, Phenyl, ein Hetaryl und ein Het, der gegebenenfalls durch einen oder mehrere Reste ausgewählt aus der Gruppe bestehend aus OH, Halogen, Methyl, Ethyl, Propyl, Isopropyl, O-Methyl, O-Ethyl, O-Propyl, O-Isopropyl und Phenyl substituiert sein kann,
wobei **R^{2.2}** und **R^{2.3}** unabhängig voneinander H oder ein Rest ausgewählt sind aus der Gruppe bestehend aus Methyl, Ethyl, Propyl, Isopropyl, monocyclisches C₃₋₇ Cycloalkyl, Phenyl-C₁₋₃-alkylen, Hetaryl-C₁₋₃-alkylen, Phenyl, Het, Hetaryl, CO-NH₂, CO-NHCH₃, CON(CH₃)₂, SO₂-(C₁-C₂-Alkyl), CO-R^{2.1} und COOR^{2.1},
der gegebenenfalls durch einen oder mehrere Reste ausgewählt aus der Gruppe bestehend aus OH, F, Cl, Br, Methyl, Ethyl, Propyl, Isopropyl, Phenyl und COOR^{2.1} substituiert sein kann,
wobei
- **Het**: ein drei- bis siebengliedriger, monocyclischer, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
- **Hetaryl**: ein fünf- bis sechsgliedriger, monocyclisches, aromatisches Heteroaryl ist, das 1, 2 oder 3 Heteroatome unabhängig voneinander ausgewählt aus der Gruppe bestehend aus N, S oder O enthält,
und wobei
- **Cycloalkyl**: gesättigt oder teilweise gesättigt sein kann,
oder
- **R²**: ein monocyclisches C₃₋₇ Cycloalkyl, das gegebenenfalls mit einem Rest ausgewählt aus der Gruppe bestehend aus verzweigtes oder unverzweigtes C₁₋₂-Alkanol, C₁₋₃-Fluoroalkyl, C₁₋₃-alkylen-OR^{2.1}, OR^{2.1}, COOR^{2.1}, SO₂-NR^{2.2}R^{2.3},
Het, Methyl, Ethyl, Propyl, Isopropyl, Phenyl , Phenyl-C₁₋₂-alkylen, Hetaryl-C₁₋₂-alkylen, monocyclisches C₃₋₇ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, Halogen, CF₃, CHF₂, CH₂F, Methyl, Ethyl, Propyl, Isopropyl, Phenyl und NR^{2.2}R^{2.3} substituiert sein kann,
oder
- **R²**: ein Phenyl, das gegebenenfalls durch OH, SH, F, Cl oder Br oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₇-Cycloalkyl, Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋₂-alkylen, Phenyl, SO₂-CH₃, SO₂-CH₂CH₃ und SO₂-NR^{2.2}R^{2.3} substituiert sein kann, der wiederum gegebenenfalls durch einen oder mehrere oder mehrere Reste ausgewählt aus der Gruppe bestehend aus OH, OR^{2.1}, Oxo, F, Cl, Br, CF₃, CHF₂, CH₂F, Methyl, Ethyl, Propyl, Isopropyl, Phenyl und NR^{2.2}R^{2.3} substituiert sein kann,
oder
- **R²**: ein Rest ausgewählt aus einer Gruppe bestehend aus einem Het und einem Hetaryl, welcher gegebenenfalls durch einen oder mehrere Reste ausgewählt aus der Gruppe F, Cl, Br, OH, Oxo, CF₃, CHF₂, CH₂F und SH oder durch einen oder mehrere Reste ausgewählt aus der Gruppe OR^{2.1}, C₁₋₃-alkylen-OR^{2.}1, SR^{2.1},SO-R^{2.1}, SO₂-R^{2.1}, COOR^{2.1}, COR^{2.}1,C₁₋₂-Alkanol, C₃₋₁₀-Cycloalkyl, Phenyl, Methyl, Ethyl, Propyl, Isopropyl, Phenyl-C₁₋₂-alkylen, Hetaryl-C₁₋₂-alkylen, Het, Hetaryl, C₁₋₂-Alkanol und NR^{2.2}R^{2.3} substituiert sein kann,
der gegebenenfalls wiederum durch einen oder mehrere Reste ausgewählt aus der Gruppe bestehend aus OH, OR^{2.1}, Oxo, F, Cl, Br, CF₃, CHF₂, CH₂F, C₁₋₆-Alkyl, Phenyl und NR^{2.2}R^{2.3} substituiert sein kann,
und worin
- **R³**: ein Rest ausgewählt aus der Gruppe bestehend aus einem gesättigten oder teilweise gesättigten, monocyclischem drei- bis siebengliedrigen Heterocyclus, einem gesättigten oder teilweise gesättigten, bicyclischem fünf- bis elfgliedrigem Heterocyclus, einem monocyclischen, fünf- bis sechsgliedrigem Heteroaryl und einem bicyclischem, sieben- bis elfgliedrigem Heteroaryl ist,
der jeweils 1, 2, 3 oder 4 Heteroatome unabhängig voneinander ausgewählt aus der Gruppe bestehend aus N, O und S enthält
und der jeweils gegebenenfalls durch einen oder mehrere Reste ausgewählt aus der Gruppe bestehend aus
Halogen, C₁₋₃-Fluoroalkyl, CN, OH, Oxo, -C₁₋₆-Alkyl, -O-R^{2.1}, -COOR^{2.1}, SO-R^{2.1}, SO₂-R^{2.1}, C₆₋₁₀-Aryl, C₁₋₃-alkylen-C₆₋₁₀-Aryl, -C₁₋₃-alkylen-NR^{2.2}R^{2.3}, -NR^{2.2}R^{2.3}, einem C₃₋₁₀-Cycloalkyl, einem C₁₋₃-alkylen-C₃₋₁₀-Cycloalkyl, Het, einem Hetaryl, C₁₋₃-alkylen-Hetaryl, und C₁₋₃-alkylen-Het substituiert sein kann,
die wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁₋₃-Alkyl) und O-(C₁₋₃-Alkyl) substituiert sein kann,
bedeuten,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten. Bevorzugt sind Arzneimittelkombinationen, die neben einer Verbindung der allgemeinen Formel **1** als PDE4-Inhibitor, worin
- **R²**: ein Rest nach Formel **3** 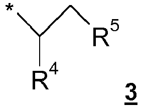
ist,
- worin **R⁵**: OH oder NH₂ ist und
- worin **R⁴**: ein Rest ausgewählt aus der Gruppe bestehend aus C₁₋₄-Alkyl, Hetaryl und Phenyl,
der gegebenenfalls mit einem oder mehreren Resten ausgewählt aus der Gruppe bestehend aus OH, F, Br, OR^{2.1}, Oxo, Methyl, Ethyl, C₁₋₂-Alkanol, Phenyl, COOR^{2.1}, CH₂-NR^{2.2}R^{2.3} und NR^{2.2}R^{2.3} substituiert sein kann,
bedeuten,
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R²**: ein Rest nach Formel **3** 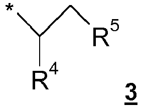
ist,
- worin **R⁵**: OH oder NH₂ ist und
- worin **R⁴**: Methyl, Ethyl, Propyl, Isopropyl
bedeuten
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R²**: ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂, CH₂F und C₂₋₄-Fluoroalkyl substituiert sein kann, wobei
- R^{2.1}: ausgewählt ist aus der Gruppe bestehend aus Methyl, Ethyl, Propyl, Isopropyl, Butyl, Isobutyl,
bedeuten,
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R²**: ein Phenyl ist, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂ und N(CH₃)₂ substituiert ist, wobei R^{2.1} H, Methyl oder Ethyl sein kann,
bedeuten
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R²**: ein monocyclischer, gesättigter drei-, vier-, fünf-, sechs- oder siebengliedriger Heterocyclus mit 1, 2 oder 3 Heteroatomen jeweils ausgewählt aus der Gruppe bestehend aus N, O und S ist, der gegebenenfalls durch einen oder mehrere Reste ausgewählt aus der Gruppe bestehend aus Fluor, Chlor, Brom, CF₃, CHF₂, CH₂F, OH, Oxo und SH oder durch einen oder mehrere Reste ausgewählt aus der Gruppe bestehend aus OR^{2.1}, C₁₋₃-alkylen-OR^{2.1}, SR^{2.1},SO-R^{2.1}, SO₂-R^{2.1}, COOR^{2.}1, COR^{2.1}, C₁₋₆-Alkanol, C₃₋₁₀-Cycloalkyl, Phenyl, C₁₋₆-Alkyl, Phenyl-C₁₋₆-alkylen, Hetaryl-C₁₋₆-alkylen, Het, Hetaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
bedeuten
wobei **R^{2.1}, R^{2.2}** und **R^{2.3}** und die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel 1 als PDE4-Inhibitor, worin
- **R²**: ein monocyclischer, gesättigter, sechsgliedriger Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃, N(CH₃)₂, Methyl, Ethyl, Propyl, Isopropyl, Cyclopropyl, Methoxy und Ethoxy substituiert sein kann,
bedeuten
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R²**: ein Rest ausgewählt aus einer Gruppe bestehend aus Piperidin oder Tetrahydropyran, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
und worin die übrigen Reste wie vorstehend definiert sind,
bedeuten,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R³**: monocyclischer fünf- oder sechsgliedriger Heteroarylring ist, der gegebenenfalls durch einen oder mehrere Reste ausgewählt aus der Gruppe bestehend aus F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO-(CH₃), SO₂-(CH₂CH₃), SO-(CH₂CH₃), Phenyl, -methylen-Phenyl, -ethylen-Phenyl, -NH₂, -NH(CH₃), N(CH₃)₂, -methylen-NH₂, -methylen-NH(CH₃), -methylen-N(CH₃)₂, einem C₃₋₆-Cycloalkyl, einem methylen-C₃₋₆-Cycloalkyl, einem gesättigtem oder teilweise gesättigtem, fünf- bis sechsgliedrigem Heterocyclus, einem fünf- oder sechsgliedrigem Heteroaryl, -methylen-Hetaryl, und -methylen-Het substituiert sein kann, die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl, -COO(CH₃), -O-Methyl und -O-Ethyl substituiert sein können,
bedeuten,
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel 1 als PDE4-Inhibitor, worin
- **R³**: ein bicyclischer 9- bis 11-gliedriger gesättigter, ungesättigter oder teilweise gesättigter Heterocyclus ist, der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO-(CH₃), SO₂-(CH₂CH₃), SO-(CH₂CH₃), Phenyl, -methylen-Phenyl, -ethylen-Phenyl, -NH2, -NH(CH₃), N(CH₃)₂, -methylen-NH₂, -methylen-NH(CH₃), -methylen-N(CH₃)₂, einem -C₃₋₆-Cycloalkyl, einem -methylen-C₃₋₆-Cycloalkyl, einem gesättigtem, teilweise ungesättigtem oder ungesättigtem 5-bis 6-gliedriger Heterocyclus, einem 5-bis 6-gliedrigem Heteroaryl, ,-methylen-Hetaryl, und -methylen-Het substituiert sein kann, die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl,-COO(CH₃), -O-Methyl und -O-Ethyl substituiert sein können,
bedeuten,
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R³**: ein monocyclischer fünf- oder sechsgliedriger Heteroarylring ausgewählt aus der Gruppe bestehend aus Pyrrol, Pyrazol, Furan, Thiophen, Thiazol, Imidazol, Oxazol, Pyridazin, Pyrimidin, Pyrazin, Thiadiazol, Oxadiazol, Triazin, Isooxazol, Isothiazol und Pyridin ist, der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO₂-(CH₂CH₃), Phenyl, -methylen-Phenyl, -ethylen-Phenyl, -NH₂, -NH(CH₃), N(CH₃)₂, -methylen-NH₂, -methylen-NH(CH₃), -methylen-N(CH₃)₂, einem C₃₋₆-Cycloalkyl, einem methylen-C₃₋₆-Cycloalkyl, einem Het, einem Hetaryl,-methylen-Hetaryl, und -methylen-Het substituiert sein kann,
die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl,-COO(CH₃), -O-Methyl und -O-Ethyl substituiert sein können,
bedeuten,
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor, worin
- **R³**: bicyclischer 9- bis 11-gliedriger Heterocyclus ausgewählt aus der Gruppe bestehend aus Benzoxazol, Benzodioxol, Dihydrobenzodioxin, Benzodioxin, Benzisoxazol, Benzothiazol, Benzisothiazol, Thienopyrimidin, Furopyrimidin, Thienopyridin, Furopyridin, Indol, Isoindol, Chinoxalin, Naphthyridin, Pyridopyrazin, Pyridopyrimidin, Chinolin, Isochinolin, Benzoimidazol, 6,7,8,9-Tetrahydro-5H-pyrazino[2,3-d]azepin, Benzothiophen, Benzofuran, Chinazolin, Indazol, Isobenzofuran und Pteridin ist,
der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO₂-(CH₂CH₃), Phenyl, -methylen-Phenyl, -ethylen-Phenyl, -NH₂, -NH(CH₃), N(CH₃)₂, -methylen-NH₂, -methylen-NH(CH₃), -methylen-N(CH₃)₂, einem C₃₋₆-Cycloalkyl, einem methylen-C₃₋₆-Cycloalkyl, einem Het, einem Hetaryl, ,-methylen-Hetaryl, und -methylen-Het substituiert sein kann,
die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl,-COO(CH₃), -O-Methyl und -O-Ethyl substituiert sein können,
bedeuten,
und worin die übrigen Reste wie vorstehend definiert sind,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor,
worin
- **R¹**: H ist,
- **R²**: ein Rest ausgewählt ist aus der Gruppe bestehend aus 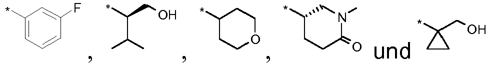 ist,
- **R³**: ein Rest ausgewählt ist aus der Gruppe bestehend aus 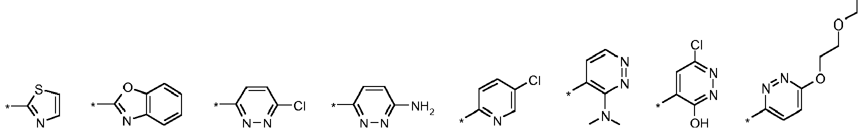 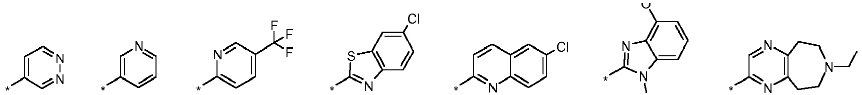 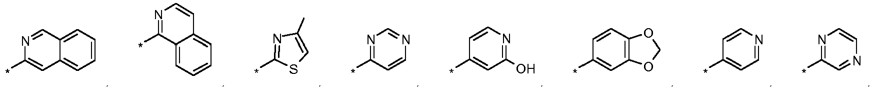 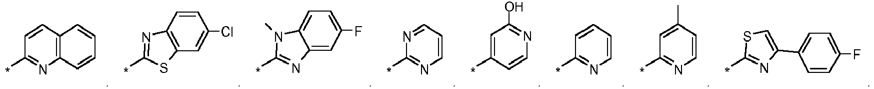 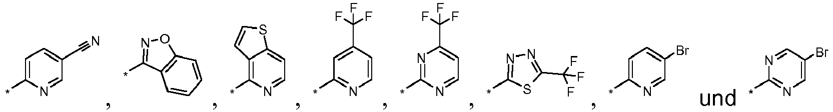 ist,
bedeuten,
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Weiterhin bevorzugt sind Arzneimittelkombinationen, die neben einer Verbindung der allgemeinen Formel **1** als PDE4-Inhibitor ausgewählt aus der Gruppe bestehend aus:
1.1. (3-Fluorphenyl)-[5-oxo-2-(4-thiazol-2-yl-piperazin-1-yl)-6,7-dihydro-5*H*-5λ⁴-thieno[3,2-*d*]pyrimidin-4-yl]-amin
1.2. (R)-3-Methyl-2-[5-oxo-2-(4-thiazol-2-yl-piperazin-1-yl)-6,7-dihydro-5*H*-5λ⁴-thieno[3,2-*d*]pyrimidin-4-ylamino]-butan-1-ol
1.3. [2-(4-Benzoxazol-2-yl-piperazin-1-yl)-5-oxo-6,7-dihydro-5*H*-5λ□⁴-thieno[3,2-*d*]pyrimidin-4-yl]-(3-fluorphenyl)-amin
1.4.[2-(4-Benzoxazol-2-yl-piperazin-1-yl)-5-oxo-6,7-dihydro-5*H*-5λ⁴-thieno[3,2-*d*]pyrimidin-4-yl]-(tetrahydropyran-4-yl)-amin
1.5. (R)-2-{2-[4-(6-Chlorpyridazin-3-yl)-piperazin-1-yl]-5-oxo-6,7-dihydro-5*H*-5λ⁴-thieno[3,2-*d*]pyrimidin-4-ylamino}-3-methylbutan-1-ol
1.6. {2-[4-(6-Chlorpyridazin-3-yl)-piperazin-1-yl]-5-oxo-6,7-dihydro-5*H*-5λ⁴-thieno[3,2-*d*]pyrimidin-4-yl}-(3-fluorphenyl)-amin
1.7. (R)-2-[2-(4-Benzoxazol-2-yl-piperazin-1-yl)-5-oxo-6,7-dihydro-5*H*-5λ⁴-thieno[3,2-*d*]pyrimidin-4-ylamino]-3-methylbutan-1-ol
1.8. (1-{2-[4-(5-Chlorpyridin-2-yl)-piperazin-1-yl]-5-oxo-6,7-dihydro-5*H*-5λ⁴-thieno[3,2-*d*]pyrimidin-4-ylamino}-cyclopropyl)-methanol
1.9. {2-[4-(5-Chlorpyridin-2-yl)-piperazin-1-yl]-5-oxo-6,7-dihydro-5*H*-5λ⁴-thieno[3,2-*d*]pyrimidin-4-yl}-(tetrahydropyran-4-yl)-amin
1.10. {2-[4-(3-Dimethylaminopyridazin-4-yl)-piperazin-1-yl]-5-oxo-6,7-dihydro-5*H*-⁵λ⁴-thieno[3,2-*d*]pyrimidin-4-yl}-(3-fluorphenyl)-amin
1.11. 6-Chlor-4-{4-[4-(3-fluorphenylamino)-5-oxo-6,7-dihydro-5*H*-5λ⁴-thieno[3,2-*d*]pyrimidin-2-yl]-piperazin-1-yl}-pyridazin-3-ol
1.12. 2-{4-[6-(2-Ethoxyethoxy)-pyridazin-3-yl]-piperazin-1-yl}-5-oxo-6,7-dihydro-5*H*-5λ⁴-thieno[3,2-*d*]pyrimidin-4-yl)-(3-fluorphenyl)-amin
1.13. (3-Fluorphenyl)-[5-oxo-2-(4-pyridazin-4-yl-piperazin-1-yl)-6,7-dihydro-5*H*-5λ⁴-thieno[3,2-*d*]pyrimidin-4-yl]-amin
1.14.(R)-2-{2-[4-(4-Methoxy-1-methyl-1*H-*benzimidazol-2-yl)-piperazin-1-yl]-5-oxo-6,7-dihydro-5*H*-5λ⁴-thieno[3,2-*d*]pyrimidin-4-ylamino}-3-methylbutan-1-ol
1.15. (R)-2-{2-[4-(7-Ethyl-6,7,8,9-tetrahydro-5*H*-pyrazino[2,3-*d*]azepin-2-yl)-piperazin-1-yl]-5-oxo-6,7-dihydro-5*H*-5λ⁴-thieno[3,2-*d*]pyrimidin-4-ylamino}-3-methylbutan-1-ol
1.16. (R)-3-Methyl-2-[5-oxo-2-(4-pyrimidin-4-yl-piperazin-1-yl)-6,7-dihydro-5H-5λ⁴-thieno[3,2-*d*]pyrimidin-4-ylamino]-butan-1-ol
1.17. 4-{4-[4-((R)-1-Hydroxymethyl-2-methylpropylamino)-5-oxo-6,7-dihydro-5*H*-5λ⁴-thieno[3,2-*d*]pyrimidin-2-yl]-piperazin-1-yl}-pyridin-2-ol
1.18. (R)-3-Methyl-2-[5-oxo-2-(4-pyridin-4-yl-piperazin-1-yl)-6,7-dihydro-5*H*-5λ⁴-thieno[3,2-*d*]pyrimidin-4-ylamino]-butan-1-ol
1.19. (R)-2-{2-[4-(3-Dimethylaminopyridazin-4-yl)-piperazin-1-yl]-5-oxo-6,7-dihydro-5*H*-5λ⁴-thieno[3,2-*d*]pyrimidin-4-ylamino}-3-methylbutan-1-ol
1.20. 6-Chlor-4-{4-[4-((R)-1-hydroxymethyl-2-methylpropylamino)-5-oxo-6,7-dihydro-5*H*-5λ⁴-thieno[3,2-*d*]pyrimidin-2-yl]-piperazin-1-yl}-pyridazin-3-ol
1.21. (R)-2-(2-{4-[6-(2-Ethoxyethoxy)-pyridazin-3-yl]-piperazin-1-yl}-5-oxo-6,7-dihydro-5*H-*5λ⁴-thieno[3,2-*d*]pyrimidin-4-ylamino)-3-methylbutan-1-ol
1.22. (R)-3-Methyl-2-[5-oxo-2-(4-pyridazin-4-yl-piperazin-1-yl)-6,7-dihydro-5*H*-5λ⁴-thieno[3,2-*d*]pyrimidin-4-ylamino]-butan-1-ol
1.23. {1-[5-Oxo-2-(4-pyrimidin-4-yl-piperazin-1-yl)-6,7-dihydro-5*H*-5λ⁴-thieno[3,2-*d*]pyrimidin-4-ylamino]-cyclopropyl}-methanol
1.24. {1-[5-Oxo-2-(4-pyridin-4-yl-piperazin-1-yl)-6,7-dihydro-5*H*-5λ4-thieno[3,2-*d*]pyrimidin-4-ylamino]-cyclopropyl}-methanol
1.25th (S)-1-Methyl-5-[5-oxo-2-(4-pyrimidin-4-yl-piperazin-1-yl)-6,7-dihydro-5*H*-5λ⁴-thieno[3,2-*d*]pyrimidin-4-ylamino]-piperidin-2-on
1.26th {2-[4-(5-Fluor-1-methyl-1*H*-benzoimidazol-2-yl)-piperazin-1-yl]-5-oxo-6,7-dihydro-5*H-*5λ⁴-thieno[3,2-*d*]pyrimidin-4-yl}-(tetrahydropyran-4-yl)-amin
1.27th [5-Oxo-2-(4-pyridin-4-yl-piperazin-1-yl)-6,7-dihydro-5*H*-5λ⁴-thieno[3,2-*d*]pyrimidin-4-yl]-(tetrahydropyran-4-yl)-ami
1.28th (3-Fluorphenyl)-{2-[4-(4-methoxy-1-methyl-1*H*-benzimidazol-2-yl)-piperazin-1-yl]-5-oxo-6,7-dihydro-5*H*-5λ4-thieno[3,2-d]pyrimidin-4-yl}-amin
1.29th {2-[4-(7-Ethyl-6,7,8,9-tetrahydro-5*H*-pyrazino[2,3-*d*]azepin-2-yl)-piperazin-1-yl]-5-oxo-6,7-dihydro-5*H*-5λ⁴-thieno[3,2-*d*]pyrimidin-4-yl}-(3-fluorphenyl)-amin
1.30th (3-Fluorphenyl)-[5-oxo-2-(4-pyrimidin-4-yl-piperazin-1-yl)-6,7-dihydro-5*H*-5λ⁴-thieno[3,2-*d*]pyrimidin-4-yl]-amin
1.31 st 4-{4-[4-(3-Fluorphenylamino)-5-oxo-6,7-dihydro-5H-5λ4-thieno[3,2-d]pyrimidin-2-yl]-piperazin-1-yl}-pyridin-2-ol
1.32nd (3-Fluorphenyl)-[5-oxo-2-(4-pyridin-4-yl-piperazin-1-yl)-6,7-dihydro-5H-5λ⁴-thieno[3,2-d]pyrimidin-4-yl]-amin
1.33rd (3-Fluorphenyl)-(2-{4-[4-(4-fluorphenyl)-thiazol-2-yl]-piperazin-1-yl}-5-oxo-6,7-dihydro-5H-5λ⁴-thieno[3,2-d]pyrimidin-4-yl)-amin
1.34th [2-(4-Benzo[d]isoxazol-3-yl-piperazin-1-yl)-5-oxo-6,7-dihydro-5H-5λ4-thieno[3,2-d]pyrimidin-4-yl]-(3-fluorphenyl)-amin
1.35th (R)-2-(2-{4-[4-(4-Fluorphenyl)-thiazol-2-yl]-piperazin-1-yl}-5-oxo-6,7-dihydro-5H-5λ4-thieno[3,2-d]pyrimidin-4-ylamino)-3-methylbutan-1-ol
1.36th (R)-2-[2-(4-Benzo[d]isoxazol-3-yl-piperazin-1-yl)-5-oxo-6,7-dihydro-5H-5λ4thieno[3,2-d]pyrimidin-4-ylamino]-3-methylbutan-1-ol
wenigstens ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) enthalten.

Die oben genannten Verbindungen der Formel **1** werden wie in den Synthesevorschriften im Detail beschrieben hergestellt.

Weiterhin bevorzugt sind die oben genannten Arzneimittelkombinationen, wobei der PDE4-Inhibitor der allgemeinen Formel **1** in einer Einzeldosis von 0,01 mg bis 50 mg, vorzugsweise von 0,05 bis 30 mg, bevorzugter von 0,1 bis 20 mg, insbesondere von 0,5 bis 10 mg verabreicht wird.

Insbesondere betrifft die Erfindung die oben genannten Arzneimittelkombinationen, bei denen die oder zumindest eine oder mehrere der PDE4-Inhibitor-vermittelten Nebenwirkungen in erheblichem Ausmaß reduziert oder völlig vermieden wird im Vergleich zu einer alleinigen Applikation des in der Arzneimittelkombination verwendeten PDE4-Inhibitors. Diese PDE4-Inhibitor-vermittelten Nebenwirkungen sind vorzugsweise ausgewählt aus Körpergewichtsverlust, Leukozytose, Neutrophilie, Übelkeit, Erbrechen, Diarrhoe (einschließlich des Auftretens von Entzündungsparametern und der Fibroblasten-Proliferation im Mesenterium). Diese PDE4-Inhibitor-vermittelten Nebenwirkungen sind stärker bevorzugt ausgewählt aus Körpergewichtsverlust, Leukozytose, Neutrophilie und Diarrhoe. Diese PDE4-Inhibitor-vermittelten Nebenwirkungen betreffen insbesondere das Auftreten von Diarrhoe.

Ein weiterer Gegenstand der vorliegenden Erfindung ist die Verwendung eines NSAIDs (2) zur Reduzierung der Nebenwirkungen eines oder mehrerer PDE4-Inhibitoren bei der Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des peripheren oder zentralen Nervensystems.

Ein anderer Gegenstand der vorliegenden Erfindung ist die Verwendung einer Kombination enthaltend einen oder mehrere PDE4-Inhibitoren undwenigstens ein NSAID **(2)** zur Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

Weiterhin bevorzugt ist die Verwendung einer Kombination eines PDE4-Inhibitors der allgemeinen Formel **1** 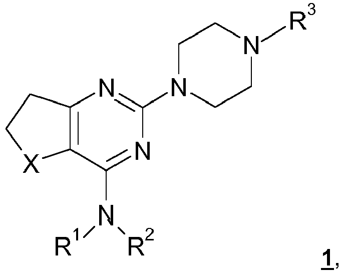 worin X, R¹, R² und R³ wie vorstehend definiert und bevorzugt definiert sind,
und wenigstens eines NSAIDs **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) zur Herstellung einer Arzneimittelkombination zur Behandlung einer der Erkrankungen ausgewählt aus Atemwegserkrankungen, Lungenerkrankungen, gastrointestinale Beschwerden, Erkrankungen, und auch entzündliche Erkrankungen der Gelenke, der Haut oder der Augen, Krebserkrankungen, Erkrankungen des periphären oder zentralen Nervensystems, insbesondere jedoch zur Behandlung von entzündlichen und obstruktiven Erkrankungen wie COPD, chronischer Sinusitis, Asthma, Morbus Crohn, Colitis ulcerosa.

Bevorzugter Gegenstand der Erfindung ist hierbei die Verwendung der Kombination enthaltend einen PDE4-Inhibitor nach Formel **1** - und wenigstens einen NSAID **(2)** zur Herstellung einer Arzneimittelkombination zur Behandlung der oben genannten Erkrankungen, dadurch gekennzeichnet, dass der PDE4-Inhibitor der Formel **1** - ein NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) in einer einzigen Formulierung gemeinsam und gleichzeitig verabreicht werden, wobei diese einzige Formulierung vorzugsweise eine orale Formulierung ist wie beispielsweise eine Tablette oder Kapsel. Weiterhin bevorzugt ist hierbei die Verwendung der Kombination enthaltend einen PDE4-Inhibitor nach Formel **1** -und wenigstens einen NSAID **(2)** ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) zur Herstellung einer Arzneimittelkombination zur Behandlung der oben genannten Erkrankungen, dadurch gekennzeichnet, dass der PDE4-lnhibitor und das wenigstens eine NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) in zwei separaten Formulierungen getrennt voneinander innerhalb eines zeitlichen Abstandes von 0 bis 6 Stunden verabreicht werden. Bei dieser getrennten Applikation in zwei separaten Formulierungen kann die Formulierung enthaltend den PDE4-Inhibitor eine orale oder inhalative Formulierung sein, ist aber vorzugsweise eine orale Formulierung, und die Formulierung enthaltend das wenigstens eine NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) ist vorzugsweise eine orale Formulierung. Weiterhin wird bei der Verwendung der Kombination in getrennten Formulierungen zur Herstellung einer Arzneimittelkombination zur Behandlung der oben genannten Erkrankungen die Formulierung enthaltend den PDE4-Inhibitor vorzugsweise einmal täglich und die Formulierung enthaltend das wenigstens eine NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) vorzugsweise entweder einmal oder zweimal täglich verabreicht.

Weiterhin bevorzugt ist die Verwendung der Kombination enthaltend einen oder mehrere PDE4-Inhibitoren nach Formel **1** und des wenigstens einen NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) zur Herstellung einer Arzneimittelkombination zur Behandlung der oben genannten Erkrankungen, wobei PDE4-lnhibitoren der allgemeinen Formel **1** verwendet werden, worin
- **R¹**: H ist,
- **R²**: ein Rest ausgewählt ist aus der Gruppe bestehend aus 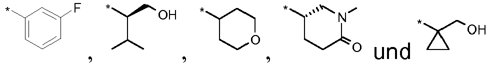 ist,
- **R³**: ein Rest ausgewählt ist aus der Gruppe bestehend aus

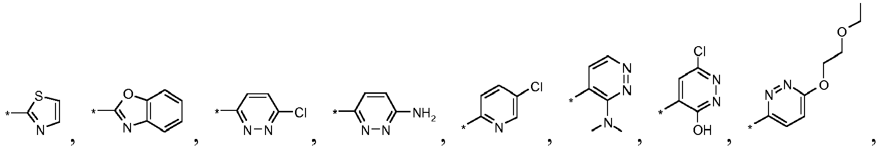

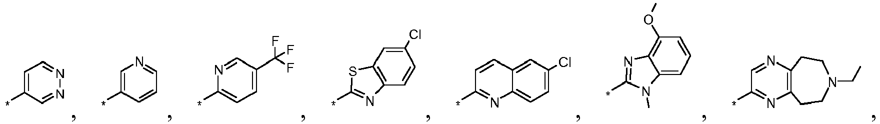

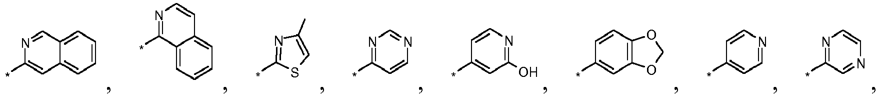

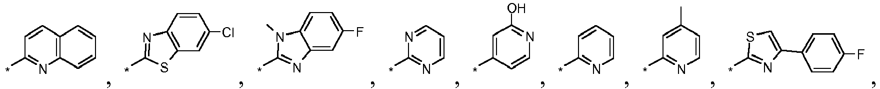

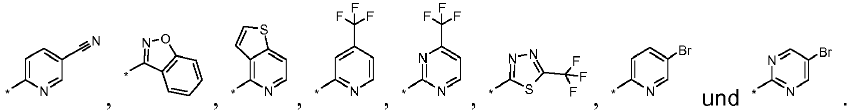

Insbesondere sind bei den oben genannten Verwendungen die PDE4-lnhibitoren der Formel **1** ausgewählt aus:
1.1. (3-Fluorphenyl)-[5-oxo-2-(4-thiazol-2-yl-piperazin-1-yl)-6,7-dihydro-5*H*-5λ⁴-thieno[3,2-*d*]pyrimidin-4-yl]-amin
1.2. (R)-3-Methyl-2-[5-oxo-2-(4-thiazol-2-yl-piperazin-1-yl)-6,7-dihydro-5*H*-5λ⁴-thieno[3,2-*d*]pyrimidin-4-ylamino]-butan-1-ol
1.3. [2-(4-Benzoxazol-2-yl-piperazin-1-yl)-5-oxo-6,7-dihydro-5*H*-5λ□4-thieno[3,2-*d*]pyrimidin-4-yl]-(3-fluorphenyl)-amin
1.4. [2-(4-Benzoxazol-2-yl-piperazin-1-yl)-5-oxo-6,7-dihydro-5*H*-5λ⁴-thieno[3,2-*d*]pyrimidin-4-yl]-(tetrahydropyran-4-yl)-amin
1.5. (R)-2-{2-[4-(6-Chlorpyridazin-3-yl)-piperazin-1-yl]-5-oxo-6,7-dihydro-5*H*-5λ⁴-thieno[3,2-*d*]pyrimidin-4-ylamino}-3-methylbutan-1-ol
1.6. {2-[4-(6-Chlorpyridazin-3-yl)-piperazin-1-yl]-5-oxo-6,7-dihydro-5*H*-5λ⁴-thieno[3,2-*d*]pyrimidin-4-yl}-(3-fluorphenyl)-amin
1.7. (R)-2-[2-(4-Benzoxazol-2-yl-piperazin-1-yl)-5-oxo-6,7-dihydro-5*H*-5λ⁴-thieno[3,2-*d*]pyrimidin-4-ylamino]-3-methylbutan-1-ol
1.8. (1-{2-[4-(5-Chlorpyridin-2-yl)-piperazin-1-yl]-5-oxo-67-dihydro-5*H*-5λ⁴-thieno[3,2-*d*]pyrimidin-4-ylamino}-cyclopropyl)-methanol
1.9. {2-[4-(5-Chlorpyridin-2-yl)-piperazin-1-yl]-5-oxo-6,7-dihydro-5*H*-5λ⁴-thieno[3,2-*d*]pyrimidin-4-yl}-(tetrahydropyran-4-yl)-amin
1.10. {2-[4-(3-Dimethylaminopyridazin-4-yl)-piperazin-1-yl]-5-oxo-6,7-dihydro-5*H*-5λ⁴-thieno[3,2-*d*]pyrimidin-4-yl}-(3-fluorphenyl)-amin
1.11. 6-Chlor-4-{4-[4-(3-fluorphenylamino)-5-oxo-6,7-dihydro-5*H*-5λ⁴-thieno[3,2-*d*]pyrimidin-2-yl]-piperazin-1-yl}-pyridazin-3-ol
1.12. 2-{4-[6-(2-Ethoxyethoxy)-pyridazin-3-yl]-piperazin-1-yl}-5-oxo-6,7-dihydro-5*H*-5λ⁴-thieno[3,2-*d*]pyrimidin-4-yl)-(3-fluorphenyl)-amin
1.13. (3-Fluorphenyl)-[5-oxo-2-(4-pyridazin-4-yl-piperazin-1-yl)-6,7-dihydro-5*H*-5λ⁴-thieno[3,2-*d*]pyrimidin-4-yl]-amin
1.14. (R)-2-{2-[4-(4-Methoxy-1-methyl-1*H*-benzimidazol-2-yl)-piperazin-1-yl]-5-oxo-6,7-dihydro-5*H*-5λ⁴-thieno[3,2-*d*]pyrimidin-4-ylamino}-3-methylbutan-1-ol
1.15. (R)-2-{2-[4-(7-Ethyl-6,7,8,9-tetrahydro-5*H*-pyrazino[2,3-*d*]azepin-2-yl)-piperazin-1-yl]-5-oxo-6,7-dihydro-5*H*-5λ⁴-thieno[3,2-*d*]pyrimidin-4-ylamino}-3-methylbutan-1-ol
1.16. (R)-3-Methyl-2-[5-oxo-2-(4-pyrimidin-4-yl-piperazin-1-yl)-6,7-dihydro-5*H*-5λ⁴-thieno[3,2-*d*]pyrimidin-4-ylamino]-butan-1-ol
1.17. 4-{4-[4-((R)-1-Hydroxymethyl-2-methylpropylamino)-5-oxo-6,7-dihydro-5*H*-5λ⁴-thieno[3,2-*d*]pyrimidin-2-yl]-piperazin-1-yl}-pyridin-2-ol
1.18. (R)-3-Methyl-2-[5-oxo-2-(4-pyridin-4-yl-piperazin-1-yl)-6,7-dihydro-5*H*-5λ⁴-thieno[3,2-*d*]pyrimidin-4-ylamino]-butan-1-ol
1.19. (R)-2-{2-[4-(3-Dimethylaminopyridazin-4-yl)-piperazin-1-yl]-5-oxo-6,7-dihydro-5*H*-5λ⁴-thieno[3,2-*d*]pyrimidin-4-ylamino}-3-methylbutan-1-ol
1.20. 6-Chlor-4-{4-[4-((R)-1-hydroxymethyl-2-methylpropylamino)-5-oxo-6,7-dihydro-5*H*-5λ⁴-thieno[3,2-*d*]pyrimidin-2-yl]-piperazin-1-yl}-pyridazin-3-ol
1.21. (R)-2-(2-{4-[6-(2-Ethoxyethoxy)-pyridazin-3-yl]-piperazin-1-yl}-5-oxo-6,7-dihydro-5*H-*5λ⁴-thieno[3,2-*d*]pyrimidin-4-ylamino)-3-methylbutan-1-ol
1.22. (R)-3-Methyl-2-[5-oxo-2-(4-pyridazin-4-yl-piperazin-1-yl)-6,7-dihydro-5*H*-5λ⁴-thieno[3,2-*d*]pyrimidin-4-ylamino]-butan-1-ol
1.23. {1-[5-Oxo-2-(4-pyrimidin-4-yl-piperazin-1-yl)-6,7-dihydro-5*H*-5λ⁴-thieno[3,2-*d*]pyrimidin-4-ylamino]-cyclopropyl}-methanol
1.24. {1-[5-Oxo-2-(4-pyridin-4-yl-piperazin-1-yl)-6,7-dihydro-5*H*-5λ⁴-thieno[3,2-*d*]pyrimidin-4-ylamino]-cyclopropyl}-methanol
1.25th (S)-1-Methyl-5-[5-oxo-2-(4-pyrimidin-4-yl-piperazin-1-yl)-6,7-dihydro-5*H*-5λ⁴-thieno[3,2-d]pyrimidin-4-ylamino]-piperidin-2-on
1.26th {2-[4-(5-Fluor-1-methyl-1H-benzoimidazol-2-yl)-piperazin-1-yl]-5-oxo-6,7-dihydro-5*H-*5λ4-thieno[3,2-d]pyrimidin-4-yl}-(tetrahydropyran-4-yl)-amin
1.27th [5-Oxo-2-(4-pyridin-4-yl-piperazin-1-yl)-6,7-dihydro-5*H*-5λ4-thieno[3,2-d]pyrimidin-4-yl]-(tetrahydropyran-4-yl)-amin
1.28th (3-Fluorphenyl)-{2-[4-(4-methoxy-1-methyl-1H-benzimidazol-2-yl)-piperazin-1-yl]-5-oxo-6,7-dihydro-5*H*-5λ4-thieno[3,2-*d*]pyrimidin-4-yl}-amin
1.29th {2-[4-(7-Ethyl-6,7,8,9-tetrahydro-5*H*-pyrazino[2,3-*d*]azepin-2-yl)-piperazin-1-yl]-5-oxo-6,7-dihydro-5*H*-5λ4-thieno[3,2-d]pyrimidin-4-yl}-(3-fluorphenyl)-amin
1.30th (3-Fluorphenyl)-[5-oxo-2-(4-pyrimidin-4-yl-piperazin-1-yl)-6,7-dihydro-5*H*-5λ4-thieno[3,2-d]pyrimidin-4-yl]-amin
1.31 st 4-{4-[4-(3-Fluorphenylamino)-5-oxo-6,7-dihydro-5*H*-5λ4-thieno[3,2-d]pyrimidin-2-yl]-piperazin-1-yl}-pyridin-2-ol
1.32nd (3-Fluorphenyl)-[5-oxo-2-(4-pyridin-4-yl-piperazin-1-yl)-6,7-dihydro-5*H*-5λ4-thieno[3,2-*d*]pyrimidin-4-yl]-amin
1.33rd (3-Fluorphenyl)-(2-{4-[4-(4-fluorphenyl)-thiazol-2-yl]-piperazin-1-yl}-5-oxo-6,7-dihydro-5H-5λ4-thieno[3,2-d]pyrimidin-4-yl)-amin
1.34th [2-(4-Benzo[d]isoxazol-3-yl-piperazin-1-yl)-5-oxo-6,7-dihydro-5*H*-5λ4-thieno[3,2-d]pyrimidin-4-yl]-(3-fluorphenyl)-amin
1.35th (R)-2-(2-{4-[4-(4-Fluorphenyl)-thiazol-2-yl]-piperazin-1-yl}-5-oxo-6,7-dihydro-5*H*-5λ4-thieno[3,2-*d*]pyrimidin-4-ylamino)-3-methylbutan-1-ol
1.36th (R)-2-[2-(4-Benzo[d]isoxazol-3-yl-piperazin-1-yl)-5-oxo-6,7-dihydro-5*H*-5λ4-thieno[3,2-*d*]pyrimidin-4-ylamino]-3-methylbutan-1-ol.

Insbesondere werden bei den oben genannten Verwendungen die NSAIDS (**2**) ausgewählt aus der Gruppe bestehend aus
- Meloxicam (2.77), bevorzugt in einer Einzeldosis von 7,5 bis 30 mg, stärker bevorzugt von 10 bis 20 mg und
- Naproxen (2.82), bevorzugt in einer Einzeldosis von 250 bis 1000 mg, stärker bevorzugt von 250 bis 750 mg, und
wobei diese Einzeldosis ein- oder zweimal täglich verabreicht werden kann.

Bevorzugt wird bei den oben genannten Verwendungen der Kombination zur Therapie der oben genannten Erkrankungen der PDE4-lnhibitor der allgemeinen Formel **1** in einer Einzeldosis von 0,01 mg bis 50 mg, vorzugsweise von 0,05 bis 30 mg, stärker bevorzugt von 0,1 bis 20 mg, insbesondere von 0,5 bis 10 mg verabreicht.

Insbesondere betrifft die Erfindung die oben genannten Verwendungen, bei denen die oder zumindest eine oder mehrere der PDE4-lnhibitor-vermittelten Nebenwirkungen in erheblichem Ausmaß reduziert oder völlig vermieden wird im Vergleich zu einer alleinigen Applikation des in der Arzneimittelkombination verwendeten PDE4-Inhibitors.

Insbesondere betrifft die Erfindung weiterhin die Verwendung von NSAIDs, vorzugsweise wie vorstehend definiert und bevorzugt definiert, zur Reduktion oder Vermeidung einer oder mehrerer PDE4-lnhibitor-vermittelter Nebenwirkungen.

Diese PDE4-lnhibitor-vermittelten Nebenwirkungen sind vorzugsweise ausgewählt aus Körpergewichtsverlust, Leukozytose, Neutrophilie, Übelkeit, Erbrechen, Diarrhoe (einschließlich des Auftretens von Entzündungsparametern und der Fibroblasten-Proliferation im Mesenterium). Diese PDE4-lnhibitor-vermittelten Nebenwirkungen sind stärker bevorzugt ausgewählt aus Körpergewichtsverlust, Leukozytose, Neutrophilie und Diarrhoe. Diese PDE4-Inhibitor-vermittelten Nebenwirkungen betreffen insbesondere das Auftreten von Diarrhoe.

### SYNTHESEVORSCHRIFTEN

Die Verbindungen der allgemeinen Formel (I) können nach folgendem allgemeinen Syntheseschema hergestellt werden, wobei die Substituenten der allgemeinen Formel (I) die zuvor genannten Bedeutungen haben. Diese Verfahren sind als Erläuterung der Erfindung zu verstehen ohne selbige auf deren Gegenstand zu beschränken.

### ALLGEMEINES SYNTHESE SCHEMA

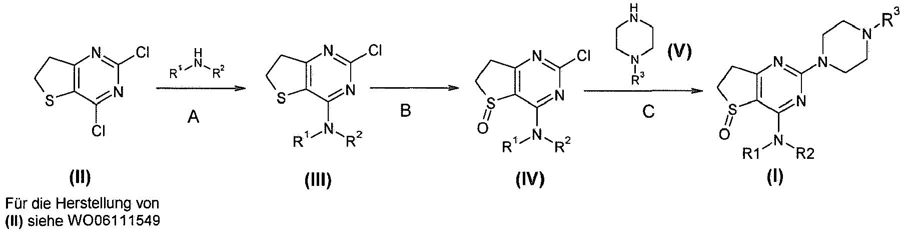

### 1. SYNTHESE VON (3-FLUORPHENYL)-[5-OXO-2-(4-THIAZOL-2-YL-PIPERAZIN-1-YL)-6,7-DIHYDRO-5H-5λ⁴-THIENO[3,2-d]PYRIMIDIN-4-YL]-AMIN (BEISPIEL 1.1)

### 1.1 (2-Chlor-6,7-dihydrothieno[3,2-d]pyrimidin-4-yl)-(3-fluorphenyl)-amin (III-1)

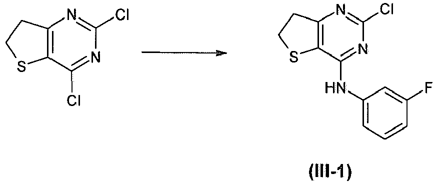

4 g **(II)** werden in 15 ml Dimethylformamid vorgelegt, anschliessend werden 4,5 ml Diisopropylethylamin und dann 2,5 ml 3-Fluorphenylamin zugegeben. Das Reaktionsgemisch wird bei 120°C, bis keine weitere Umsetzung erfolgt, erhitzt und nach Abkühlen eingedampft. Der Rückstand wird mit Wasser versetzt. Das Produkt wird mit Dichlormethan extrahiert und chromatographisch (Kieselgel, Petrolether/Essigester 80/20 bis 60/40) gereinigt. 2,6 g **(III-1)** werden als Feststoff erhalten. Analytische HPLC (Methode A): RT = 3,27 min.

### 1.2 2-Chlor-5-oxo-6,7-dihydro-5H-5λ⁴-thieno[3,2-d]pyrimidin-4-yl)-(3-fluorphenyl)-amin (IV-1)

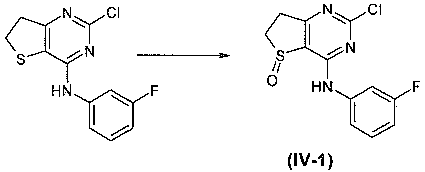

0,102 g S-(-)-1,1'-Bi-2-naphtol werden in 0,5 ml Chloroform unter Argon vorgelegt, dann 0,052 ml Titan(IV)-isopropylat und 0,064 ml Wasser zugegeben. Das Reaktionsgemisch wird 45 Minuten bei Raumtemperatur gerührt. Anschließend wird eine Suspension von 0,5 g **(III-1)** in 25 ml Chloroform zugegeben. Das Reaktionsgemisch wird auf -2°/-4°C abgekühlt und nach 20 Minuten werden 0,323 ml tert-Butylhydroperoxid 5-6 M in Decan zugetropft. Das Reaktionsgemisch wird bei -2/-4°C, bis keine weitere Umsetzung erfolgt, weitergerührt und mit Wasser versetzt. Das Produkt wird mit Dichlormethan extrahiert und chromatographisch (Kieselgel, Dichlormethan/Methanol 100/0 bis 95/5) gereinigt. 0,47 g **(IV-1)** werden als Feststoff erhalten. Analytische HPLC-MS (Methode A): RT = 1,16 min.

### 1.3 (3-Fluorphenyl)-[5-oxo-2-(4-thiazol-2-yl-piperazin-1-yl)-6,7-dihydro-5H-5λ⁴-thieno[3,2-d]pyrimidin-4-yl]-amin (Beispiel 1.1)

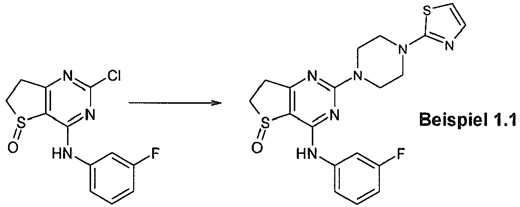

0,2 g **(IV-1)** wird in 3 ml Dioxan vorgelegt, 240 µl Diisopropylethylamin und 0,24 g 1-Thiazol-2-yl-piperazin werden zugefügt. Das Reaktionsgemisch wird bei 120°C, bis keine weitere Umsetzung erfolgt, in der Mikrowelle erhitzt und mit Wasser versetzt. Der ausgefallene Feststoff wird abgesaugt und chromatographisch (Kieselgel, Essigester/Methanol 100/0 bis 80/20) gereinigt. 0,17 g **Beispiel 1.1** werden als Feststoff erhalten. Analytische HPLC-MS (Methode A): RT = 1,07 min.

### 2. SYNTHESE VON (R)-3-METHYL-2-[5-OXO-2-(4-THIAZOL-2-YL-PIPERAZIN-1-YL)-6,7-DIHYDRO-5H-5λ⁴-THIENO[3,2-d]PYRIMIDIN-4-YLAMINO]-BUTAN-1-OL (BEISPIEL 1.2)

### 2.1 (R)-2-(2-Chlor-6,7-dihydro-thieno[3,2-d]pyrimidin-4-y)amino)-3-methyl-butan-1-ol (III-2):

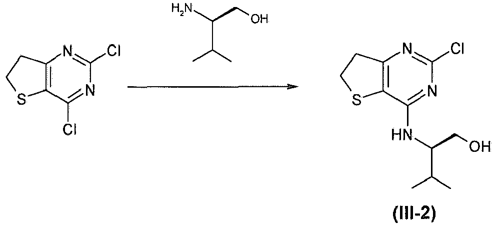

7,2 g 2,4-Dichlor-6,7-dihydro-thieno[3,2-*d*]pyrimidin **(II)** werden in 36 ml Dioxan vorgelegt, anschliessend werden 18 ml Diisopropylethylamin und dann 6,1 g (R)-(-)-2-Amino-3-Methyl-1-butanol zugegeben. Das Reaktionsgemisch wird bei 100°C, bis keine weitere Umsetzung erfolgt, erhitzt und nach Abkühlen eingedampft. Der Rückstand wird mit Petrolether/Essigester 9:1 im Ultraschallbad behandelt und der Feststoff abgesaugt und getrocknet. 8,3 g **(III-2)** werden als Feststoff erhalten. Analytische HPLC (Methode A): RT = 2,75 min

### 2.2 (R)-2-(2-Chlor-5-oxo-6,7-dihydro-5H-5λ⁴-thieno[3,2-d]pyrimidin-4-ylamino)-3-methylbutan-1-ol (IV-2) :

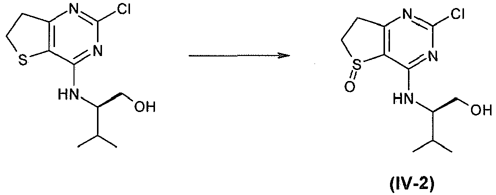

4,1 g S-(-)-1,1'-Bi-2-naphtol werden in 15 ml Chloroform unter Argon vorgelegt, dann 0,44 ml Titan(IV)-isopropylat und 0,54 ml Wasser zugegeben. Das Reaktionsgemisch wird 1 Stunde bei Raumtemperatur gerührt. Anschließend wird eine Suspension von 4,1 g **(III-2)** in 107 ml Dichlormethan zugegeben. Das Reaktionsgemisch wird auf -2°C abgekühlt und nach 30 Minuten werden 2,7 ml tert-Butylhydroperoxid 5-6 M in Decan zugetropft. Das Reaktionsgemisch wird bei -2 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 100/0 bis 86/14) gereinigt. 2,45 g **(IV-2)** werden als Feststoff erhalten. Analytische HPLC-MS (Methode A): RT = 0,98 min

### 2.3 (R)-3-Methyl-2-[5-oxo-2-(4-thiazol-2-yl-piperazin-1-yl)-6,7-dihydro-5H-5λ⁴-thieno[3,2-d]pyrimidin-4-ylamino]-butan-1-ol (Beispiel 1.2)

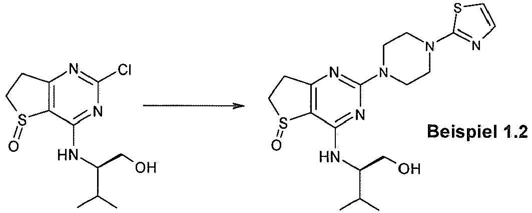

Ausgehend von 0,2 g **(IV-2)** und 0,245 g 1-Thiazol-2-yl-piperazin werden 0,13 g **Beispiel 1.2** analog zu Beispiel 1.1 (siehe 1.3) hergestellt. Das Reaktionsgemisch wird mit Wasser versetzt und das Produkt mit Dichlormethan extrahiert und chromatographisch (Kieselgel, Dichlormethan/Methanol 100/0 bis 90/10) gereinigt. Analytische HPLC-MS (Methode A): RT = 0,87 min.

### 3. SYNTHESE VON [2-(4-BENZOXAZOL-2-YL-PIPERAZIN-1-YL)-5-OXO-6,7-DI-HYDRO-5H-5λ⁴-THIENO[3,2-d]PYRIMIDIN-4-YL]-(3-FLUORPHENYL)-AMIN (BEISPIEL 1.3)

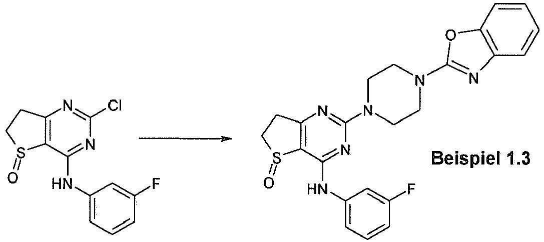

Ausgehend von 0,2 g **(IV-1)** (siehe 1.2) und 0,287 g 2-Piperazin-1-yl-benzoxazol werden 0,31 g **Beispiel 1.3** analog zu Beispiel 1.1 (siehe 1.3) hergestellt. Das Reaktionsgemisch wird mit Wasser versetzt und das Produkt abgesaugt. Analytische HPLC-MS (Methode A): RT = 1,23 min.

### 4. SYNTHESE VON [2-(4-BENZOXAZOL-2-YL-PIPERAZIN-1-YL)-5-OXO-6,7-DIHYDRO-5H-5λ⁴-THIENO[3,2-d]PYRIMIDIN-4-YL]-(TETRAHYDROPYRAN-4-YL)-AMIN (BEISPIEL 1.4)

### 4.1 (2-Chlor-6,7-dihydrothieno[3,2-d]pyrimidin-4-yl)-(tetrahydropyran-4-yl)-amin (III-3):

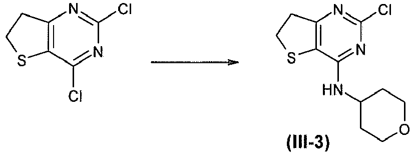

0,68 g **(II)** werden in 6 ml Dioxan vorgelegt, anschliessend werden 1,72 ml Diisopropylethylamin und dann 0,6 g 4-Aminotetrahydropyran zugegeben. Das Reaktionsgemisch wird bei 130°C, bis keine weitere Umsetzung erfolgt, erhitzt und nach Abkühlen eingedampft. Das Produkt wird mit Wasser im Ultraschallbad behandelt und der Feststoff abgesaugt und getrocknet. 0,66 g **(III-3)** werden erhalten. Analytische HPLC-MS (Methode A): RT = 1,08 min.

### 4.2 (2-Chlor-5-oxo-6,7-dihydro-5H-5λ⁴-thieno[3,2-d]pyrimidin-4-yl)-(tetrahydropyran-4-yl)-amin (IV-3):

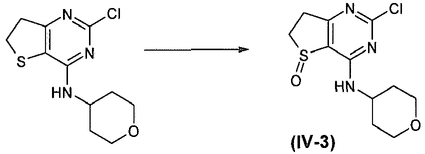

0,14 g S-(-)-1,1'-Bi-2-naphtol werden in 5 ml Chloroform unter Argon vorgelegt, dann 0,072 ml Titan(IV)-isopropylat und 0,087 ml Wasser zugegeben. Das Reaktionsgemisch wird 45 Minuten bei Raumtemperatur gerührt. Anschließend wird eine Suspension von 0,66 g **(III-3)** in 25 ml Chloroform zugegeben. Das Reaktionsgemisch wird auf -10°C abgekühlt und nach 60 Minuten werden 0,444 ml tert-Butylhydroperoxid 5-6 M in Decan zugetropft. Das Reaktionsgemisch wird bei -10 bis -4°C, bis keine weitere Umsetzung erfolgt, weitergerührt und mit Wasser versetzt. Das Produkt wird mit Dichlormethan extrahiert und chromatographisch (Kieselgel, Essigester/Methanol 100/0 bis 80/20) gereinigt. 0,42 g **(IV-3)** werden als Feststoff erhalten. Analytische HPLC-MS (Methode A): RT = 0,94 min.

### 4.3 [2-(4-Benzoxazol-2-yl-piperazin-1-yl)-5-oxo-6,7-dihydro-5H-5λ⁴-thieno[3,2-d]pyrimidin-4-yl]-(tetrahydropyran-4-yl)-amin (Beispiel 1.4)

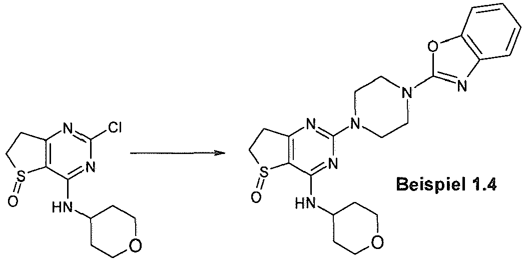

Ausgehend von 0,2 g **(IV-3)** und 0,315 g 2-Piperazin-1-yl-benzoxazol werden 0,3 g **Beispiel 1.4** analog zu **Beispiel 1.3** (siehe 3.) hergestellt und aufgearbeitet. Analytische HPLC-MS (Methode A): RT = 1,04 min.

### 5. SYNTHESE VON (R)-2-{2-[4-(6-CHLORPYRIDAZIN-3-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL (BEISPIEL 1.5)

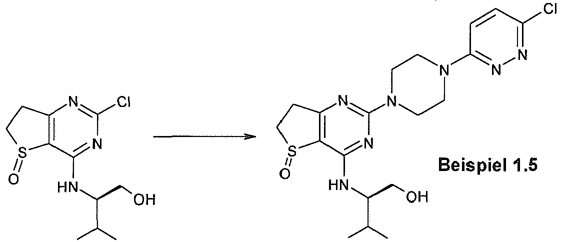

Ausgehend von 0,2 g **(IV-2)** (siehe 2.2) und 0,287 g 3-Chlor-6-piperazin-1-yl-pyridazin, werden 0,257g **Beispiel 1.5** analog zu Beispiel 1.3 (siehe 3.) hergestellt und aufgearbeitet. Analytische HPLC-MS (Methode A): RT = 0,98 min.

### 6. SYNTHESE VON (2-[4-(6-CHLORPYRIDAZIN-3-YL)-PIPERAZIN-1-YL]-5-OXO-6,7-DIHYDRO-5H-5λ⁴-THIENO[3,2-d]PYRIMIDIN-4-YL}-(3-FLUORPHENYL)-AMIN (BEISPIEL 1.6)

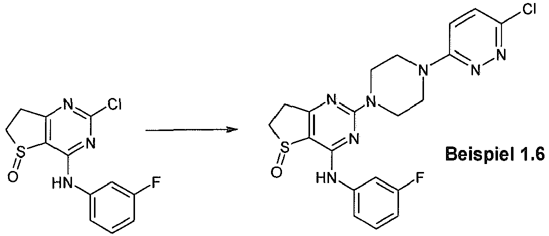

Ausgehend von 0,2 g **(IV-1)** (siehe 1.2) und 0,28 g 3-Chlor-6-piperazin-1-yl-pyridazin, werden 0,31 g **Beispiel 1.6** analog zu **Beispiel 1.3** (siehe 3.) hergestellt. Analytische HPLC-MS (Methode A): RT = 1,12 min.

### 7. SYNTHESE VON (R)-2-[2-(4-BENZOXAZOL-2-YL-PIPERAZIN-1-YL)-5-OXO-6,7-DIHYDRO-5H-5λ⁴-THIENO[3,2-d]PYRIMIDIN-4-YLAMINO]-3-METHYLBUTAN-1-OL (BEISPIEL 1.7)

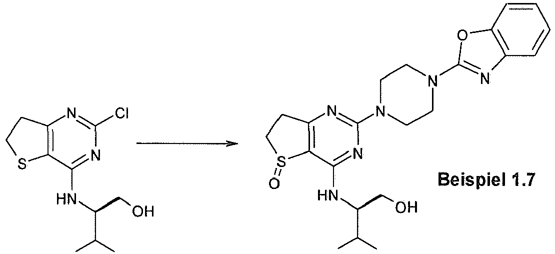

Ausgehend von 0,2 g **(IV-2)** (siehe 2.2) und 0,313 g 2-Piperazin-1-yl-benzoxazol werden 0,16 g **Beispiel 1.7** analog zu Beispiel 1.1 (siehe 1.3) hergestellt. Das Reaktionsgemisch wird mit Wasser versetzt und das Produkt mit Dichlormethan extrahiert und chromatographisch (Kieselgel, Essigester/Methanol 100/0 bis 80/20) gereinigt. Analytische HPLC-MS (Methode A): RT = 1,06 min.

### 8. SYNTHESE VON (1-{2-[4-(5-CHLORPYRIDIN-2-YL)-PIPERAZIN-1-YL]-5-OXO-6,7-DIHYDRO-5H-5λ⁴-THIENO[3,2-d]PYRIMIDIN-4-YLAMINO}-CYCLOPROPYL)-METHANOL (BEISPIEL 1.8)

### 8.1 (1-Hydroxymethylcyclopropyl)-carbamidsäure tert-butylester:

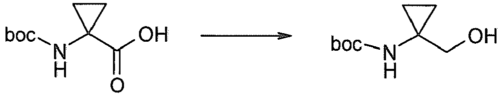

1 g 1-(BOC-amino)-cyclopropancarbonsäure wird in 20 ml Dimethoxyethan gelöst und auf - 70°C abgekühlt. Dann werden 0,65 ml N-Methylmorpholin zugegeben und 0,71 ml Isobutylchloroformiat in 5 ml Dimethoxyethan zugetropft. Das Reaktionsgemisch wird auf - 5°C erwärmt. Der Niederschlag wird abgesaugt. Das Eluat wird auf -15°C abgekühlt und 0,303 g Natriumborhydrid werden langsam zugegeben. Das Reaktionsgemisch wird anschliessend 30 Minuten bei Raumtemperatur gerührt, mit Wasser versetzt und das Produkt mit Dichlormethan extrahiert. Die organische Phase wird getrocknet und zur Trockne eingedampft. 1,04 g Produkt werden als Feststoff erhalten. ¹H NMR (400 MHz, DMSO): 1,36 (9H, s); 0,61 (2H, t); 0,52 (2H, t).

### 8.2 1-Aminocyclopropanmethanol:

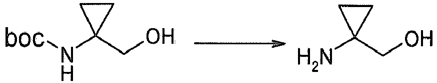

1,04 g (1-Hydroxymethylcyclopropyl)-carbamidsäure *tert*-butylester werden in 5 ml Dioxan vorgelegt. 2,5 ml HCl in Dioxan (4 mol/l) werden zugetropft. Das Reaktionsgemisch wird bei Raumtemperatur 15 h gerührt. Das Lösungsmittel wird zur Hälfte eingedampft und der ausgefallene Feststoff abgesaugt. 0,5 g Produkt werden als Hydrochlorid erhalten. ¹H NMR (400 MHz, DMSO): 5,27 (1 H, t); 0,91 (2H, t); 0,71 (2H, t).

### 8.3 [1-(2-Chlor-6,7-dihydrothieno[3,2-d]pyrimidin-4-ylamino)-cyclopropyl]-methanol (III-4):

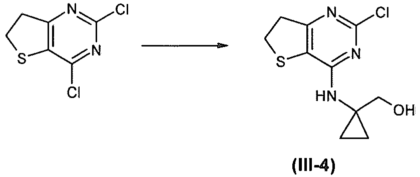

1,4 g **(II)** werden in 10 ml Dioxan vorgelegt, erst werden 3,6 ml Diisopropylethylamin, dann 1 g 1-Aminocyclopropanmethanol (siehe 8.2) zugegeben. Das Reaktionsgemisch wird bei 160°C, bis keine weitere Umsetzung erfolgt, erhitzt und nach Abkühlen eingedampft.

Der Rückstand wird mit Cyclohexan/Essigester (8:2) im Ultraschallbad behandelt und der Feststoff abgesaugt und getrocknet. 1,24 g **(III-4)** werden als Feststoff erhalten. Analytische HPLC-MS (Methode A): RT = 1,01 min.

### 8.4 [1-(2-Chlor-5-oxo-6,7-dihydro-5H-5λ⁴-thieno[3,2-d]pyrimidin-4-ylamino)-cyclopropyl]-methanol (IV-4) :

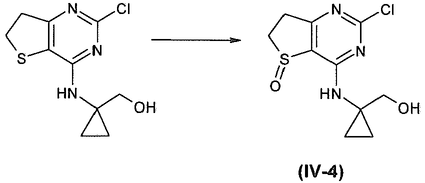

0,28 g S-(-)-1,1'-Bi-2-naphtol werden in 20 ml Chloroform unter Argon vorgelegt, dann werden 0,14 ml Titan(IV)-isopropylat und 0,17 ml Wasser zugegeben. Das Reaktionsgemisch wird 1 Stunde bei Raumtemperatur gerührt. Anschließend wird eine Suspension von 1,2 g **(III-4)** in 40 ml Dichlormethan und 2 ml Methanol zugegeben. Das Reaktionsgemisch wird auf -5°C abgekühlt und nach 30 Minuten werden 0,91 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Die Wasserphase wird wird mit Dichlormethan gewaschen und gefriergetrocknet. 1 g **(IV-4)** wird als Feststoff erhalten. Analytische HPLC-MS (Methode A) RT = 0,85 min.

### 8.5 (1-{2-[4-(5-Chlorpyridin-2-yl)-piperazin-1-yl]-5-oxo-6,7-dihydro-5H-5λ⁴-thieno[3,2-d]pyrimidin-4-ylamino}-cyclopropyl)-methanol (Beispiel 1.8)

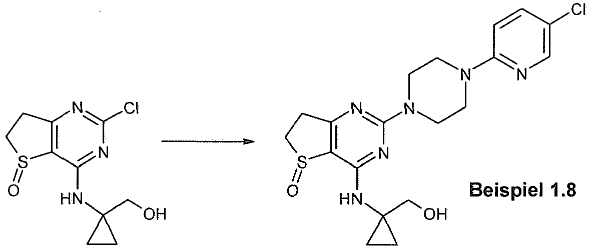

0,1 g **(IV-4)** wird in 3 ml N-Methyl-2-pyrrolidon vorgelegt, anschliessend werden 182 µl Diisopropylethylamin und 0,08 g 1-(5-Chlorpyridin-2-yl)-piperazin zugefügt. Das Reaktionsgemisch wird bei 120°C, bis keine weitere Umsetzung erfolgt, in der Mikrowelle erhitzt. Das Produkt wird chromatographisch (präparative HPLC, Methode A) gereinigt. Analytische HPLC-MS (Methode B): RT = 1,09 min.

### 9. SYNTHESE VON {2-[4-(5-CHLORPYRIDIN-2-YL)-PIPERAZIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.9)

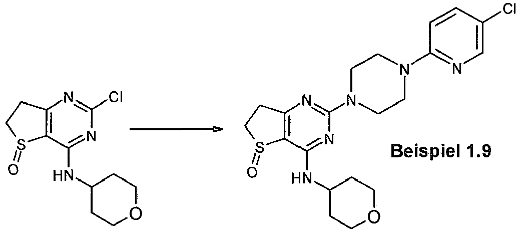

Ausgehend von 0,11 g **(IV-3)** (siehe 4.2) und 0,083 g 1-(5-Chlorpyridin-2-yl)-piperazin werden 0,14 g **Beispiel 1.9** analog zu Beispiel 1.8 (siehe 8.5) hergestellt und aufgereinigt. Analytische HPLC-MS (Methode B): RT = 1,14 min.

### 10. SYNTHESE VON {2-[4-(3-DIMETHYLAMINOPYRIDAZIN-4-YL)-PIPERAZIN-1-YL]-5-OXO-6,7-DIHYDRO-5H-5λ⁴-THIENO[3,2-D]PYRIMIDIN-4-YL}-(3-FLUORPHENYL)-AMIN (BEISPIEL 1.10)

### 10.1 3,4,6-Trichlorpyridazin

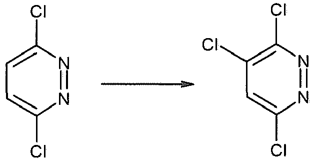

44 g 3,6-Dichlorpyridazin und 22 g Aluminiumtrichlorid werden auf 140°C erwärmt. Bei dieser Temperatur werden 10,6 l Chlor über 4 Stunden in das Reaktionsgemisch eingeleitet. Nach dem Abkühlen wird das Produkt mit Toluol extrahiert, mit einer 10%igen Natriumchlorid Lösung gewaschen und destilliert (Kp = 127 - 129°C). Es werden 44,1 g Produkt erhalten.

### 10.2 3,6-Dichlor-4-piperazin-1-yl-pyridazin

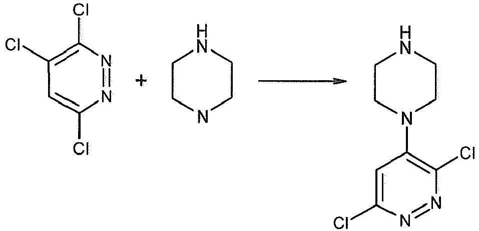

18 g 3,4,6-Trichlor-pyridazin und 34 g Piperazin werden in 100 ml Ethanol suspendiert und bei Raumtemperatur 30 Minuten gerührt. Der ausgefallene Feststoff wird abgesaugt. 500 ml Wasser werden zu der Mutterlauge zugegeben und das ausgefallene Produkt wird abgesaugt. 14 g Produkt werden als Feststoff erhalten. Smp = 111-115°C.

### 10.3 (6-Chlor-4-piperazin-1-yl-pyridazin-3-yl)-dimethylamin

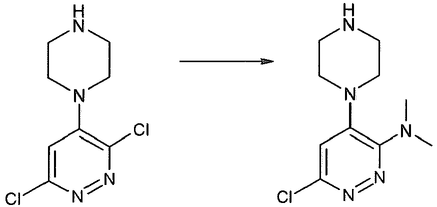

23 g 3,6-Dichlor-4-piperazin-1-yl-pyridazin und 45 g Dimethylamin werden in 200 ml Methanol suspendiert und 4 Stunden bei 100°C autoklaviert. Das Reaktionsgemisch wird zur Trockne eingedampft und das Produkt mit Chloroform extrahiert und mit Natronlauge gewaschen. Das Hydrochlorid wird mit einer etherischen HCl Lösung gefällt. 27 g Produkt werden erhalten. Smp = 291 °C.

### 10.4 Dimethyl-(4-piperazin-1-yl-pyridazin-3-yl)-amin (V-1)

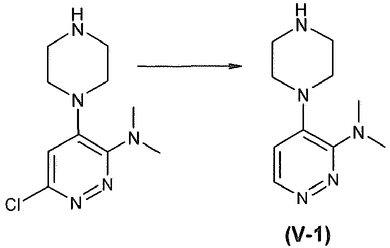

9,4 g (6-Chlor-4-piperazin-1-yl-pyridazin-3-yl)-dimethylamin Hydrochlorid und 7,3 g Natriumacetat werden in 150 ml Methanol suspendiert und mit 1 g Pd/C 10% bei Raumtemperatur hydriert. Der Katalysator wird abgesaugt, das Filtrat zur Trockne eingedampft und das Produkt mit Chloroform extrahiert und mit Natronlauge gewaschen. Das Hydrochlorid wird mit einer etherischen HCl Lösung gefällt. 7 g **(V-1)** werden erhalten. Smp = 335°C.

### 10.5 {2-[4-(3-Dimethylaminopyridazin-4-yl)-piperazin-1-yl]-5-oxo-6,7-dihydro-5H-5λ⁴-thieno[3,2-d]pyrimidin-4-yl)-(3-fluorphenyl)-amin (Beispiel 1.10)

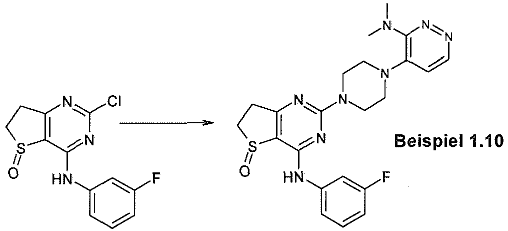

**(IV-1)** (siehe 1.2) (0,1 mmol) wird in 750 µl N-Methyl-2-pyrrolidon (NMP) und 50 µl Diisopropylethylamin vorgelegt, mit einer Lösung von **(V-1)** (0,1 mmol) in 400µl NMP versetzt und 30 min bei 120°C in der Mikrowelle erhitzt. Anschliessend werden 600 µL DMF zugesetzt, die Reaktionslösung wird chromatographisch (präparative HPLC-MS, Methode A) aufgereinigt und die Produktfraktionen gefriergetrocknet. **Beispiel 1.10** wird als Trifluoracetat erhalten. Analytische HPLC-MS (Methode C): RT = 1,61 min.

### 11. SYNTHESE VON 6-CHLOR-4-{4-[4-(3-FLUORPHENYLAMINO)-5-OXO-6,7-DIHYDRO-5H-5λ⁴-THIENO[3,2-d]PYRIMIDIN-2-YL]-PIPERAZIN-1-YL}-PYRIDAZIN-3-OL (BEISPIEL 1.11)

### 11.1 (6-Chlor-4-piperazin-1-yl-pyridazin-3-yloxy)-ethanol

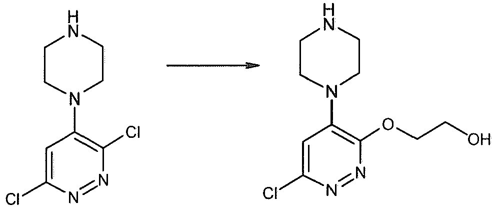

23 g 3,6-Dichlor-4-piperazin-1-yl-pyridazin (siehe 10.2) werden in 100 ml Ethylenglycol suspendiert und zu einer Suspension von 2,3 g Natrium in 100 ml Ethylenglycol getropft. Das Reaktionsgemisch wird 3 Stunden bei 100°C erhitzt und zur Trockne eingedampft. Der Rückstand wird in Acetonitril suspendiert und der Feststoff abgesaugt. Die Mutterlauge wird zur Trockne eingedampft, das Produkt mit Dichlormethan extrahiert und mit konz. NaOH gewaschen. Das Produkt wird in Ethanol suspendiert und als Fumarat mit Fumarsäure gefällt. 13 g Produkt werden erhalten. Smp = 179°C

### 11.2 6-Chlor-4-piperazin-1-yl-pyridazin-3-ol (V-2)

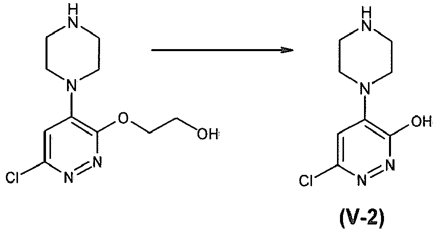

15 g (6-Chlor-4-piperazin-1-yl-pyridazin-3-yloxy)-ethanol Fumarat werden in 90 ml Bromwasserstoff (48%) suspendiert. Das Reaktionsgemisch wird 1 Stunde unter Rückfluß gerührt und zur Trockne eingedampft. 19 g Produkt werden als Hydrobromid erhalten. Smp = 35°C.

### 11.3 6-Chlor-4-{4-[4-(3-fluorphenylamino)-5-oxo-6,7-dihydro-5H-5λ⁴-thieno[3,2-d]pyrimidin-2-yl]-piperazin-1-yl}-pyridazin-3-ol (Beispiel 1.11)

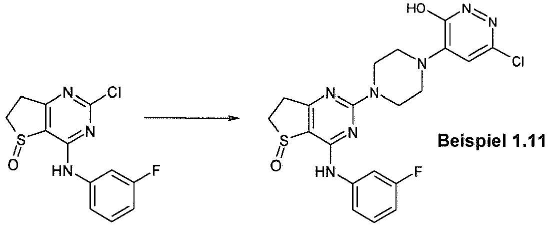

Ausgehend von **(IV-1)** (siehe 1.2) und **(V-2)** wird **Beispiel 1.11** analog zu Beispiel 1.10 (siehe 10.5) hergestellt und aufgereinigt. Analytische HPLC-MS (Methode C): RT = 1,86 min.

### 12. SYNTHESE VON (2-{4-[6-(2-ETHOXYETHOXY)-PYRIDAZIN-3-YL]-PIPERAZIN-1-YL}-5-OXO-6,7-DIHYDRO-5H-5λ⁴-THIENO[3,2-d]PYRIMIDIN-4-YL)-(3-FLUORPHENYL)-AMIN (BEISPIEL 1.12)

### 12.1 3-Ethoxyethoxy-6-piperazin-1-yl-pyridazin (V-3)

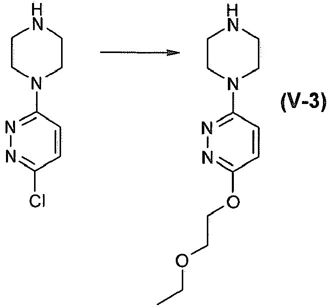

18 g 3-Chlor-6-piperazin-1-yl-pyridazin und 30 g Kaliumhydroxid in 30 ml Wasser werden in 180 ml Ethylglycol suspendiert und 4 Stunden unter Rückfluß gerührt. Das Reaktionsgemisch wird zur Trockne eingedampft. Das Produkt wird mit Diethylether extrahiert, einer konzentrierten Kaliumcarbonat Lösung gewaschen und destilliert (Kp = 190°C). 18 g **(V-3)** werden erhalten.

### 12.2 (2-{4-[6-(2-Ethoxyethoxy)-pyridazin-3-yl]-piperazin-1-yl}-5-oxo-6,7-dihydro-5H-5λ⁴-thieno[3,2-d]pyrimidin-4-yl)-(3-fluorphenyl)-amin (Beispiel 1.12)

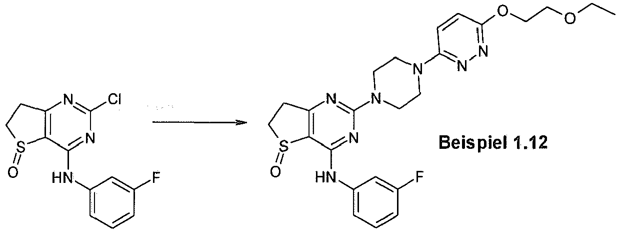

Ausgehend von **(IV-1)** (siehe 1.2) und **(V-3)** wird **Beispiel 1.12** als Trifluoracetat analog zu Beispiel 1.10 (siehe 10.5) hergestellt und aufgereinigt. Analytische HPLC-MS (Methode C): RT = 1,66 min.

### 13. SYNTHESE VON (3-FLUORPHENYL)-[5-OXO-2-(4-PYRIDAZIN-4-YL-PIPERAZIN-1-YL)-6,7-DIHYDRO-5H-5λ⁴-THIENO[3,2-d]PYRIMIDIN-4-YL]-AMIN (BEISPIEL 1.13)

### 13.1 4-Piperazin-1-yl-pyridazin (V-4)

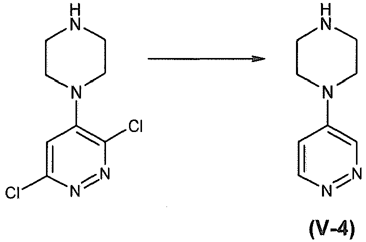

9,3 g 3,6-Dichlor-4-piperazin-1-yl-pyridazin (siehe 10.2) und 6,5 g Natriumacetat werden in 100 ml Methanol suspendiert und mit 1 g Pd/C 10% und Raumtemperatur hydriert. Der Katalysator wird abgesaugt und das Filtrat zur Trockne eingedampft. Das Produkt wird mit Chloroform extrahiert, mit Natronlauge gewaschen und als Hydrochlorid mit einer etherischen HCl Lösung gefällt. 8,6 g **(V-4)** werden erhalten. Smp > 300°C.

### 13.2 (3-Fluorphenyl)-[5-oxo-2-(4-pyridazin-4-yl-piperazin-1-yl)-6,7-dihydro-5H-5λ⁴-thieno[3,2-d]pyrimidin-4-yl)-amin (Beispiel 1.13)

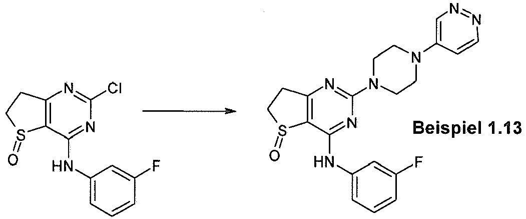

Ausgehend von **(IV-1)** (siehe 1.2) und **(V-4)** wird **Beispiel 1.13** als Trifluoracetat analog zu Beispiel 1.10 (siehe 10.5) hergestellt und aufgereinigt. Analytische HPLC-MS (Methode C): RT = 1,54 min.

### 14. SYNTHESE VON (R)-2-{2-[4-(4-METHOXY-1-METHYL-1H-BENZIMIDAZOL-2-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL TRIFLUORACETAT (BEISPIEL 1.14)

### 14.1 (3-Methoxy-2-nitrophenyl)-carbamidsäure tert-butylester

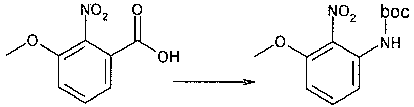

10 g 3-Methoxy-2-nitrobenzoesäure und 7 ml Triethylamin werden in 100 ml *tert*-Butanol vorgelegt und 11 ml Diphenylphosphorylazid zugetropft. Das Reaktionsgemisch wird dann 6 Stunden unter Rückfluß gerührt und zur Trockne eingedampft. Das Produkt wird mit Essigester extrahiert, mit einer10% igen Zitronensäure, einer gesättigten Natriumhydrogencarbonat und einer gesättigten Natriumchlorid Lösung gewaschen. 12,4 g Produkt werden als Feststoff werden erhalten. Smp = 90°C.

### 14.2 (3-Methoxy-2-nitrophenyl)-methyl-carbamidsäure tert-butylester

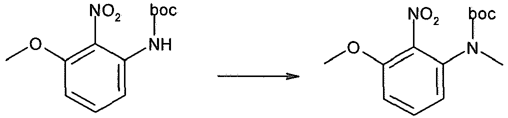

12,2 g (3-Methoxy-2-nitrophenyl)-methyl-carbamidsäure *tert*-butylester werden in 80 ml Dimethylformamid vorgelegt und bei 0°C gekühlt. 2,4 g Natriumhydrid (50% ig in Mineralöl) werden langsam zugegeben. Das Reaktionsgemisch wird 30 Minuten bei 0°C gerührt. Dann werden 3,1 ml Methyliodid zugetropft. Das Reaktionsgemisch wird 2 Stunden bei Raumtemperatur gerührt und mit Wasser versetzt. Das Produkt wird mit Essigester extrahiert. 12.5 g Produkt werden als Öl erhalten.

### 14.3 (3-Methoxy-2-nitrophenyl)-methylamin

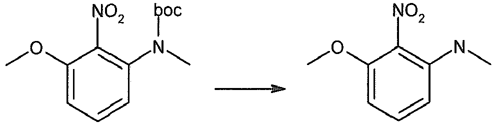

12,5 g (3-Methoxy-2-nitrophenyl)-methyl-carbamidsäure *tert*-butylester und 78 ml Salzsäure (4 M) werden in 300 ml Essigester suspendiert und 5 Stunden bei 60°C erhitzt. Das Reaktionsgemisch wird zur Trockne eingedampft, der Rückstand mit einer gesättigten Natriumhydrogencarbonat Lösung versetzt und das Produkt mit Essigester extrahiert. 7,5 g Produkt werden als Feststoff erhalten. Smp = 58-59°C.

### 14.4 3-Methoxy-N¹-methylbenzol-1,2-diamin

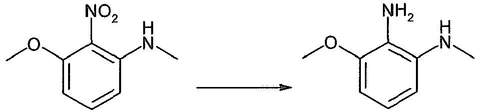

7,4 g (3-Methoxy-2-nitrophenyl)-methylamin werden in 150 ml Essigester suspendiert und mit 1 g Pd/C 10% bei einem Druck von 50 psi und Raumtemperatur hydriert. Nach 4,5 Stunden wird der Katalysator abgesaugt und das Filtrat zur Trockne eingdampft. 5,9 g des Produktes werden als Öl erhalten.

### 14.5 4-Methoxy-1-methyl-1,3-dihydrobenzimidazol-2-on

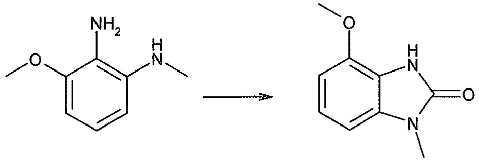

5,9 g 3-Methoxy-N1-methylbenzol-1,2-diamin werden in 70 ml Tetrahydrofuran suspendiert und 6,3 g N,N'-Carbonyldiimidazol zugegeben. Das Reaktionsgemisch wird 5 Stunden bei Raumtemperatur gerührt, mit Wasser versetzt und das Produkt mit Essigester extrahiert. 3,9 g Produkt als Feststoff werden erhalten.

### 14.6 2-Chlor-4-methoxy-1-methyl-1H-benzimidazol

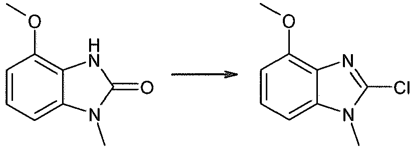

3,7 g 4-Methoxy-1-methyl-1,3-dihydrobenzimidazol-2-on werden in 15 ml Phosphoroxychlorid suspendiert. Das Reaktionsgemisch wird 3 Stunden unter Rückfluß gerührt, langsam mit Eiswasser versetzt und mit konz. Ammoniak alkalisch gestellt. Das ausgefallene Produkt wird abgesaugt. 3,6 g Produkt werden als Feststoff erhalten. Smp = 118 - 119°C.

### 14.7 4-Methoxy-1-methyl-2-piperazin-1-yl-1--benzimidazol (V-5)

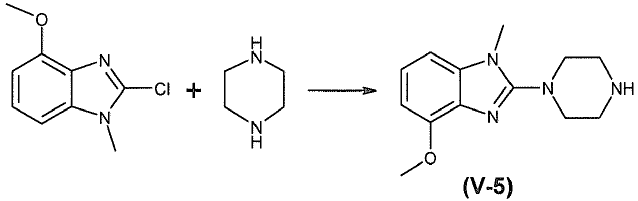

2 g 2-Chlor-4-methoxy-1-methyl-1*H*-benzimidazol und 4,4 g Piperazin werden in 20 ml n-Butanol suspendiert und 5 Stunden unter Rückfluß gerührt. Das Reaktionsgemisch wird zur Trockne eingedampft und das Produkt chromatographisch (Kieselgel, Dichlormethan/ Methanol 10:1) gereinigt. 1,6 g **(V-5)** werden als Feststoff erhalten. Smp = 147°C.

### 14.8 (R)-2-{2-[4-(4-Methoxy-1-methyl-1H-benzimidazol-2-yl)-piperazin-1-yl]-5-oxo-6,7-dihydro-5H-5λ⁴-thieno[3,2-d]pyrimidin-4-ylamino}-3-methylbutan-1-ol (Beispiel 1.14)

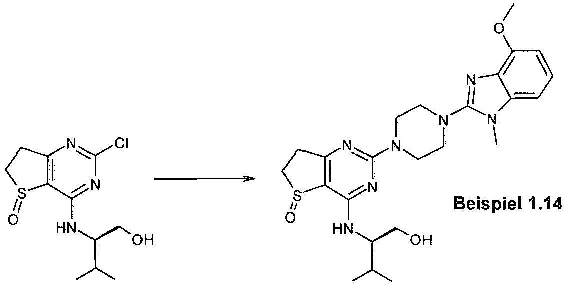

Ausgehend von **(IV-2)** (siehe 2.2) und **(V-5)** wird **Beispiel 1.14** als trifluoracetat analog zu Beispiel 1.10 (siehe 10.5) hergestellt und aufgereinigt. Analytische HPLC-MS (Methode C): RT = 1,5 min.

### 15. SYNTHESE VON (R)-2-{2-[4-(7-ETHYL-6,7,8,9-TETRAHYDRO-5H-PYRAZINO[2,3-d]AZEPIN-2-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL (BEISPIEL 1.15)

### 15.1 2-Chlor-7-ethyl-6,7,8,9-tetrahydro-5H-pyrazino[2,3-d]azepin

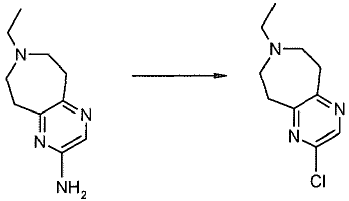

26,5 g 7-Ethyl-6,7,8,9-tetrahydro-5*H*-pyrazino[2,3-*d*]azepin-2-ylamin (US4409220) werden in 130 ml konz. Salzsäure suspendiert, mit 0,1 g Kupfer(I)bromid versetzt und auf -5°C abgekühlt. Eine Suspension von 11 g Natriumnitrit in 14 ml Wasser wird langsam zugetropft. Das Reaktionsgemisch wird 15 Stunden bei Raumtemperatur gerührt und fast zur Trockne eingedampft. Der Rückstand wird langsam auf Eiswasser und Kaliumcarbonat gegeben. Das Produkt wird mit Dichlormethan extrahiert und als Hydrochlorid mit einer etherischen HCl Lösung gefällt. 14,3 g Produkt werden erhalten. Smp = 258 - 262°C

### 15.2 7-Ethyl-2-piperazin-1-yl-6,7,8,9-tetrahydro-5H-pyrazino[2,3-d]azepin (V-6)

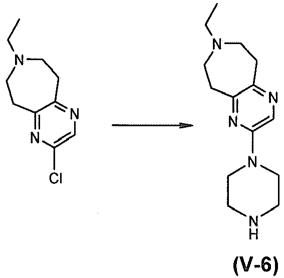

3 g 2-Chlor-7-ethyl-6,7,8,9-tetrahydro-5*H*-pyrazino[2,3-d]azepin werden mit 23,3 g Piperazin versetzt und 5 Stunden bei 145°C erhitzt. Überschüssiges Piperazin wird abdestilliert und der Rückstand mit Dichlormethan und Methanol behandelt. Ausgefallenes Produkt wird abgesaugt und chromatographisch (Alox, Dioxan/Toluol/Methanol/ NH₄OH 50/20/20/2) gereinigt. 1,95 g Produkt werden erhalten.

### 15.3 (R)-2-{2-[4-(7-Ethyl-6,7,8,9-tetrahydro-5H-pyrazino[2,3-d]azepin-2-yl)-piperazin-1-yl]-5-oxo-6,7-dihydro-5H-5λ⁴-thieno[3,2-d]pyrimidin-4-ylamino}-3-methylbutan-1-ol (Beispiel 1.15)

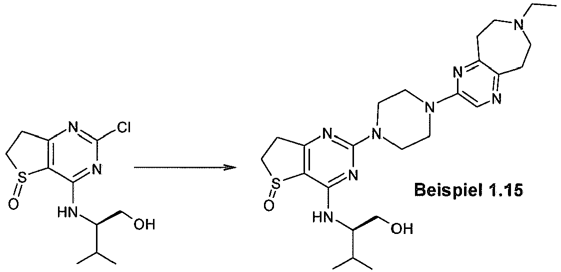

Ausgehend von **(IV-2)** (siehe 2.2) und **(V-6)** wird **Beispiel 1.15** als Trifluoracetat analog zu Beispiel 1.10 (siehe 10.5) hergestellt und aufgereinigt. Analytische HPLC-MS (Methode C): RT = 1,38 min.

### 16. SYNTHESE VON (R)-3-METHYL-2-[5-OXO-2-(4-PYRIMIDIN-4-YL-PIPERAZIN-1-YL)-6,7-DIHYDRO-5H-5λ⁴-THIENO[3,2-d]PYRIMIDIN-4-YLAMINO]-BUTAN-1-OL (BEISPIEL 1.16)

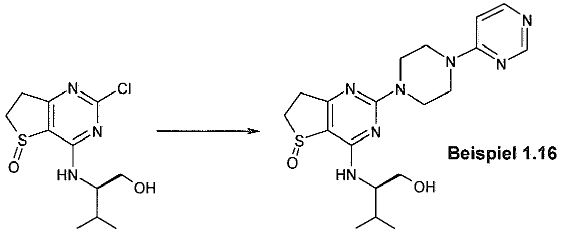

Ausgehend von **(IV-2)** (siehe 2.2) und 4-Piperazin-1-yl-pyrimidin (J. Org. Chem. 1953, 1484) wird **Beispiel 1.16** als Trifluoracetat analog zu Beispiel 1.10 (siehe 10.5) hergestellt und aufgereinigt. Analytische HPLC-MS (Methode C): RT = 1,31 min.

### 17. SYNTHESE VON 4-{4-[4-((R)-1-HYDROXYMETHYL-2-METHYLPROPYLAMINO)-5-OXO-6,7-DIHYDRO-5H-5λ⁴-THIENO[3,2-d]PYRIDIN-2-YL]-PIPERAZIN-1-YL}-PYRIDIN-2-OL (BEISPIEL 1.17)

### 17.1 4-(1-Oxypyridin-4-yl)-piperazin-1-BOC:

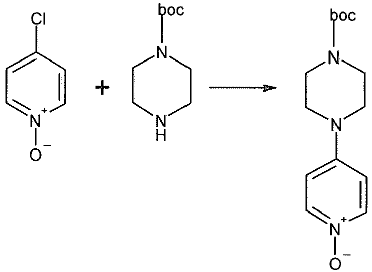

3 g 4-Chlorpyridin-N-oxid und 13,2 g Piperazin-1-BOC werden bei 90°C 4 Stunden erhitzt. Das Produkt wird chromatographisch (Kieselgel, Dichlormethan/Methanol/Ammoniak 90/10/1) gereinigt. 2,9 g Produkt werden als Feststoff erhalten.

### 17.2 4-(2-Hydroxypyridin-4-yl)-piperazin-1-BOC

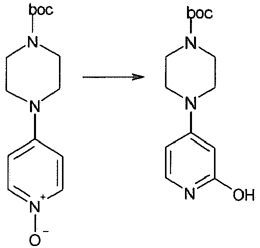

1,75 g 4-(1-Oxypyridin-4-yl)-piperazin-1-BOC werden in 15 ml Essigsäureanhydrid suspendiert und 24 h bei 150°C erhitzt. Das Reaktionsgemisch wird zur Trockne eingedampft und das Produkt chromatographisch (Kieselgel, Essigester/Methanol/Ammoniak 95/5/0,5) gereinigt. 0,51 g Produkt werden als Feststoff erhalten

### 17.3 4-Piperazin-1-yl-pyridin-2-ol (V-7)

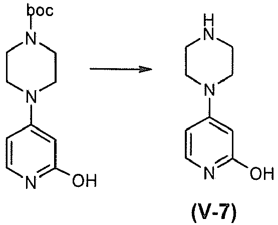

0,51 g 4-(2-Hydroxypyridin-4-yl)-piperazin-1-BOC und 2 ml Trifluoressigsäure werden in 15 ml Dichlormethan suspendiert und 2 Stunden bei Raumtemperatur gerührt. Das Reaktionsgemisch wird zur Trockne eingedampft. 1 g **(V-7)** werden als Öl erhalten. ¹H NMR (400 MHz, DMSO): 7,30 (1H, d); 5,99 (1H, dd); 5,34 (1H, d).

### 17.4 4-{4-[4-((R)-1-Hydroxymethyl-2-methylpropylamino)-5-oxo-6,7-dihydro-5H-5λ⁴-thieno[3,2-d]pyrimidin-2-yl]-piperazin-1-yl}-pyridin-2-ol (Beispiel 1.17)

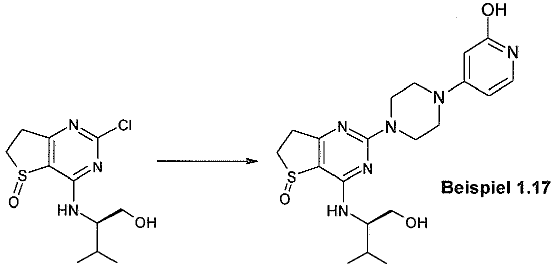

Ausgehend von **(IV-2)** (siehe 2.2) und **(V-7)** wird **Beispiel 1.17** analog zu Beispiel 1.10 (siehe 10.5) hergestellt und aufgereinigt. Analytische HPLC-MS (Methode C): RT = 1,37 min.

### 18. SYNTHESE VON (R)-3-METHYL-2-[5-OXO-2-(4-PYRIDIN-4-YL-PIPERAZIN-1-YL)-6,7-DIHYDRO-5H-5λ⁴-THIENO[3,2-d]PYRIMIDIN-4-YLAMINO]-BUTAN-1-OL TRIFLUORACETAT (BEISPIEL 1.18)

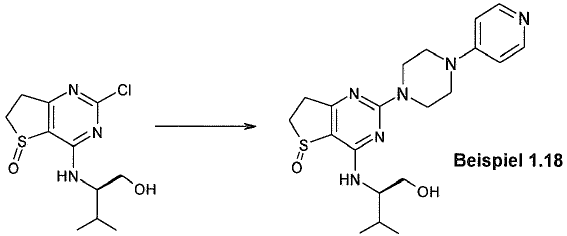

Ausgehend von **(IV-2)** (siehe 2.2) und 1-Pyridin-4-yl-piperazin wird **Beispiel 1.18** als Trifluoracetat analog zu Beispiel 1.10 (siehe 10.5) hergestellt und aufgereinigt. Analytische HPLC-MS (Methode C): RT = 1,33 min.

### 19. SYNTHESE VON (R)-2-{2-[4-(3-DIMETHYLAMINOPYRIDAZIN-4-YL)-PIPERAZIN-1-YL]-5-OXO-6,7-DIHYDRO-5H-5λ⁴-THIENO[3,2-d]PYRIMIDIN-4-YLAMINO}-3-METHYLBUTAN-1-OL (BEISPIEL 1.19)

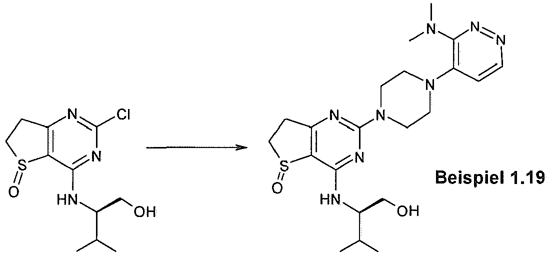

Ausgehend von **(IV-2)** (siehe 2.2) und **(V-1)** (siehe 10.4) wird **Beispiel 1.19** als Trifluoracetat analog zu Beispiel 1.10 (siehe 10.5) hergestellt und aufgereinigt. Analytische HPLC-MS (Methode C): RT = 1,37 min.

### 20. SYNTHESE VON 6-CHLOR-4-{4-[4-((R)-1-HYDROXYMETHYL-2-METHYL-PROPYLAMINO)-5-OXO-6,7-DIHYDRO-5H-5λ⁴-THIENO[3,2-d]PYRIMIDIN-2-YL]-PIPERAZIN-1-YL}-PYRIDAZIN-3-OL (BEISPIEL 1.20)

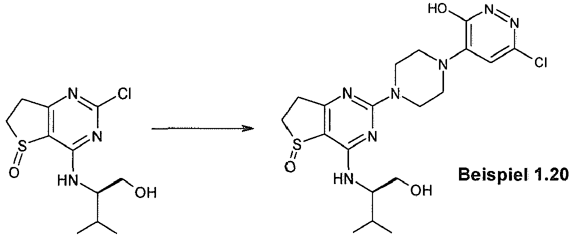

Ausgehend von **(IV-2)** (siehe 2.2) und **(V-2)** (siehe 11.2) wird **Beispiel 1.20** analog zu Beispiel 1.10 (siehe 10.5) hergestellt und aufgereinigt. Analytische HPLC-MS (Methode C): RT = 1,55 min.

### 21. SYNTHESE VON (R)-2-(2-{4-[6-(2-ETHOXYETHOXY)-PYRIDAZIN-3-YL]-PIPERAZIN-1-YL}-5-OXO-6,7-DIHYDRO-5H-5λ⁴-THIENO[3,2-d]PYRIMIDIN-4-YLAMINO)-3-METHYLBUTAN-1-OL (BEISPIEL 1.21)

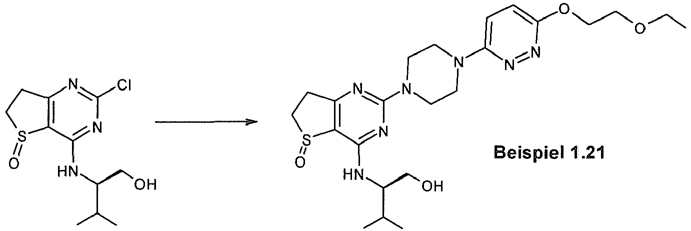

Ausgehend von **(IV-2)** (siehe 2.2) und **(V-3)** (siehe 12.1) wird **Beispiel 1.21** als Trifluoracetat analog zu Beispiel 1.10 (siehe 10.5) hergestellt und aufgereinigt. Analytische HPLC-MS (Methode C): RT = 1,45 min.

### 22. SYNTHESE VON (R)-3-METHYL-2-[5-OXO-2-(4-PYRIDAZIN-4-YL-PIPERAZIN-1-YL)-6,7-DIHYDRO-5H-5λ⁴-THIENO[3,2-d]PYRIMIDIN-4-YLAMINO]-BUTAN-1-OL (BEISPIEL 1.22)

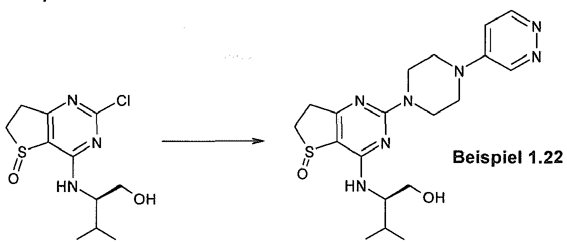

Ausgehend von **(IV-2)** (siehe 2.2) und **(V-4)** (siehe 13.1) wird **Beispiel 1.22** als Trifluoracetat analog zu Beispiel 1.10 (siehe 10.5) hergestellt und aufgereinigt. Analytische HPLC-MS (Methode C): RT = 0,56 min.

### 23. SYNTHESE VON {1-[5-OXO-2-(4-PYRIMIDIN-4-YL-PIPERAZIN-1-YL)-6,7-DI-HYDRO-5H-5λ⁴-THIENO[3,2-d]PYRIMIDIN-4-YLAMINO]-CYCLOPROPYL}-METHANOL TRIFLUORACETAT (BEISPIEL 1.23)

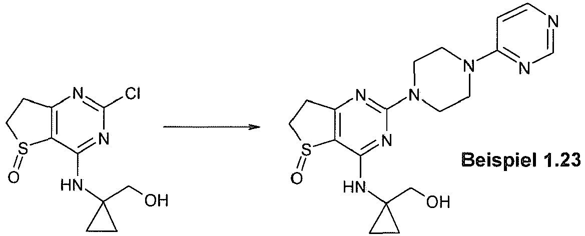

Ausgehend von **(IV-4)** (siehe 8.4) und 4-Piperazin-1-yl-pyrimidin (J. Org. Chem. 1953, 1484) wird **Beispiel 1.23** als Trifluoracetat analog zu Beispiel 1.10 (siehe 10.5) hergestellt und aufgereinigt. Analytische HPLC-MS (Methode C): RT = 1,29 min.

### 24 SYNTHESE VON {1-[5-OXO-2-(4-PYRIDIN-4-YL-PIPERAZIN-1-YL)-6,7-DIHYDRO-5H-5λ⁴-THIENO[3,2-d]PYRIMIDIN-4-YLAMINO]-CYCLOPROPYL}-METHANOL TRIFLUORACETAT (BEISPIEL 1.24)

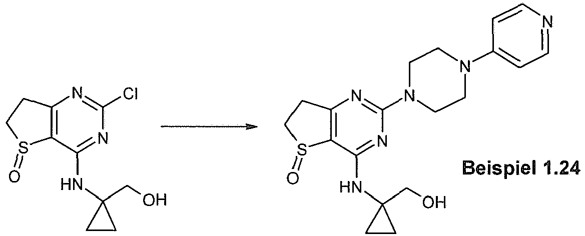

Ausgehend von **(IV-4)** (siehe 8.4) und 1-Pyridin-4-yl-piperazin wird **Beispiel 1.24** als Trifluoracetat analog zu Beispiel 1.10 (siehe 10.5) hergestellt und aufgereinigt. Analytische HPLC-MS (Methode C): RT = 1,29 min.

### 25. SYNTHESE VON (S)-1-METHYL-5-[5-OXO-2-(4-PYRIMIDIN-4-YL-PIPERAZIN-1-YL)-6,7-DIHYDRO-5H-5λ⁴-THIENO[3,2-d]PYRIMIDIN-4-YLAMINO]-PIPERIDIN-2-ON (BEISPIEL 1.25)

### 25.1 (S)-5-Dibenzylaminopiperidin-2-on:

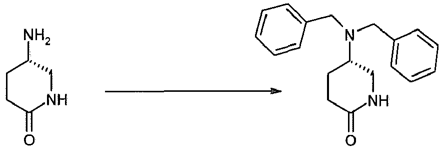

0,600 g 4-(S)-Amino-delta-valerolactam Hydrochlorid, 0,970 ml Benzylbromid und 1,5 g Natriumhydrogencarbonat werden in 30 ml Ethanol suspendiert. Das Reaktionsgemisch wird dann 8 Stunden bei 80°C gerührt und anschießend zur Trockne eingedampft. Der Rückstand wird in Wasser suspensiert und das Produkt mit Dichlormethan extrahiert und chromatographisch (Kieselgel, Dichlormethan/Methanol 100/0 bis 95/5) gereinigt. 0,500 g des Produktes werden als Öl erhalten. Analytische HPLC-MS (Methode A): RT = 1,01 min.

### 25.2 (S)-5-Dibenzylamino-1-methylpiperidin-2-on:

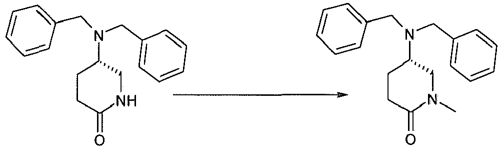

0,500 g (S)-5-Dibenzylaminopiperidin-2-on werden in 15 ml Tetrahydrofuran suspendiert. Unter Eisbadkühlung werden 0,175 g Kalium-*tert*-butylat zugegeben. Das Reaktionsgemisch wird dann 30 Minuten bei Raumtemperatur gerührt. Unter Eisbadkühlung werden 0,095 ml Methyliodid zugegeben. Das Reaktionsgemisch wird dann 48 Stunden bei Raumtemperatur gerührt und anschließend mit einer gesättigten NaCl Lösung versetzt. Das Produkt wird mit Essigester extrahiert. 0,450 g des Produktes werden als Öl erhalten. Analytische HPLC-MS (Methode A): RT = 1,07 min.

### 25.3 (S)-5-Amino-1-methylpiperidin-2-on:

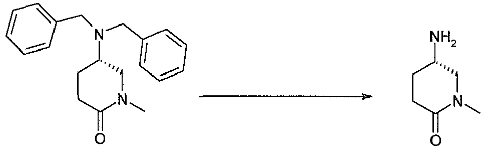

0,450 g (S)-5-Dibenzylamino-l-methylpiperidin-2-on werden in 25 ml Methanol suspendiert und mit 0,150 g Pd/C 10% bei einem Druck von 3 bar und einer Temperatur von 60 °C hydriert. Nach 16 Stunden wird der Katalysator abgesaugt und das Filtrat zur Trockne eingedampft. 0,190 g des Produktes werden als Öl erhalten. ¹H NMR (400 MHz, DMSO): 2,76 (3H, s).

### 25.4 (S)-5-(2-Chlor-6,7-dihydrothieno[3,2-d]pyrimidin-4-ylamino)-1-methylpiperidin-2-on (III-5):

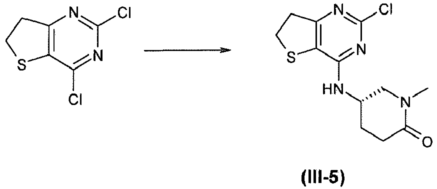

0,27 g **(II)** werden in 3 ml Dioxan vorgelegt, anschliessend werden 0,45 ml Diisopropylethylamin und 0,25 g (S)-5-Amino-l-methylpiperidin-2-on zugegeben. Das Reaktionsgemisch wird bei 130°C, bis keine weitere Umsetzung erfolgt, erhitzt und nach Abkühlen eingedampft. Das Produkt wird mit Dichlormethan extrahiert und chromatographisch (präparative HPLC, Methode B) gereinigt. 0,26 g **(III-5)** werden als Feststoff erhalten. Analytische HPLC-MS (Methode A): RT = 1,06 min.

### 25.5 (S)-5-(2-Chlor-5-oxo-6,7-dihydro-5H-5λ⁴-thieno[3,2-d]pyrimidin-4-ylamino)-1-methylpiperidin-2-on (IV-5):

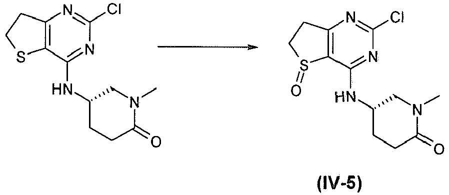

0,04 g S-(-)-1,1'-Bi-2-naphtol werden in 5 ml Chloroform unter Argon vorgelegt, dann 0,02 ml Titan(IV)-isopropylat und 0,025 ml Wasser zugegeben. Das Reaktionsgemisch wird 1 Stunde bei Raumtemperatur gerührt. Anschließend wird eine Suspension von 0,2 g **(III-5)** in 4 ml Dichlormethan zugegeben. Das Reaktionsgemisch wird auf -5°C abgekühlt und nach 20 Minuten werden 0,12 ml tert-Butylhydroperoxid 5-6 M in Decan zugetropft. Das Reaktionsgemisch wird bei -5 °C, bis keine weitere Umsetzung erfolgt, weitergerührt und mit NH₄OH basisch gestellt. Das Produkt wird mit Dichlormethan extrahiert und chromatographisch (Kieselgel, Essigester/Methanol 100/0 bis 60/40) gereinigt. 0,09 g **(IV-5)** werden als Feststoff erhalten. Analytische HPLC-MS (Methode A): RT = 0,83 min.

### 25.6 (S)-1-Methyl-5-[5-oxo-2-(4-pyrimidin-4-yl-piperazin-1-yl)- 6,7-dihydro-5H-5λ⁴-thieno[3,2-d]pyrimidin-4-ylamino]-piperidin-2-on (Beispiel 1.25)

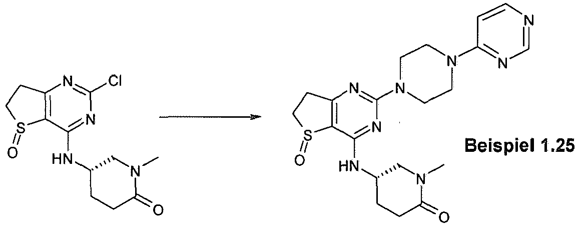

Ausgehend von **(IV-5)** und 4-Piperazin-1-yl-pyrimidin (J. Org. Chem. 1953, 1484) wird **Beispiel 1.25** als Trifluoracetat analog zu Beispiel 1.10 (siehe 10.5) hergestellt und aufgereinigt. Analytische HPLC-MS (Methode C): RT = 1,28 min.

### 26. SYNTHESE VON {2-[4-(5-FLUOR-1-METHYL-1H-BENZIMIDAZOL-2-YL)-PIPERAZIN-1-YL]-5-OXO-6,7-DIHYDRO-5H-5λ⁴-THIENO[3,2-d]PYRIMIDIN-4-YL}-(TETRAHYDROPYRAN-4-YL)-AMIN (BEISPIEL 1.26)

### 26.1 (4-Fluor-2-nitrophenyl)-methylamin

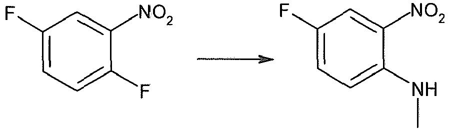

Zu einer 30 ml 40%igen wässrigen Methylamin Lösung werden 7,3 g 1,4-Difluor-2-nitrobenzol unter Eiskühlung langsam zugegeben und das Reaktionsgemisch 1 Stunde bei Raumtemperatur gerührt. Das ausgefallene Produkt wird abgesaugt und aus Wasser und Ethanol umkristallisiert. 6,3 g Produkt werden als Feststoff erhalten. Smp = 74-76°C.

### 26.2 4-Fluor-N¹-methylbenzol-1,2-diamin

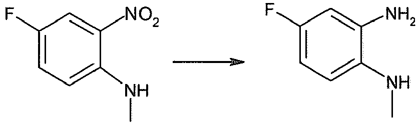

6,2 g (4-Fluor-2-nitrophenyl)-methylamin werden in 200 ml Essigester suspendiert und mit 1 g Raney-Nickel bei einem Druck von 5 bar und Raumtemperatur hydriert. Nach 4,5 Stunden wird der Katalysator abgesaugt und das Filtrat zur Trockne eingedampft. 3,9 g Produkt werden als Öl erhalten.

### 26.3 5-Fluor-1-methyl-1,3-dihydrobenzimidazol-2-on

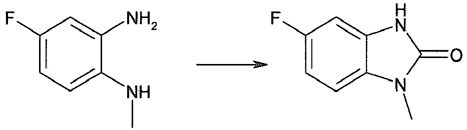

6 g 4-Fluor-N1-methylbenzol-1,2-diamin werden in 200 ml Tetrahydrofuran suspendiert und 7,1 g N,N'-Carbonyldiimidazol zugegeben. Das Reaktionsgemisch wird 48 Stunden bei Raumtemperatur gerührt und das ausgefallene Produkt abgesaugt und aus Dioxan umkristallisiert. 3,9 g Produkt werden als Feststoff erhalten. Smp = 207°C.

### 26.4 2-Chlor-5-fluor-1-methyl-1H-benzimidazol

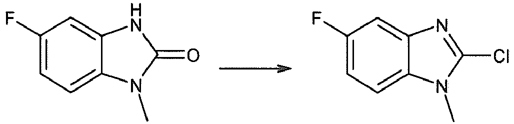

3,9 g 5-Fluor-1-methyl-1,3-dihydro-benzimidazol-2-on werden in 80 ml Phosphoroxychlorid suspendiert und das Reaktionsgemisch 2 Stunden unter Rückfluß gerührt. 50 ml Diethylanilin werden zugegeben. Das Reaktionsgemisch wird weitere 10 Minuten unter Rückfluß gerührt und langsam mit Eiswasser versetzt. Das Produkt wird mit Dichlormethan extrahiert und chromatographisch (Kieselgel, Cyclohexan, Methylenchlorid/Aceton 20/1) gereinigt. 1,4 g Produkt werden als Feststoff erhalten. Smp = 138-141°C.

### 26.5 5-Fluor-1-methyl-2-piperazin-1-yl-1H-benzimidazol (V-8)

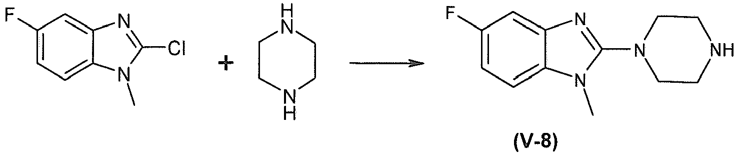

0,7 g 2-Chlor-5-fluor-1-methyl-1*H*-benzimidazol und 1,3 g Piperazin werden in 10 ml n-Butanol suspendiert und 48 Stunden bei Raumtemperatur gerührt. Das Reaktionsgemisch wird zur Trockne eingedampft und das Produkt chromatographisch (Aluminiumoxid, Methylenchlorid/Methanol 10/1) gereinigt. 0,73 g **(V-8)** werden als Feststoff erhalten. ¹H NMR (400 MHz, DMSO): 6,9 (1H, t); 3,6 (3H, s).

### 26.6 {2-[4-(5-Fluor-1-methyl-1H-benzoimidazol-2-yl)-piperazin-1-yl]-5-oxo-6,7-dihydro-5H-5λ⁴-thieno[3,2-d]pyrimidin-4-yl}-(tetrahydropyran-4-yl)-amin (Beispiel 1.26)

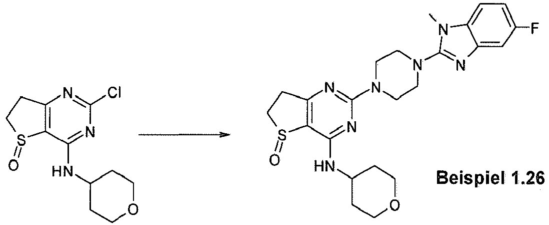

Ausgehend von **(IV-3)** (siehe 4.2) und **(V-8)** wird **Beispiel 1.26** analog zu Beispiel 1.10 (siehe 10.5) hergestellt und aufgereinigt. Analytische HPLC-MS (Methode C): RT = 1,48 min.

### 27. SYNTHESE VON [5-OXO-2-(4-PYRIDIN-4-YL-PIPERAZIN-1-YL)-6,7-DIHYDRO-5H-5λ⁴-THIENO[3,2-d]PYRIMIDIN-4-YL]-(TETRAHYDROPYRAN-4-YL)-AMIN (BEISPIEL 1.27)

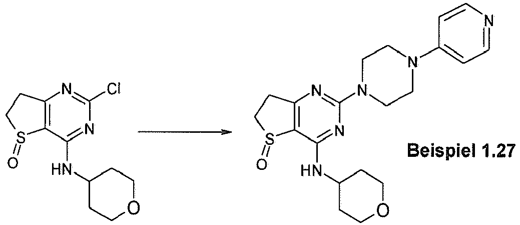

Ausgehend von **(IV-3)** (siehe 4.2) und 1-Pyridin-4-yl-piperazin wird **Beispiel 1.27** als Trifluoracetat analog zu Beispiel 15 (siehe 10.5) hergestellt und aufgereinigt. Analytische HPLC-MS (Methode C): RT = 1,32 min.

### 28. SYNTHESE VON (3-FLUORPHENYL)-{2-[4-(4-METHOXY-1-METHYL-1H-BENZIMIDAZOL-2-YL)-PIPERAZIN-1-YL]-5-OXO-6,7-DIHYDRO-5H-5λ⁴-THIENO[3,2-d]PYRIMIDIN-4-YL}-AMIN (BEISPIEL 1.28)

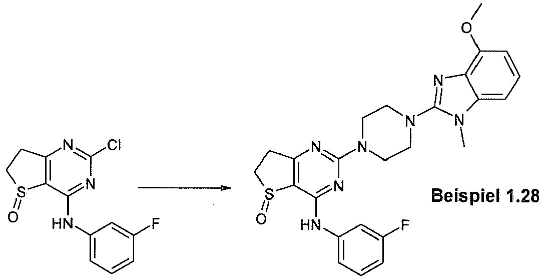

Ausgehend von **(IV-1)** (siehe 1.2) und **(V-5)** (siehe 14.7) wird **Beispiel 1.28** als Trifluoracetat analog zu Beispiel 1.10 (siehe 10.5) hergestellt und aufgereinigt. Analytische HPLC-MS (Methode C): RT = 1,73 min.

### 29. SYNTHESE VON {2-[4-(7-ETHYL-6,7,8,9-TETRAHYDRO-5H-PYRAZINO[2,3-d]AZEPIN-2-YL)-PIPERAZIN-1-YL]-5-OXO-6,7-DIHYDRO-5H-5λ⁴-THIENO[3,2-d]PYRIMIDIN-4-YL}-(3-FLUORPHENYL)-AMIN (BEISPIEL 1.29)

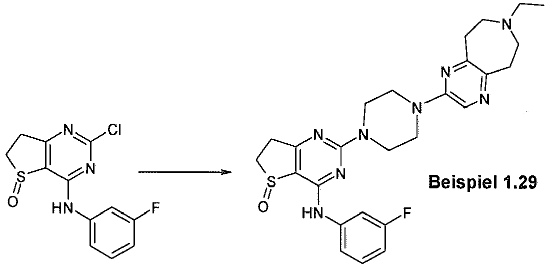

Ausgehend von **(IV-1)** (siehe 1.2) und **(V-6)** (siehe 15.2) wird **Beispiel 1.29** als Trifluoracetat analog zu Beispiel 1.10 (siehe 10.5) hergestellt und aufgereinigt. Analytische HPLC-MS (Methode C): RT = 1,6 min.

### 30. SYNTHESE VON (3-FLUORPHENYL)-[5-OXO-2-(4-PYRIMIDIN-4-YL-PIPERAZIN-1-YL)-6,7-DIHYDRO-5H-5λ⁴-THIENO[3,2-d]PYRIMIDIN-4-YL]-AMIN (BEISPIEL 1.30)

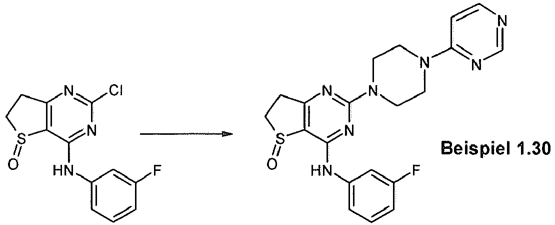

Ausgehend von **(IV-1)** (siehe 1.2) und 4-Piperazin-1-yl-pyrimidin (J. Org. Chem. 1953, 1484) wird **Beispiel 1.30** als Trifluoracetat analog zu Beispiel 1.10 (siehe 10.5) hergestellt und aufgereinigt. Analytische HPLC-MS (Methode C): RT = 1,56 min.

### 31. SYNTHESE VON 4-{4-[4-(3-FLUORPHENYLAMINO)-5-OXO-6,7-DIHYDRO-5H-5λ⁴-THIENO[3,2-d]PYRIMIDIN-2-YL]-PIPERAZIN-1-YL}-PYRIDIN-2-OL (BEISPIEL 1.31)

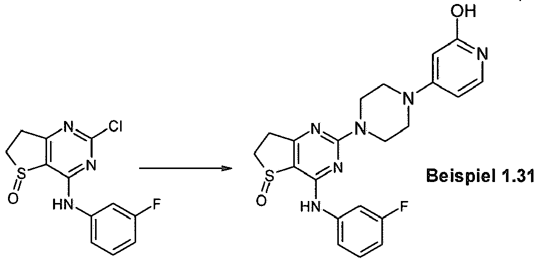

Ausgehend von **(IV-1)** (siehe 1.2) und **(V-7)** (siehe 17.3) wird **Beispiel 1.31** analog zu Beispiel 1.10 (siehe 10.5) hergestellt und aufgereinigt. Analytische HPLC-MS (Methode C): RT = 1,61 min.

### 32. SYNTHESE VON (3-FLUORPHENYL)-[5-OXO-2-(4-PYRIDIN-4-YL-PIPERAZIN-1-YL)-6,7-DIHYDRO-5H-5λ⁴-THIENO[3,2-d]PYRIMIDIN-4-YL]-AMIN (BEISPIEL 1.32)

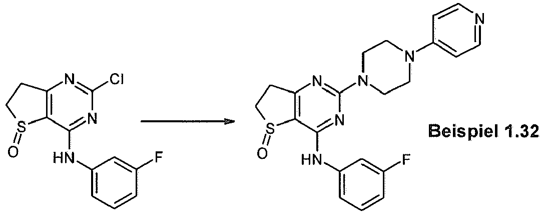

Ausgehend von **(IV-1)** (siehe 1.2) und 1-Pyridin-4-yl-piperazin wird **Beispiel 1.32** analog zu Beispiel 1.10 (siehe 10.5) hergestellt und aufgereinigt. Analytische HPLC-MS (Methode C): RT = 1,56 min.

### 33. SYNTHESE VON (3-FLUORPHENYL)-(2-{4-[4-(4-FLUORPHENYL)-THIAZOL-2-YL]-PIPERAZIN-1-YL)-5-OXO-6,7-DIHYDRO-5H-5λ⁴-THIENO[3,2-d]PYRIMIDIN-4-YL)-AMIN (BEISPIEL 1.33)

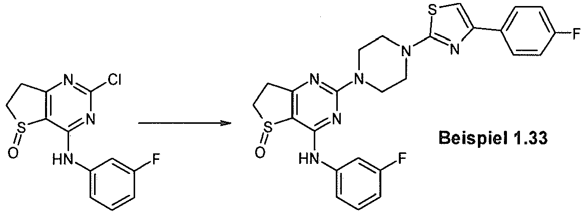

Ausgehend von **(IV-1)** (siehe 1.2) und 1-[4-(4-Fluorphenyl)-thiazol-2-yl]-piperazin wird **Beispiel 1.33** analog zu Beispiel 1.10 (siehe 10.5) hergestellt und aufgereinigt. Analytische HPLC-MS (Methode C): RT = 2,42 min.

### 34. SYNTHESE VON [2-(4-BENZO[d]ISOXAZOL-3-YL-PIPERAZIN-1-YL)-5-OXO-6,7-DIHYDRO-5H-5λ⁴-THIENO[3,2-d]PYRIMIDIN-4-YL]-(3-FLUORPHENYL)-AMIN (BEISPIEL 1.34)

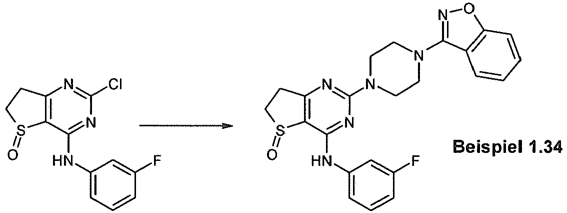

Ausgehend von **(IV-1)** (siehe 1.2) und 3-Piperazin-1-yl-benzo[*d*]isoxazol wird **Beispiel 1.34** analog zu Beispiel 1.10 (siehe 10.5) hergestellt und aufgereinigt. Analytische HPLC-MS (Methode C): RT = 2,19 min.

### 35. SYNTHESE VON (R)-2-(2-{4-[4-(4-FLUORPHENYL)-THIAZOL-2-YL]-PIPERAZIN-1-YL}-5-OXO-6,7-DIHYDRO-5H-5λ⁴-THIENO[3,2-d]PYRIMIDIN-4-YLAMINO)-3-METHYLBUTAN-1-OL (BEISPIEL 1.35)

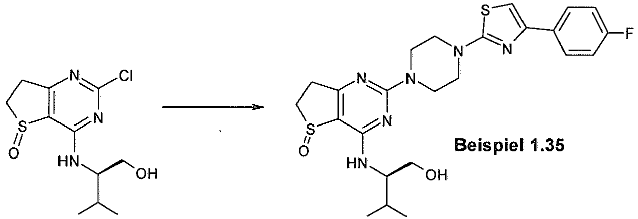

Ausgehend von **(IV-2)** (siehe 2.2) und 1-[4-(4-Fluorphenyl)-thiazol-2-yl]-piperazin wird **Beispiel 1.35** analog zu Beispiel 1.10 (siehe 10.5) hergestellt und aufgereinigt. Analytische HPLC-MS (Methode C): RT = 1,91 min.

### 36. SYNTHESE VON (R)-2-[2-(4-BENZO[d]ISOXAZOL-3-YL-PIPERAZIN-1-YL)-5-OXO-6,7-DIHYDRO-5H-5λ⁴-THIENO[3,2-d]PYRIMIDIN-4-YLAMINO]-3-METHYLBUTAN-1-OL (BEISPIEL 1.36)

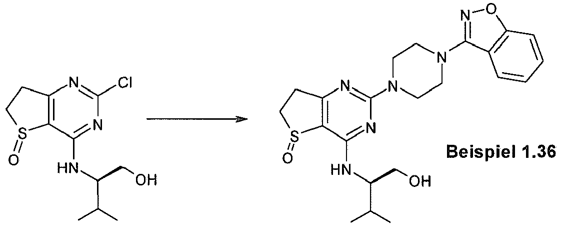

Ausgehend von **(IV-2)** (siehe 2.2) und 3-Piperazin-1-yl-benzo[*d*]isoxazol wird **Beispiel 1.36** analog zu Beispiel 1.10 (siehe 10.5) hergestellt und aufgereinigt. Analytische HPLC-MS (Methode C): RT = 1,76 min.

### CHROMATOGRAPHIE METHODEN

Die nach den obigen Synthese-Schema hergestellten Beispielverbindungen wurden durch folgende chromatographische Methoden, die - sofern sie durchgeführt wurden - im einzelnen in der Tabelle A angegeben sind, charakterisiert.

### Analytische HPLC-MS, Methode A

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 2.50 |
| 0.20 | 95 | 5 | 2.50 |
| 1.50 | 2 | 98 | 2.50 |
| 1.70 | 2 | 98 | 2.50 |
| 1.90 | 95 | 5 | 2.50 |
| 2.20 | 95 | 5 | 2.50 |

Als stationäre Phase dient eine Säule Merck Chromolith™ Flash RP-18e, 4.6 mm x 25 mm (Säulentemperatur: konstant bei 25°C).

### Analytische HPLC-MS, Methode B

Waters ZMD Masse Spektrometer (positive Ionisation (ESI+)), Alliance 2690/2695 HPLC (Diodenarraydetektor, Wellenlängenbereich: 210 to 500 nm), Waters 2700 Autosampler, Waters 996/2996.
A: Wasser mit 0.10% NH₃
B: Acetonitril mit 0.10% NH₃

| Zeit in min | %A | %B | Flussrate in ml/min |
|---|---|---|---|
| 0.00 | 95 | 5 | 3.00 |
| 0.20 | 95 | 5 | 3.00 |
| 1.50 | 2 | 98 | 3.00 |
| 1.90 | 2 | 98 | 3.00 |
| 2.00 | 2 | 98 | 3.00 |

Als stationäre Phase dient Waters, X-Bridge, C18, 3,5 nm, 4,6 X 20 mm. Raumtemperatur

### Analytische HPLC-MS, Methode C

Waters ZQ2000 Massenspektrometer (positive Ionisation (ESI+)), HP1100 HPLC (DAD, Wellenlängenbereich: 210 to 500 nm), and Gilson 215 Autosampler.
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.50 |
| 2.00 | 0 | 100 | 1.50 |
| 2.50 | 0 | 100 | 1.50 |
| 2.60 | 95 | 5 | 1.50 |

Als stationäre Phase dient eine Säule Sunfire C18, 4,6 X 50mm, 3,5 µm, Säulentemperatur 40°C.

### Analytische HPLC, Methode A

Agilent 1100, Diodenarraydetektion erfolgt im Wellenlängenbereich 210-380 nm.
A: Wasser mit 0.10% TFA
B: Acetonitril mit 0.13% TFA

| Zeit in min | %A | %B | Flussrate in ml/min |
|---|---|---|---|
| 0.00 | 95 | 5 | 1.50 |
| 0.60 | 95 | 5 | 1.50 |
| 3.40 | 2 | 98 | 1.50 |
| 3.90 | 2 | 98 | 1.50 |
| 4.20 | 95 | 5 | 1.50 |
| 4.90 | 95 | 5 | 1.50 |

Als stationäre Phase dient eine Säule Varian Microsorb, RP C18, 3 µm, 100 A, Raumtemperatur.

### Präparative HPLC-MS, Methode A

Waters ZQ2000 Masse Spektrometer (positive Ionisation (ESI+)), HP1100 HPLC (DAD, Wellenlängenbereich: 210 - 500 nm), and Gilson 215 Autosampler.
A: Wasser mit 0.10% TFA
B: Acetonitril

| Zeit in min | %A | %B | Flussrate in ml/min |
|---|---|---|---|
| 0.00 | 90 | 10 | 50 |
| 1.50 | 90 | 10 | 50 |
| 8.00 | 40 | 60 | 50 |
| 10.00 | 40 | 60 | 50 |
| 11.00 | 90 | 10 | 50 |

Als stationäre Phase dient eine Säule Sunfire C18, 30 X 100 mm, 5 µm, Raumtemperatur.

### Präparative HPLC, Methode A

Gilson HPLC mit Gilson UV-VIS-155 Detektor, Sampling Injektor 231 XL.

Als Wellenlänge wird das substanzspezifische UV-Maximum angegeben.
A: Wasser mit 0.1% Ammoniak 35%ig
B: Acetonitril

| Zeit in min | %A | %B | Flussrate in ml/min |
|---|---|---|---|
| 0.00 | 95 | 5 | 180 |
| 1.40 | 95 | 5 | 180 |
| 17.00 | 2 | 98 | 180 |
| 18.50 | 2 | 98 | 180 |
| 18.70 | 95 | 5 | 180 |
| 20.-50 | 95 | 5 | 180 |

Als stationäre Phase dient eine Säule Pursuit XRS RP 18, 10 µm, 50 X 150 mm, Raumtemperatur.

### Präparative HPLC, Methode B

Gilson HPLC mit Gilson UV-VIS-155 Detektor, Sampling Injektor 231 XL.

Als Wellenlänge wird das substanzspezifische UV-Maximum angegeben.
A: Wasser mit 0.13% TFA
B: Acetonitril mit 0,1% TFA

| Zeit in min | %A | %B | Flussrate in ml/min |
|---|---|---|---|
| 0.00 | 95 | 5 | 165 |
| 1.30 | 95 | 5 | 165 |
| 8.90 | 2 | 98 | 165 |
| 10.00 | 2 | 98 | 165 |
| 10.50 | 95 | 5 | 165 |
| 11.60 | 95 | 5 | 165 |

Als stationäre Phase dient eine Säule Microsorb RP 18, 8 µm, 50 X 65 mm, Raumtemperatur

### INDIKATIONSGEBIETE

Wie gefunden wurde, zeichnen sich die erfindungsgemäßen Kombinationen enthaltend eine Verbindung der Formel **1** und mindestens ein NSAID durch vielfältige Anwendungsmöglichkeiten auf therapeutischem Gebiet aus. Hervorzuheben sind solche Anwendungsmöglichkeiten, für welche die erfindungsgemäßen Kombinationen aufgrund ihrer pharmazeutischen Wirksamkeit als PDE4-lnhibitor bevorzugt zur Anwendung gelangen können. Beispielhaft genannt seinen Atemwegs- oder gastrointestinalen Erkrankungen oder Beschwerden, entzündliche Erkrankungen der Gelenke, der Haut oder der Augen, Krebserkrankungen, sowie Erkrankungen des periphären oder zentralen Nervensystems.

Hierbei bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einher gehen. Beispielhaft hierfür seinen genannt, akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers' Krankheit, hyperreaktive Atemwege, infektiöse Bronchitis oder Pneumonitis, pediatrisches Asthma, Bronchiectasien, Lungenfibrose, ARDS (akutes Atemnotsyndrom des Erwachsenen), Bronchialödem, Lungenödem, Bronchitis, Pneumonie oder interstitielle Pneumonie ausgelöst durch verschiedene Ursachen wie Aspiration, Inhalation von toxischen Gasen oder Bronchitis, Pneumonie oder interstitielle Pneumonie ausgelöst durch Herzinsuffizienz, Bestrahlung, Chemotherapie zystische Fibrose oder Mukoviszidose, alpha1-Antitrypsin-Mangel.

Ebenfalls bevorzugt genannt sei die Behandlung von entzündlichen Erkrankungen des Gastrointestinaltraktes. Beispielhaft hierfür seinen genannt, akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, zur Behandlung von entzündlichen Erkrankungen der Gelenke wie rheumatoide Arthritis oder entzündliche Erkrankungen der Haut und der Augen.

Ebenfalls bevorzugt genannt sei die Behandlung von Krebserkrankungen. Beispielhaft hierfür seinen genannt alle Formen von akuten und chronischen Leukämien wie, akute lymphatische und akute myeloische Leukämie, chronisch lymphatische und chronisch myeloische Leukämie, sowie Knochentumoren wie das Osteosarkom und sowie alle Arten von Gliomen wie Oligodendrogliom und Glioblastom.

Des Weiteren bevorzugt genannt sei die Vorbeugung und Behandlung von Erkrankungen des periphären oder zentralen Nervensystems. Beispielhaft hierfür seien genannt Depression, bipolare oder manische Depression, akute und chronische Angstzustände, Schizophrenie, Alzheimer'sche Erkrankung, Parkinson'sche Erkrankung, akute und chronische Multiple Sklerose oder akute und chronische Schmerzzustände sowie Verletzungen des Gehirns hervorgerufen durch Schlaganfall, Hypoxie oder Schädel-Hirn-Trauma.

Besonders bevorzugt betrifft die vorliegende Erfindung die Verwendung der erfindungsgemäßen Kombinationen zur Herstellung eines Arzneimittels zur Behandlung entzündlicher oder obstruktiver Erkrankungen der oberen und unteren Atmungsorgane einschließlich der Lunge wie beispielsweise allergische Rhinitis, chronische Rhinitis, Bronchiectasis, zystische Fibrose, idiopathische Lungenfibrose, fibrosierende Alveolitis, COPD, chronische Bronchitis, chronischer Sinusitis, Asthma, Morbus Crohn, Colitis ulcerosa, insbesondere COPD, chronische Bronchitis und Asthma.

Am meisten bevorzugt ist die Verwendung der erfindungsgemäßen Kombinationen zur Behandlung von entzündlichen und obstruktiven Erkrankungen wie COPD, chronische Bronchitis, chronischer Sinusitis, Asthma, Morbus Crohn, Colitis ulcerosa, insbesondere COPD, chronische Bronchitis und Asthma.

Ebenfalls bevorzugt ist die Verwendung der erfindungsgemäßen Kombinationen zur Behandlung von Erkrankungen des periphären oder zentralen Nervensystems wie Depression, bipolare oder manische Depression, akute und chronische Angstzustände, Schizophrenie, Alzheimer'sche Erkrankung, Parkinson'sche Erkrankung, akute und chronische Multiple Sklerose, Amyotrophe Lateralsklerose (ALS) oder akute und chronische Schmerzzustände sowie Verletzungen des Gehirns hervorgerufen durch Schlaganfall, Hypoxie oder Schädel-Hirn-Trauma.

Ein herausragender Aspekt der erfindungsgemäßen Formulierungen enhaltend eine Kombination einer Verbindung der Formel **1** und mindestens ein NSAID ist deren reduziertes Profil an Nebenwirkungen im Vergleich zu Formulierungen, die die gleiche Verbindung der Formel **1** in gleicher Menge in Abwesenheit eines NSAIDs enthalten. Nebenwirkungen, die bei der Verabreichung eines PDE4-Inhibitors häufig auftreten, sind unter anderen vorzugsweise Diarrhoe, Übelkeit und Erbrechen. Im Rattenmodell konnten noch weitere Nebenwirkungen nach PDE4-Inhibitor-Verabreichung beobachtet werden wie beispielsweise Körpergewichtsverlust, Leukozytose und Neutrophilie, aber auch Diarrhoe.

Unter einem reduzierten Profil an Nebenwirkungen wird im Rahmen der Erfindung insbesondere verstanden, eine therapeutisch wirksame Dosis eines PDE4-Inhibitors in einer der erfindungsgemäßen pharmazeutischen Zusammensetzung verabreichen zu können, ohne beim Patienten die oder zumindest eine der häufig bei der Verabreichung von PDE4-Inhibitoren beobachteten Nebenwirkungen in nennenswertem Ausmaß auszulösen. Besonders bevorzugt ist die Verabreichung einer therapeutisch wirksamen Substanzmenge eines PDE4-Inhibitors in der erfindungsgemäßen pharmazeutischen Zusammensetzung in jedem Stadium des Krankheitsverlaufs, ohne die typischen PDE4-Inhibitor-vermittelten Nebenwirkungen der Diarrhoe, des Körpergewichtsverlustes, der Leukozytose oder der Neutrophilie auszulösen. Ein besonderer Gegenstand der vorliegenden Erfindung betrifft die Verabreichung einer therapeutisch wirksamen Substanzmenge der erfindungsgemäßen pharmazeutischen Zusammensetzung in jedem Stadium des Krankheitsverlaufs, ohne die typische PDE4-lnhibitor-vermittelte Nebenwirkung der Diarrhoe in nennenswertem Ausmaß auszulösen.

Im folgenden konnte experimentell am Rattenmodell gezeigt werden, dass die erfindungsgemäßen pharmazeutischen Zusammensetzungen enthaltend einen PDE4-Inhibitor und mindestens ein NSAID viele der Nebenwirkungen, die bei der Verabreichung des entsprechenden PDE4-lnhibitors alleine auftreten, erheblich reduzieren oder sogar völlig vermeiden.

### EXPERIMENTELLE DURCHFÜHRUNG:

### Versuch 1 (Referenz): Diclofenac schützt vor Roflumilast-vermittelten Effekten wie Körpergewichtsverlust, Leukozytose, und Neutrophilie:

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-lnhibitor) um 13 Uhr.
Gruppe 3 ("Roflumilast + Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 10 mg/kg Roflumilast (PDE4-Inhibitor) um 13 Uhr.
Gruppe 4 ("Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 0.5% Natrosol (Placebo) um 13 Uhr.

Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung. Das gleiche traf für eine Ratte aus der Roflumilast-Gruppe zu, die zwischen Versuchstag 4 und 5 verstarb.

In Abbildung 1 A werden die Körpergewichte der Ratten aus den verschiedenen Gruppen als Veränderung in % 1. Applikationszeitpunkt (= Tag 1, 8 Uhr (= Zeitpunkt t₀)) dargestellt. Der Mittelwert ± Standardabweichung der Körpergewichte zum Zeitpunkt t₀ betrug 355 ± 17 g. Am Versuchsende (95 Stunden nach t₀ (= dem Zeitpunkt der ersten Applikation am Tag 1, 8 Uhr)) wurden aus dem Blut von 4 bzw. 5 der Ratten aus den einzelnen Gruppen der Anteil von Weißen Blutzellen (x 1000 Zellen/ µl Blut, Abbildung 1 B, linke Abbildung) und der Anteil der Neutrophilen (in % der Weißen Blutzellen, Abbildung 1 B, rechte Abbildung) bestimmt.

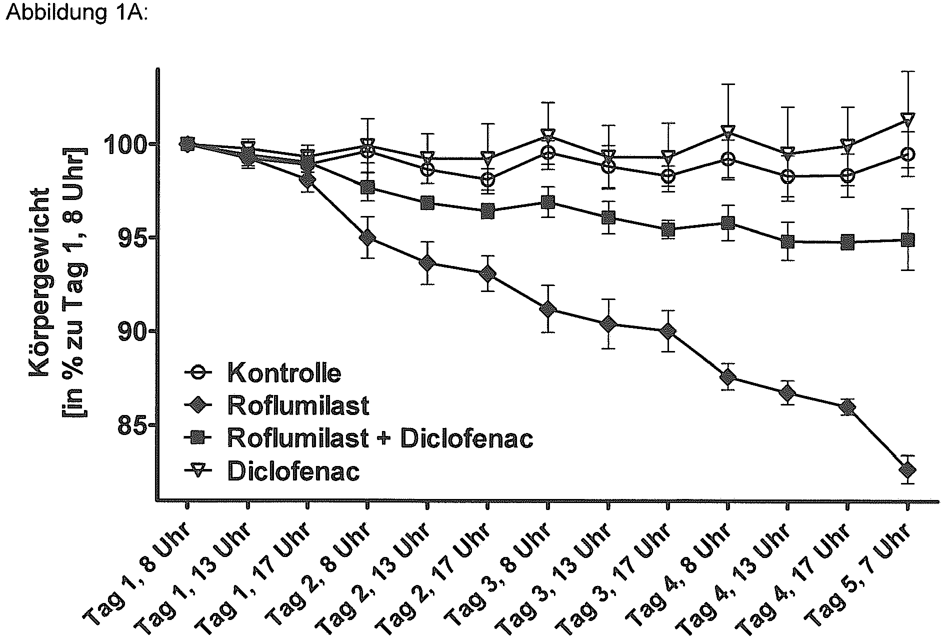

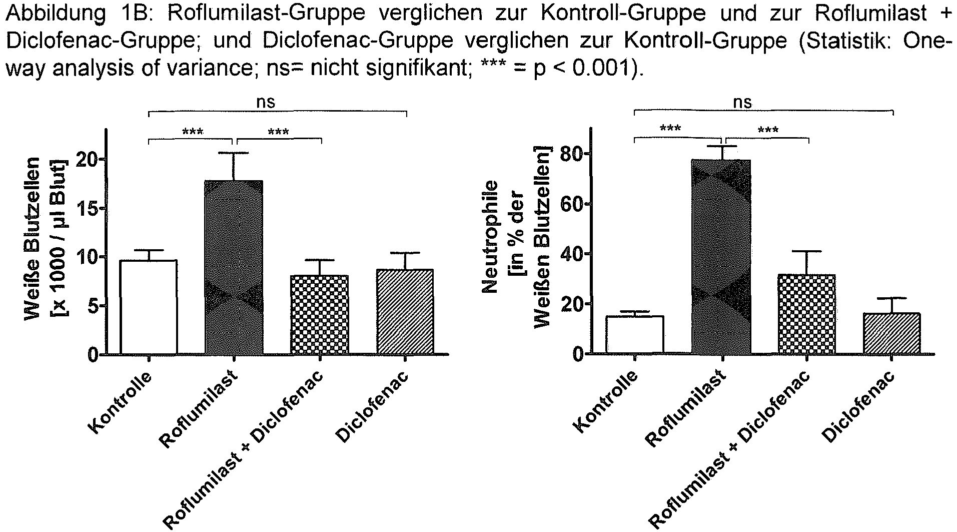

### Versuch 2 (Referenz): Diclofenac schützt for Roflumilast-vermittelten Effekten wie Diarrhoe

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-Inhibitor) um 13 Uhr.
Gruppe 3 ("Roflumilast + Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 10 mg/kg Roflumilast (PDE4-Inhibitor) um 13 Uhr.
Gruppe 4 ("Diclofenac-Gruppe"): Sechs männliche Wistar-Ratten erhielten eine tägliche Applikation von 1 mg/kg Diclofenac (NSAID) zu den Zeitpunkten 8 Uhr und 17 Uhr und von 0.5% Natrosol (Placebo) um 13 Uhr.

Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung. Das gleiche traf für eine Ratte aus der Roflumilast-Gruppe zu, die zwischen Versuchstag 4 und 5 verstarb.

Am Versuchsende (95 Stunden nach t₀ (= dem Zeitpunkt der ersten Applikation am Tag 1, 8 Uhr)) wurden die Ratten aus den einzelnen Gruppen auf das Auftreten multifokaler perivaskulärer mononukleärer Infiltration (=Entzündungsparameter) im Mesenterium und auf das Auftreten der Proliferation von Fibroblasten im Mesenterium hin phänotypisch und histopathologisch untersucht. Weiterhin wurde das Auftreten von Diarrhoe bei den Ratten der verschiedenen Gruppen festgehalten. Die Befunde sind in Tabelle 1 wie folgt zusammengefasst:

**Tabelle 1: Phänotypische und histopathologische Befunde**

| Parameter | Kontrolle (Gruppe 1) | Roflumilast (Gruppe 2) | Roflumilast + Diclofenac (Gruppe 3) | Diclofenac (Gruppe 4) |
|---|---|---|---|---|
| Diarrhoe | 0/6 (= 0 von 6 Tieren) | 5/6 | 0/6 | 0/6 |
| Mesenterium: multifokale perivaskuläre mononukleäre Infiltration (= Entzündungsparameter) | 0/5 | 4/4 | 0/5 | 0/5 |
| Mesenterium: Proliferation von Fibroblasten | 0/5 | 4/4 | 0/5 | 0/5 |

Zusammenfassend lässt sich sagen, dass die in der Roflumilast-Gruppe beobachteteten PDE4-Inhibitor-vermittelten Nebenwirkungen wie Körpergewichtsverlust (Abb. 1A), Leukozytose (Abb. 1B, links), Neutrophilie (Abb. 1B, rechts) und Diarrhoe (einschließlich des Autretens von Entzündungsparametern und der Fibroblasten-Proliferation im Mesenterium) erheblich reduziert oder verhindert werden können (häufig sogar Reduktion auf den Wert der Kontroll-Gruppe), indem man gleichzeitig bzw. nur um einige Stunden versetzt, ein NSAID wie Diclofenac koappliziert (siehe Roflumilast + Diclofenac-Gruppe). Die gemessenen Parameter nach Applikation von Diclofenac alleine zeigten sich sehr ähnlich zu den Kontrollgruppen.

### Versuch 3: Der COX-2 selektive Inhibitor Lumiracoxib, aber nicht der COX-1 selektive Inhibitor SC-560, schützt vor Roflumilast-vermittelten Effekten wie Körpergewichtsverlust, Leukozytose, und Neutrophilie:

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-Inhibitor) um 13 Uhr.
Gruppe 3 ("Roflumilast + SC-560-Gruppe"): Sechs männliche Wistar-Ratten erhielten eine tägliche Applikation von 2 mg/kg SC-560 (NSAID, selektiv für COX-1) zu den Zeitpunkten 8 Uhr und 17 Uhr und von 10 mg/kg Roflumilast (PDE4-Inhibitor) um 13 Uhr.
Gruppe 4 ("Roflumilast + Lumiracoxib-Gruppe"): Sechs männliche Wistar-Ratten erhielten eine tägliche Applikation von 2 mg/kg Lumiracoxib (NSAID, selektiv für COX-2) zu den Zeitpunkten 8 Uhr und 17 Uhr und von 10 mg/kg Roflumilast (PDE4-Inhibitor) um 13 Uhr.
Gruppe 5 ("SC-560-Gruppe"): Sechs männliche Wistar-Ratten erhielten eine tägliche Applikation von 2 mg/kg SC-560 (NSAID, selektiv für Cox-1) zu den Zeitpunkten 8 Uhr und 17 Uhr und von 0.5% Natrosol um 13 Uhr.
Gruppe 6 ("Lumiracoxib-Gruppe"): Sechs männliche Wistar-Ratten erhielten eine tägliche Applikation von 2 mg/kg Lumiracoxib (NSAID, selektiv für Cox-2) zu den Zeitpunkten 8 Uhr und 17 Uhr und von 0.5% Natrosol um 13 Uhr.
Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung.

In Abbildung 3 A werden die Körpergewichte der Ratten aus den verschiedenen Gruppen als Veränderung in % 1. Applikationszeitpunkt (= Tag 1, 8 Uhr (= Zeitpunkt t₀)) dargestellt. Der Mittelwert ± Standardabweichung der Körpergewichte zum Zeitpunkt t₀ betrug 306 ± 11 g. Am Versuchsende (95 Stunden nach t₀ (= dem Zeitpunkt der ersten Applikation am Tag 1, 8 Uhr)) wurden aus dem Blut von 5 der Ratten aus den einzelnen Gruppen der Anteil von Weißen Blutzellen (x 1000 Zellen/ µl Blut, Abbildung 3 B, linke Abbildung) und der Anteil der Neutrophilen (in % der Weißen Blutzellen, Abbildung 3 B, rechte Abbildung) bestimmt

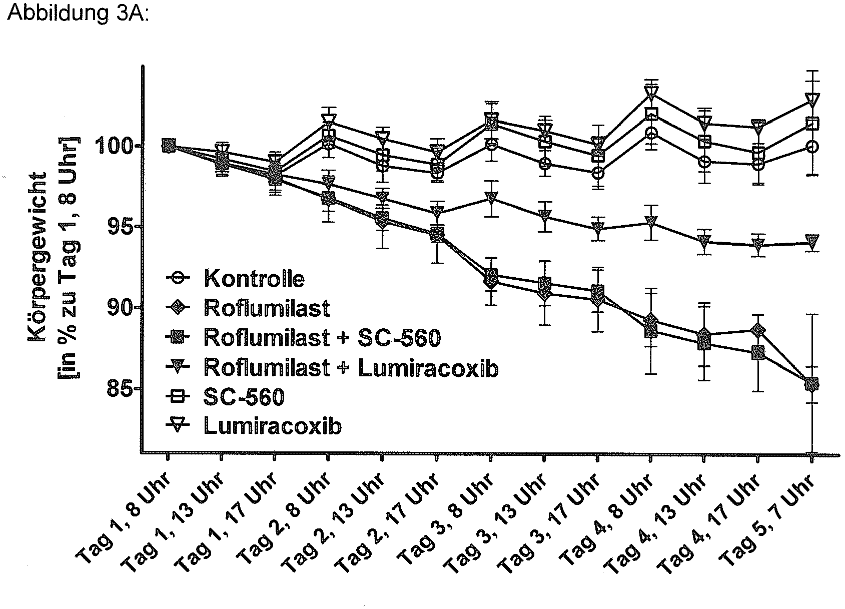

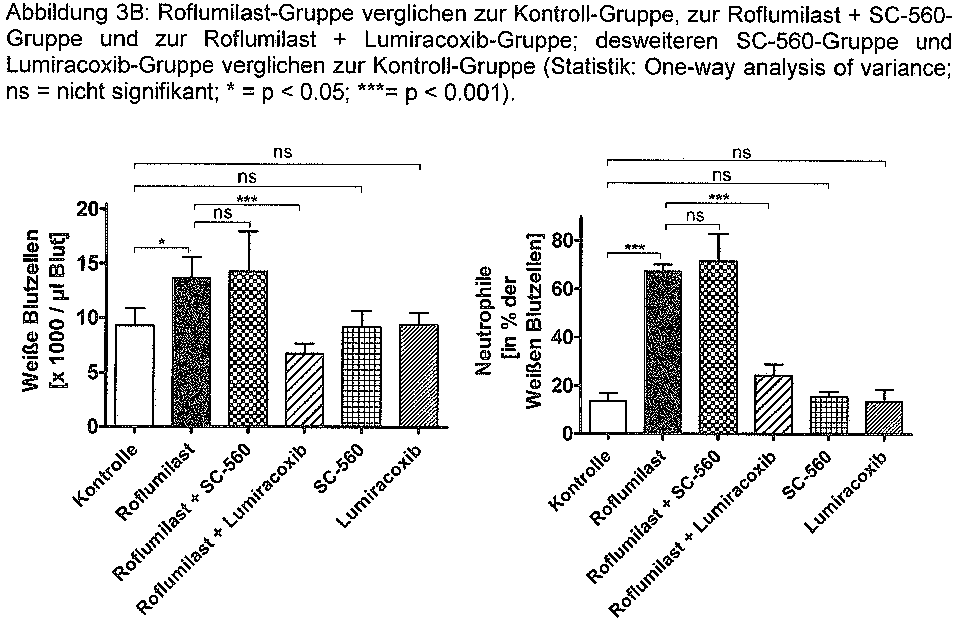

### Versuch 4: Der COX-2 selektive Inhibitor Lumiracoxib, aber nicht der COX-1 selektive Inhibitor SC-560, schützt vor Roflumilast-vermittelten Effekten wie Diarrhoe

Sechs männliche Wistar-Ratten pro Gruppe wurden für vier Tage mit folgenden Substanzen behandelt (alle Applikationen p.o. = peroral):
Gruppe 1 ("Kontroll-Gruppe"): Sechs männliche Wistar-Ratten erhielten eine tägliche Applikation von 0.5% Natrosol (Placebo) zu den Zeitpunkten 8 Uhr, 13 Uhr und 17 Uhr.
Gruppe 2 ("Roflumilast-Gruppe"): Sechs männliche Wistar-Ratten erhielten eine tägliche Applikation von 0.5% Natrosol (Placebo) zu den Zeitpunkten 8 Uhr und 17 Uhr und von 10 mg/kg Roflumilast (PDE4-Inhibitor) um 13 Uhr.
Gruppe 3 ("Roflumilast + SC-560-Gruppe"): Sechs männliche Wistar-Ratten erhielten eine tägliche Applikation von 2 mg/kg SC-560 (NSAID, selektiv für COX-1) zu den Zeitpunkten 8 Uhr und 17 Uhr und von 10 mg/kg Roflumilast (PDE4-Inhibitor) um 13 Uhr.
Gruppe 4 ("Roflumilast + Lumiracoxib-Gruppe"): Sechs männliche Wistar-Ratten erhielten eine tägliche Applikation von 2 mg/kg Lumiracoxib (NSAID, selektiv für COX-2) zu den Zeitpunkten 8 Uhr und 17 Uhr und von 10 mg/kg Roflumilast (PDE4-Inhibitor) um 13 Uhr.
Gruppe 5 ("SC-560-Gruppe"): Sechs männliche Wistar-Ratten erhielten eine tägliche Applikation von 2 mg/kg SC-560 (NSAID, selektiv für Cox-1) zu den Zeitpunkten 8 Uhr und 17 Uhr und von 0.5% Natrosol um 13 Uhr.
Gruppe 6 ("Lumiracoxib-Gruppe"): Sechs männliche Wistar-Ratten erhielten eine tägliche Applikation von 2 mg/kg Lumiracoxib (NSAID, selektiv für Cox-2) zu den Zeitpunkten 8 Uhr und 17 Uhr und von 0.5% Natrosol um 13 Uhr.
Für pharmakokinetische Analysen (Bestimmung von Plasmaspiegeln der Substanzen) wurde am Tag 4 eine Ratte per Gruppe verwendet; diese Ratten standen für weitere zu untersuchende Parameter nicht mehr zur Verfügung.

Am Versuchsende (95 Stunden nach t₀ (= dem Zeitpunkt der ersten Applikation am Tag 1, 8 Uhr)) wurden die Ratten aus den einzelnen Gruppen auf das Auftreten multifokaler perivaskulärer mononukleärer Infiltration (=Entzündungsparameter) im Mesenterium und auf das Auftreten der Proliferation von Fibroblasten im Mesenterium hin phänotypisch und histopathologisch untersucht. Weiterhin wurde das Auftreten von Diarrhoe bei den Ratten der verschiedenen Gruppen festgehalten. Die Befunde sind in Tabelle 2 wie folgt zusammengefasst:

**Tabelle 2: Phänotypische und histopathologische Befunde**

| Parameter | Kontrolle | Roflumilast | Roflumilast + SC-560 | Roflumilast+ Lumiracoxib | SC-560 | Lumiracoxib |
|---|---|---|---|---|---|---|
| | (Gruppe 1) | (Gruppe 2) | (Gruppe 3) | (Gruppe 4) | (Gruppe 5) | (Gruppe 6) |
| Diarrhoe | 0/6 (= 0 von 6 Tieren) | 2/6 | 0/6 | 0/6 | 0/6 | 0/6 |
| Mesenterium: multifokale perivaskuläre mononukleäre Infiltration (= Entzündungsparameter) | 0/5 | 5/5 | 4/5 | 0/5 | 0/5 | 0/5 |
| Mesenterium: Proliferation von Fibroblasten | 0/5 | 5/5 | 4/5 | 0/5 | 0/5 | 0/5 |

Zusammenfassend lässt sich sagen, dass die in der Roflumilast-Gruppe beobachteteten PDE4-lnhibitor-vermittelten Nebenwirkungen wie Körpergewichtsverlust (Abb. 3A), Leukozytose (Abb. 3B, links), Neutrophilie (Abb. 3B, rechts) und Diarrhoe (einschließlich des Auftretens von Entzündungsparametern und der Fibroblasten-Proliferation im Mesenterium) erheblich reduziert oder verhindert werden können (häufig sogar Reduktion auf den Wert der Kontroll-Gruppe), indem man gleichzeitig bzw. nur um einige Stunden versetzt, ein COX-2 selektives NSAID wie Lumiracoxib koappliziert (siehe Roflumilast + Lumiracoxib). Der COX-1 selektive NSAID SC-560 hat keinerlei protektive Wirkung auf Körpergewichtsverlust, Leukozytose, und Neutrophilie und nur eine sehr geringe protektive Wirkung auf die histopathologischen Befunde (multifokale perivaskuläre mononukleäre Infiltration oder Proliferation von Fibroblasten im Mesenterium). Eine Aussage bezüglich der Wirkung von SC-560 auf Diarrhoe wird dadurch erschwert, dass in diesem Versuch in der Roflumilast-Gruppe an für sich nur zwei Tiere Diarrhoe zeigten (in der Regel zeigt nach Roflumilast-Gabe ein noch größerer Prozentsatz der Tiere Diarrhoe). Die gemessenen Parameter nach Applikation von SC-560 oder von Lumiracoxib alleine zeigten sich sehr ähnlich zu den Kontrollgruppen.

Insgesamt lässt sich schlussfolgern, dass die protektive Wirkung eines NSAIDs auf die PDE4-lnhibitor-vermittelten Nebenwirkungen auf der Hemmung von COX-2 beruhen.

### DARREICHUNGSFORMEN

Die Applikation der erfindungsgemäßen Wirkstoffkombinationen aus **1** und **2** erfolgt bevorzugt auf oralem Wege. Hierzu müssen die Bestandteile (**1**) und (**2**) in geeigneten oralen Darreichungsformen bereitgestellt werden.

Geeignete orale Darreichungsformen sind beispielsweise Tabletten, Kapseln, Lösungen, Säfte, Emulsionen. Hierbei soll der Anteil der pharmazeutisch wirksamen Verbindung(en) jeweils im Bereich von 0, 1 bis 90 Gew.-%, bevorzugt 0, 5 bis 50 Gew.-% der Gesamtzusammensetzung liegen, d.h. in Mengen die ausreichend sind, um den unten angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen.

Besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat, mikrokristalliner Cellulose, Sorbitol, Mannitol, Isomaltose oder Milchzucker, Sprengmitteln, wie Maisstärke, quervernetztes Polyvinylpyrrolidon, quervernetzte Natrium Carboxymethylcellulose, Natriumstärkeglykolat oder Alginsäure, Bindemitteln, wie Stärke, Hydroxypropylmethylcellulose, Polyvinylpyrrolidon oder Gelatine, Schmiermitteln, wie Magnesiumstearat oder Talk, und/oder Mitteln zur Erzielung des Depoteffektes, wie Hydroxypropylcellulose, Hydroxypropylmethylcellulose, Ethylcellulose, Amminomethacrylat, Polyvinylpyrrolidon-Polyvinylacetat Copolymer, Carboxymethylcellulose oder Polyvinylacetat erhalten werden. Die Tabletten können auch aus mehreren Schichten bestehen.

Entsprechend können Dragees bzw. Filmtabletten durch Überziehen von analog den Tabletten hergestellten Kernen mit üblicherweise in Dragee- bzw. Filmüberzügen verwendeten Mitteln, beispielsweise Kollidon oder Schellack, Gummi arabicum, Talk, Titandioxid, Zucker, Hydroxypropylmethylcellulose, Ehthycellulose, Celluloseacetatphthalat, Polymethacrylate, Polyethylenglycol, Polyvinylalcohol, Polyvinylalkohol-Polyethylenglycol Copolymere oder Polyvinylacetat hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss-oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker), Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

### Formulierungsbeispiele:

Die folgenden Formulierungsbeispiele für Kombinationsformulierungen sollen der besseren Illustration der Erfindung dienen. Insbesondere können die Wirkstoffe **1** und **2** auch in voneinander getrennten Formulierungen vorliegen und innerhalb eines Zeitfensters von maximal 6 Stunden versetzt voneinander getrennt verabreicht werden. Die Beispiele 6 bis 10 sind Referenzbeispiele.

| | | |
|---|---|---|
| 1) | 0,05 mg | Wirkstoff **1** |
| | 500 mg | Acetylsalicylsäure (Wirkstoff **2**) |
| | 100 mg | Milchzucker |
| | 329,95 mg | Microkristalline Cellulose |
| | 30 mg | Polyvinylpyrrolidon |
| | 30 mg | quervernetztes Polyvinylpyrrolidon |
| | 10 mg | Magnesiumstearat |
| | 1000 mg | |
| | | |
| 2) | 0,1 mg | Wirkstoff **1** |
| | 500 mg | Acetylsalicylsäure (Wirkstoff **2**) |
| | 100 mg | Milchzucker |
| | 329,9 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 3) | 0,5 mg | Wirkstoff **1** |
| | 500 mg | Acetylsalicylsäure (Wirkstoff 2) |
| | 100 mg | Milchzucker |
| | 329,5 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 4) | 5 mg | Wirkstoff **1** |
| | 500 mg | Acetylsalicylsäure (Wirkstoff 2) |
| | 100 mg | Milchzucker |
| | 325 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 5) | 20 mg | Wirkstoff **1** |
| | 500 mg | Acetylsalicylsäure (Wirkstoff **2**) |
| | 100 mg | Milchzucker |
| | 310 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 6) | 0,05 mg | Wirkstoff **1** |
| | 25 mg | Diclofenac (Wirkstoff **2**) |
| | 170 mg | Milchzucker |
| | 269,95 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 7) | 0,1 mg | Wirkstoff **1** |
| | 25 mg | Diclofenac (Wirkstoff **2**) |
| | 170 mg | Milchzucker |
| | 269,9 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 8) | 0,5 mg | Wirkstoff **1** |
| | 25 mg | Diclofenac (Wirkstoff **2**) |
| | 170 mg | Milchzucker |
| | 269,5 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 9) | 5 mg | Wirkstoff **1** |
| | 25 mg | Diclofenac (Wirkstoff **2**) |
| | 170 mg | Milchzucker |
| | 265 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 10) | 20 mg | Wirkstoff **1** |
| | 25 mg | Diclofenac (Wirkstoff **2**) |
| | 170 mg | Milchzucker |
| | 240 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| 11) | 0,05 mg | Wirkstoff **1** |
| | 15 mg | Meloxicam (Wirkstoff **2**) |
| | 170 mg | Milchzucker |
| | 279,95 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 12) | 0,1 mg | Wirkstoff **1** |
| | 15 mg | Meloxicam (Wirkstoff **2**) |
| | 170 mg | Milchzucker |
| | 279,9 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 13) | 0,5 mg | Wirkstoff **1** |
| | 15 mg | Meloxicam (Wirkstoff **2**) |
| | 170 mg | Milchzucker |
| | 279,5 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 14) | 5 mg | Wirkstoff **1** |
| | 15 mg | Meloxicam (Wirkstoff **2**) |
| | 170 mg | Milchzucker |
| | 275 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| | | |
| 15) | 20 mg | Wirkstoff **1** |
| | 15 mg | Meloxicam(Wirkstoff **2**) |
| | 170 mg | Milchzucker |
| | 260 mg | Microkristalline Cellulose |
| | 15 mg | quervernetztes Polyvinylpyrrolidon |
| | 15 mg | Polyvinylpyrrolidon |
| | 5 mg | Magnesiumstearat |
| | 500 mg | |
| 16) | 0,05 mg | Wirkstoff **1** |
| | 500 mg | Naproxen (Wirkstoff **2**) |
| | 100 mg | Milchzucker |
| | 329,95 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 17) | 0,1 mg | Wirkstoff **1** |
| | 500 mg | Naproxen (Wirkstoff **2**) |
| | 100 mg | Milchzucker |
| | 329,9 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 18) | 0,5 mg | Wirkstoff **1** |
| | 500 mg | Naproxen (Wirkstoff **2**) |
| | 100 mg | Milchzucker |
| | 329,5 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 19) | 5 mg | Wirkstoff **1** |
| | 500 mg | Naproxen (Wirkstoff **2**) |
| | 100 mg | Milchzucker |
| | 325 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| | | |
| 20) | 20 mg | Wirkstoff **1** |
| | 500 mg | Naproxen (Wirkstoff **2**) |
| | 100 mg | Milchzucker |
| | 310 mg | Microkristalline Cellulose |
| | 30 mg | quervernetztes Polyvinylpyrrolidon |
| | 30 mg | Polyvinylpyrrolidon |
| | 10 mg | Magnesiumstearat |
| | 1000 mg | |
| 21) | 0,05 mg | Wirkstoff **1** |
| | 200 mg | Ibuprofen (Wirkstoff **2**) |
| | 100 mg | Milchzucker |
| | 258,95 mg | Microkristalline Cellulose |
| | 18 mg | quervernetztes Polyvinylpyrrolidon |
| | 18 mg | Polyvinylpyrrolidon |
| | 5 mg | Magnesiumstearat |
| | 600 mg | |
| | | |
| 22) | 0,1 mg | Wirkstoff **1** |
| | 200 mg | Ibuprofen (Wirkstoff **2**) |
| | 100 mg | Milchzucker |
| | 258,9 mg | Microkristalline Cellulose |
| | 18 mg | quervernetztes Polyvinylpyrrolidon |
| | 18 mg | Polyvinylpyrrolidon |
| | 5 mg | Magnesiumstearat |
| | 600 mg | |
| | | |
| 23) | 0,5 mg | Wirkstoff **1** |
| | 200 mg | Ibuprofen (Wirkstoff **2**) |
| | 100 mg | Milchzucker |
| | 258,5 mg | Microkristalline Cellulose |
| | 18 mg | quervernetztes Polyvinylpyrrolidon |
| | 18 mg | Polyvinylpyrrolidon |
| | 5 mg | Magnesiumstearat |
| | 600 mg | |
| | | |
| 24) | 5 mg | Wirkstoff **1** |
| | 200 mg | Ibuprofen (Wirkstoff **2**) |
| | 100 mg | Milchzucker |
| | 254 mg | Microkristalline Cellulose |
| | 18 mg | quervernetztes Polyvinylpyrrolidon |
| | 18 mg | Polyvinylpyrrolidon |
| | 5 mg | Magnesiumstearat |
| | 600 mg | |
| | | |
| 25) | 20 mg | Wirkstoff **1** |
| | 200 mg | Ibuprofen (Wirkstoff **2**) |
| | 100 mg | Milchzucker |
| | 239 mg | Microkristalline Cellulose |
| | 18 mg | quervernetztes Polyvinylpyrrolidon |
| | 18 mg | Polyvinylpyrrolidon |
| | 5 mg | Magnesiumstearat |
| | 600 mg | |

Der feingemahlene Wirkstoff, Milchzucker und ein Teil der Microkristallinen Cellulose werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Microkristallinen Cellulose und das quervernetzte Polyvinylpyrrolidon werden gesiebt und miteinander vermischt. Anschließend wird das Magnesiumstearat aufgesiebt und kurz untergemischt. Das Gemisch wird zu Tabletten geeigneter Form und Größe verpreßt.

## Patentansprüche

1. Arzneimittelkombination, **dadurch gekennzeichnet, dass** sie neben einem PDE4-Inhibitor der allgemeinen Formel **1**, 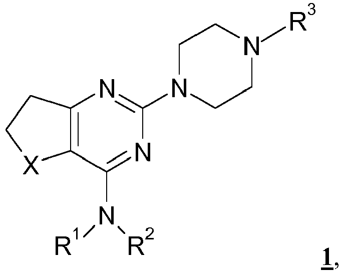 worin
**X** SO,
**R**¹ H
**R²** C₁₋₆-Alkyl ist, das gegebenenfalls durch einen oder mehrere Reste ausgewählt aus F, CF₃, CHF₂ oder CH₂F substituiert sein kann oder das gegebenenfalls durch einen oder mehrere Reste ausgewählt aus der Gruppe bestehend aus OR^{2.1}, COOR^{2.1},CONR^{2.2}R^{2.3}, SR^{2.1},SO-R^{2.1}, SO₂-R^{2.1}, Phenyl, einem Het, einem Hetaryl, einem monocyclischem C₃₋₇-Cycloalkyl, CH₂-NR^{2.2}R^{2.3} und NR^{2.2}R^{2.3} substituiert sein kann,
welcher wiederum gegebenenfalls durch einen oder mehrere Reste ausgewählt aus der Gruppe bestehend aus OH, F, Cl, Br, CF₃, CHF₂, CH₂F, OR^{2.1}, Oxo, Methyl, Ethyl, Propyl, Isopropyl, C₁₋₂-Alkanol, Phenyl, COOR^{2.1}, CH₂-NR^{2.2}R^{2.3} und NR^{2.2}R^{2.3} substituiert sein kann,
wobei **R^{2.1}** H ist oder ein Rest ausgewählt ist aus der Gruppe bestehend aus Methyl, Ethyl, Propyl, Isopropyl, monocyclisches C₃₋₇ Cycloalkyl, Phenyl-C₁₋₂-alkylen, Hetaryl-C₁₋₂-alkylen,
Het-C₁₋₂-alkylen, C₃₋₇-Cycloalkyl-C₁₋₂-alkylen, Phenyl, ein Hetaryl und ein Het, der gegebenenfalls durch einen oder mehrere Reste ausgewählt aus der Gruppe bestehend aus OH, Halogen, Methyl, Ethyl, Propyl, Isopropyl, O-Methyl, O-Ethyl, O-Propyl, O-Isopropyl und Phenyl substituiert sein kann,
wobei **R^{2.2}** und **R**^{**2**.**3**} unabhängig voneinander H oder ein Rest ausgewählt sind aus der Gruppe bestehend aus Methyl, Ethyl, Propyl, Isopropyl, monocyclisches C₃₋₇ Cycloalkyl, Phenyl-C₁₋₃-alkylen, Hetaryl-C₁₋₃-alkylen, Phenyl, Het, Hetaryl, CO-NH₂, CO-NHCH₃, CON(CH₃)₂, SO₂-(C₁-C₂-Alkyl), CO-R^{2.1} und COOR^{2.1},
der gegebenenfalls durch einen oder mehrere Reste ausgewählt aus der Gruppe bestehend aus OH, F, Cl, Br, Methyl, Ethyl, Propyl, Isopropyl, Phenyl und COOR^{2.1} substituiert sein kann,
wobei
**Het** ein drei- bis siebengliedriger, monocyclischer, gesättigter oder teilweise gesättigter Heterocyclus ist, der 1, 2 oder 3 Heteroatome unabhängig voneinander ausgewählt aus der Gruppe bestehend aus N, S oder O enthält,
und wobei
**Hetaryl** ein fünf- bis sechsgliedriger, monocyclisches, aromatisches Heteroaryl ist, das 1, 2 oder 3 Heteroatome unabhängig voneinander ausgewählt aus der Gruppe bestehend aus N, S oder O enthält,
und wobei
**Cycloalkyl** gesättigt oder teilweise gesättigt sein kann,
oder
**R²** ein monocyclisches C₃₋₇ Cycloalkyl, das gegebenenfalls mit einem Rest ausgewählt aus der Gruppe bestehend aus verzweigtes oder unverzweigtes C₁₋₂-Alkanol, C₁₋₃-Fluoroalkyl, C₁₋₃-alkylen-OR^{2.1}, OR^{2.1}, COOR^{2.1}, SO₂-NR^{2.2}R^{2.3}, Het, Methyl, Ethyl, Propyl, Isopropyl, Phenyl , Phenyl-C₁₋₂-alkylen, Hetaryl-C₁₋₂-alkylen, monocyclisches C₃₋₇ Cycloalkyl und NR^{2.2}R^{2.3} substituiert sein kann,
der gegebenenfalls durch einen oder mehrere Reste ausgewählt aus der Gruppe bestehend aus OH, OR^{2.1}, Oxo, Halogen, CF₃, CHF₂, CH₂F, Methyl, Ethyl, Propyl, Isopropyl, Phenyl und NR^{2.2}R^{2.3} substituiert sein kann,
oder
**R²** ein Phenyl, das gegebenenfalls durch OH, SH, F, Cl oder Br oder durch einen oder mehrere Reste ausgewählt aus der Gruppe bestehend aus OR^{2.1}, COOR^{2.1}, NR^{2.2}R^{2.3}, CH₂-NR^{2.2}R^{2.3},C₃₋₇-Cycloalkyl, Het, Methyl, Ethyl, Propyl, Isopropyl, CF₃, CHF₂, CH₂F, Phenyl-C₁₋₂-alkylen, Het-C₁₋₂-alkylen, Hetaryl-C₁₋₂-alkylen, Phenyl, SO₂-CH₃, SO₂-CH₂CH₃ und SO₂-NR^{2.2}R^{2.3} substituiert sein kann,
der wiederum gegebenenfalls durch einen oder mehrere oder mehrere Reste ausgewählt aus der Gruppe bestehend aus OH, OR^{2.1}, Oxo, F, Cl, Br, CF₃, CHF₂, CH₂F, Methyl, Ethyl, Propyl, Isopropyl, Phenyl und NR^{2.2}R^{2.3} substituiert sein kann,
oder
**R²** ein Rest ausgewählt aus einer Gruppe bestehend aus einem Het und einem Hetaryl, welcher gegebenenfalls durch einen oder mehrere Reste ausgewählt aus der Gruppe F, Cl, Br, OH, Oxo, CF₃, CHF₂, CH₂F und SH oder durch einen oder mehrere Reste ausgewählt aus der Gruppe OR^{2.1}, C₁₋₃-alkylen-OR^{2.1}, SR^{2.1},SO-R^{2.1}, SO₂-R^{2.1}, COOR^{2.1}, COR^{2.1},C₁₋₂-Alkanol, C₃₋₁₀-Cycloalkyl, Phenyl, Methyl, Ethyl, Propyl, Isopropyl, Phenyl-C₁₋₂-alkylen, Hetaryl-C₁₋₂-alkylen, Het, Hetaryl, C₁₋₂-Alkanol und NR^{2.2}R^{2.3} substituiert sein kann,
der gegebenenfalls wiederum durch einen oder mehrere Reste ausgewählt aus der Gruppe bestehend aus OH, OR^{2.1}, Oxo, F, Cl, Br, CF₃, CHF₂, CH₂F, C₁₋₆-Alkyl, Phenyl und NR^{2.2}R^{2.3} substituiert sein kann,
und worin
**R³** ein Rest ausgewählt aus der Gruppe bestehend aus einem gesättigten oder teilweise gesättigten, monocyclischem drei- bis siebengliedrigen Heterocyclus, einem gesättigten oder teilweise gesättigten, bicyclischem fünf- bis elfgliedrigem Heterocyclus, einem monocyclischen, fünf- bis sechsgliedrigem Heteroaryl und einem bicyclischem, sieben- bis elfgliedrigem Heteroaryl ist,
der jeweils 1, 2, 3 oder 4 Heteroatome unabhängig voneinander ausgewählt aus der Gruppe bestehend aus N, O und S enthält
und der jeweils gegebenenfalls durch einen oder mehrere Reste ausgewählt aus der Gruppe bestehend aus
Halogen, C₁₋₃-Fluoroalkyl, CN, OH, Oxo, -C₁₋₆-Alkyl, -O-R^{2.1}, -COOR^{2.1}, SO-R^{2.1}, SO₂-R^{2.1}, C₆₋₁₀-Aryl, C₁₋₃-alkylen-C₆₋₁₀-Aryl, -C₁₋₃-alkylen-NR^{2.2}R^{2.3}, -NR^{2.2}R^{2.3}, einem C₃₋₁₀-Cycloalkyl, einem C₁₋₃-alkylen-C₃₋₁₀-Cycloalkyl, Het, einem Hetaryl, C₁₋₃-alkylen-Hetaryl, und C₁₋₃-alkylen-Het substituiert sein kann,
die wiederum gegebenenfalls mit einem oder mehreren Resten ausgewählt aus der Gruppe OH, Halogen, -C₁₋₃-Fluoroalkyl, C₁₋₆-Alkyl, C₆₋₁₀-Aryl, -COO(C₁₋₃-Alkyl) und O-(C₁₋₃-Alkyl) substituiert sein kann,
bedeuten,
wenigstens ein NSAID (= non-steroidal anti-inflammatory drug) (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

2. Arzneimittelkombinationen nach Anspruch 1, **dadurch gekennzeichnet, dass** sie neben dem PDE4-Inhibitor der allgemeinen Formel **1**, worin
**R**² ein Rest nach Formel **3** 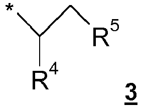
ist,
worin **R**⁵ OH oder NH₂ ist und
worin **R**⁴ ein Rest ausgewählt aus der Gruppe bestehend aus C₁₋₄-Alkyl, Hetaryl und Phenyl,
der gegebenenfalls mit einem oder mehreren Resten ausgewählt aus der Gruppe bestehend aus OH, F, Br, OR^{2.1}, Oxo, Methyl, Ethyl, C₁₋₂-Alkanol, Phenyl, COOR^{2.1}, CH₂-NR^{2.2}R^{2.3} und NR^{2.2}R^{2.3} substituiert sein kann,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

3. Arzneimittelkombination nach Anspruch 2, **dadurch gekennzeichnet, dass** sie neben dem PDE4-lnhibitor der allgemeinen Formel **1**, worin
**R**² ein Rest ist nach Formel **3** 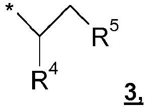
worin **R**⁵ OH oder NH₂ ist und
worin **R**⁴ Methyl, Ethyl, Propyl, Isopropyl
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

4. Arzneimittelkombination nach Anspruch 1, **dadurch gekennzeichnet, dass** sie neben dem PDE4-lnhibitor der allgemeinen Formel **1**, worin
**R**² ein monocyclischer drei-, vier-, fünf-, sechs oder siebengliedriger Cycloalkyl-Ring ist, der gegebenenfalls in spiro-Stellung mit einem Rest ausgewählt aus der Gruppe bestehend aus -CH₂-OR^{2.1}, verzweigtes oder unverzweigtes C₂₋₆-Alkylen-OR^{2.1}, Methyl, Ethyl, Propyl, Isopropyl, Butyl, Isobutyl, Cyclopropyl, -CF₃, CHF₂, CH₂F und C₂₋₄-Fluoroalkyl substituiert sein kann, wobei
R^{2.1} ausgewählt ist aus der Gruppe bestehend aus Methyl, Ethyl, Propyl, Isopropyl, Butyl, Isobutyl,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

5. Arzneimittelkombination nach Anspruch 1, **dadurch gekennzeichnet, dass** sie neben dem PDE4-Inhibitor der allgemeinen Formel **1**, worin
**R**² ein Phenyl ist, das gegebenenfalls in einer oder in beiden meta-Stellungen durch einen oder mehrere Reste ausgewählt aus der Gruppe bestehend aus Methyl, Ethyl, Propyl, Isopropyl, Cyclopropyl, F, Cl, Br, OH, OR^{2.1}, COOR^{2.1}, CF₃, CHF₂, CH₂F, NH₂ und N(CH₃)₂ substituiert ist, wobei R^{2.1} H, Methyl oder Ethyl sein kann,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

6. Arzneimittelkombination nach Anspruch 1, **dadurch gekennzeichnet, dass** sie neben dem PDE4-Inhibitor der allgemeinen Formel **1**, worin
**R**² ein monocyclischer, gesättigter drei-, vier-, fünf-, sechs- oder siebengliedriger Heterocyclus mit 1, 2 oder 3 Heteroatomen jeweils ausgewählt aus der Gruppe bestehend aus N, O und S ist, der gegebenenfalls durch einen oder mehrere Reste ausgewählt aus der Gruppe bestehend aus Fluor, Chlor, Brom, CF₃, CHF₂, CH₂F, OH, Oxo und SH oder durch einen oder mehrere Reste ausgewählt aus der Gruppe bestehend aus OR^{2.1}, C₁₋₃-alkylen-OR^{2.1}, SR^{2.1},SO-R^{2.1}, SO₂-R^{2.1}, COOR^{2.1}, COR^{2.1}, C₁₋₆-Alkanol, C₃₋₁₀-Cycloalkyl, Phenyl, C₁₋₆-Alkyl, Phenyl-C₁₋₆-alkylen, Hetaryl-C₁₋₆-alkylen, Het, Hetaryl und NR^{2.2}R^{2.3} substituiert sein kann, der gegebenenfalls wiederum durch einen oder mehrere Reste ausgewählt aus der Gruppe bestehend aus OH, OR^{2.1}, Oxo, F, Cl, CF₃, CHF₂, CH₂F, C₁₋₆-Alkyl, Phenyl und NR^{2.2}R^{2.3} substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

7. Arzneimittelkombination nach Anspruch 6, **dadurch gekennzeichnet, dass** sie neben dem PDE4-Inhibitor der allgemeinen Formel **1**, worin
**R**² ein monocyclischer, gesättigter, sechsgliedriger Heterocyclus mit einem Heteroatom ausgewählt aus der Gruppe bestehend aus N, O und S ist, der gegebenenfalls durch einen oder mehrere Reste ausgewählt aus der Gruppe F, Cl, Br, CF₃, CHF₂, CH₂F, OH, Oxo, NH₂, NHCH₃, N(CH₃)₂, Methyl, Ethyl, Propyl, Isopropyl, Cyclopropyl, Methoxy und Ethoxy substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

8. Arzneimittelkombination nach einem der Ansprüche 6 oder 7, **dadurch gekennzeichnet, dass** sie neben dem PDE4-Inhibitor der allgemeinen Formel **1**, worin
**R**² ein Rest ausgewählt aus einer Gruppe bestehend aus Piperidin oder Tetrahydropyran, der gegebenenfalls durch einen oder mehrere Reste ausgewählt aus der Gruppe F, Cl, Br, OH, CF₃, CHF₂, CH₂F, NH₂, NHCH₃, N(CH₃)₂, Oxo, Methyl und Methoxy substituiert sein kann,
bedeutet,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

9. Arzneimittelkombination nach einem der Ansprüche 1 bis 8, **dadurch gekennzeichnet, dass** sie neben dem PDE4-Inhibitor der allgemeinen Formel **1**, worin
**R**³ monocyclischer fünf- oder sechsgliedriger Heteroarylring ist, der gegebenenfalls durch einen oder mehrere Reste ausgewählt aus der Gruppe bestehend aus F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO-(CH₃), SO₂-(CH₂CH₃), SO-(CH₂CH₃), Phenyl, -methylen-Phenyl, -ethylen-Phenyl, -NH₂, -NH(CH₃), N(CH₃)₂, -methylen-NH₂, -methylen-NH(CH₃), -methylen-N(CH₃)₂, einem C₃₋₆-Cycloalkyl, einem methylen-C₃₋₆-Cycloalkyl, einem gesättigtem oder teilweise gesättigtem, fünf- bis sechsgliedrigem Heterocyclus, einem fünf- oder sechsgliedrigem Heteroaryl, -methylen-Hetaryl, und -methylen-Het substituiert sein kann, die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl, -COO(CH₃), -O-Methyl und -O-Ethyl substituiert sein können,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

10. Arzneimittelkombination nach einem der Ansprüche 1 bis 8, **dadurch gekennzeichnet, dass** sie neben dem-Inhibitor der allgemeinen Formel **1**, worin
**R³** ein bicyclischer 9- bis 11-gliedriger gesättigter, ungesättigter oder teilweise gesättigter Heterocyclus ist, der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO-(CH₃), SO₂-(CH₂CH₃), SO-(CH₂CH₃), Phenyl, -methylen-Phenyl, -ethylen-Phenyl, -NH2, -NH(CH₃), N(CH₃)₂, -methylen-NH₂, -methylen-NH(CH₃), -methylen-N(CH₃)₂, einem -C₃₋₆-Cycloalkyl, einem -methylen-C₃₋₆-Cycloalkyl, einem gesättigtem, teilweise ungesättigtem oder ungesättigtem 5-bis 6-gliedriger Heterocyclus, einem 5-bis 6-gliedrigem Heteroaryl, ,-methylen-Hetaryl, und -methylen-Het substituiert sein kann,
die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl,-COO(CH₃), -O-Methyl und -O-Ethyl substituiert sein können,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

11. Arzneimittelkombination nach Anspruch 9, **dadurch gekennzeichnet, dass** sie neben dem PDE4-Inhibitor der allgemeinen Formel **1**, worin
**R**³ ein monocyclischer fünf- oder sechsgliedriger Heteroarylring ausgewählt aus der Gruppe bestehend aus Pyrrol, Pyrazol, Furan, Thiophen, Thiazol, Imidazol, Oxazol, Pyridazin, Pyrimidin, Pyrazin, Thiadiazol, Oxadiazol, Triazin, Isooxazol, Isothiazol und Pyridin ist,
der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO₂-(CH₂CH₃), Phenyl, -methylen-Phenyl, -ethylen-Phenyl, -NH₂, -NH(CH₃), N(CH₃)₂, -methylen-NH₂, -methylen-NH(CH₃), -methylen-N(CH₃)₂, einem C₃₋₆-Cycloalkyl, einem methylen-C₃₋₆-Cycloalkyl, einem Het, einem Hetaryl,-methylen-Hetaryl, und -methylen-Het substituiert sein kann,
die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl,-COO(CH₃), -O-Methyl und -O-Ethyl substituiert sein können,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

12. Arzneimittelkombination nach Anspruch 10, **dadurch gekennzeichnet, dass** sie neben dem PDE4-Inhibitor der allgemeinen Formel 1, worin
**R**³ bicyclischer 9- bis 11-gliedriger Heterocyclus ausgewählt aus der Gruppe bestehend aus Benzoxazol, Benzodioxol, Dihydrobenzodioxin, Benzodioxin, Benzisoxazol, Benzothiazol, Benzisothiazol, Thienopyrimidin, Furopyrimidin, Thienopyridin, Furopyridin, Indol, Isoindol, Chinoxalin, Naphthyridin, Pyridopyrazin, Pyridopyrimidin, Chinolin, Isochinolin, Benzoimidazol, 6,7,8,9-Tetrahydro-5H-pyrazino[2,3-d]azepin, Benzothiophen, Benzofuran, Chinazolin, Indazol, Isobenzofuran und Pteridin ist,
der gegebenenfalls durch einen oder mehrere Reste ausgewählt aus der Gruppe bestehend aus
F, Cl, Br, CF₃, CHF₂, CH₂F, CN, OH, -Methyl, Ethyl, Propyl, Isopropyl, -O-Methyl, O-Ethyl, -COOMethyl, -COOEthyl, SO₂-(CH₃), SO₂-(CH₂CH₃), Phenyl, -methylen-Phenyl, -ethylen-Phenyl, -NH₂, -NH(CH₃), N(CH₃)₂, -methylen-NH₂, -methylen-NH(CH₃), -methylen-N(CH₃)₂, einem C₃₋₆-Cycloalkyl, einem methylen-C₃₋₆-Cycloalkyl, einem Het, einem Hetaryl, ,-methylen-Hetaryl, und -methylen-Het substituiert sein kann,
die wiederum gegebenenfalls mit einem oder mehreren Resten ausgewählt aus der Gruppe OH, F, Cl, Br, CF₃, CHF₂, CH₂F, Methyl, Ethyl, Propyl, Isopropyl, Phenyl, - COO(CH₃), -O-Methyl und -O-Ethyl substituiert sein können,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

13. Arzneimittelkombination nach Anspruch 1, **dadurch gekennzeichnet, dass** sie neben dem PDE4-Inhibitor der allgemeinen Formel **1,** worin
**R¹** H ist,
**R²** ein Rest ausgewählt ist aus der Gruppe bestehend aus 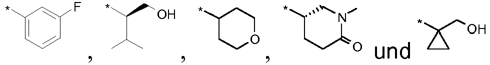 ist,
**R³** ein Rest ausgewählt ist aus der Gruppe bestehend aus 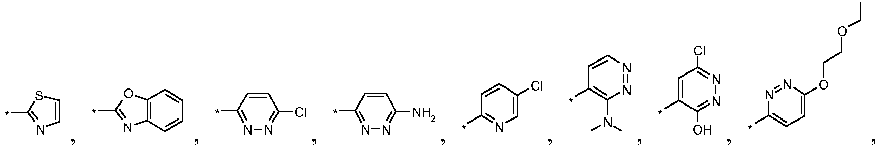 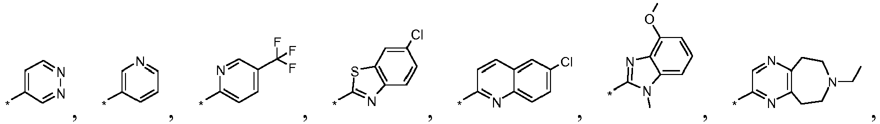 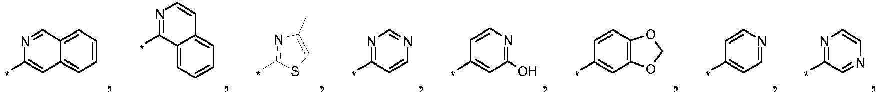 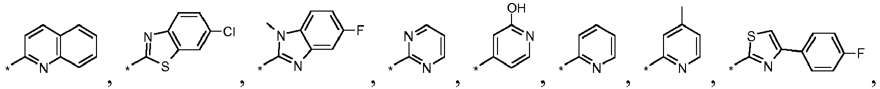 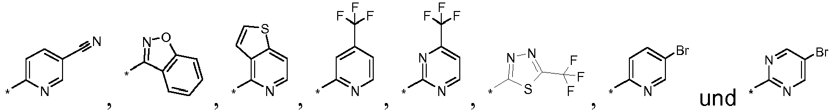 ist,
bedeuten,
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

14. Arzneimittelkombinationen nach Anspruch 1, **dadurch gekennzeichnet, dass** sie neben dem PDE4-Inhibitor der allgemeinen Formel **1** ausgewählt aus der Gruppe bestehend aus:
1.1. (3-Fluorphenyl)-[5-oxo-2-(4-thiazol-2-yl-piperazin-1-yl)-6,7-dihydro-5*H*-5λ⁴-thieno[3,2-*d*]pyrimidin-4-yl]-amin
1.2. (R)-3-Methyl-2-[5-oxo-2-(4-thiazol-2-yl-piperazin-1-yl)-6,7-dihydro-5*H*-5λ⁴-thieno[3,2-*d*]pyrimidin-4-ylamino]-butan-1-ol
1.3. [2-(4-Benzoxazol-2-yl-piperazin-1-yl)-5-oxo-6,7-dihydro-5*H*-5λ□⁴-thieno[3,2-*d*]pyrimidin-4-yl]-(3-fluorphenyl)-amin
1.4. [2-(4-Benzoxazol-2-yl-piperazin-1-yl)-5-oxo-6,7-dihydro-5*H*-5□⁴-thieno[3,2-*d*]pyrimidin-4-yl]-(tetrahydropyran-4-yl)-amin
1.5. (R)-2-{2-[4-(6-Chlorpyridazin-3-yl)-piperazin-1-yl]-5-oxo-6,7-dihydro-5*H*-5λ⁴-thieno[3,2-*d*]pyrimidin-4-ylamino}-3-methylbutan-1-ol
1.6. {2-[4-(6-Chlorpyridazin-3-yl)-piperazin-1-yl]-5-oxo-6,7-dihydro-5*H*-5λ⁴-thieno[3,2-*d*]pyrimidin-4-yl}-(3-fluorphenyl)-amin
1.7. (R)-2-[2-(4-Benzoxazol-2-yl-piperazin-1-yl)-5-oxo-6,7-dihydro-5*H*-5λ⁴-thieno[3,2-*d*]pyrimidin-4-ylamino]-3-methylbutan-1-ol
1.8. (1-{2-[4-(5-Chlorpyridin-2-yl)-piperazin-1-yl]-5-oxo-6,7-dihydro-5*H*-5λ⁴-thieno[3,2-*d*]pyrimidin-4-ylamino}-cyclopropyl)-methanol
1.9. {2-[4-(5-Chlorpyridin-2-yl)-piperazin-1-yl]-5-oxo-6,7-dihydro-5*H*-5λ⁴-thieno[3,2-*d*]pyrimidin-4-yl}-(tetrahydropyran-4-yl)-amin
1.10. {2-[4-(3-Dimethylaminopyridazin-4-yl)-piperazin-1-yl]-5-oxo-6,7-dihydro-5*H*-5λ⁴-thieno[3,2-*d*]pyrimidin-4-yl}-(3-fluorphenyl)-amin
1.11. 6-Chlor-4-{4-[4-(3-fluorphenylamino)-5-oxo-6,7-dihydro-5*H*-5λ⁴-thieno[3,2-*d*]pyrimidin-2-yl]-piperazin-1-yl}-pyridazin-3-ol
1.12. 2-{4-[6-(2-Ethoxyethoxy)-pyridazin-3-yl]-piperazin-1-yl}-5-oxo-6,7-dihydro-5*H*-5λ⁴-thieno[3,2-*d*]pyrimidin-4-yl)-(3-fluorphenyl)-amin
1.13. (3-Fluorphenyl)-[5-oxo-2-(4-pyridazin-4-yl-piperazin-1-yl)-6,7-dihydro-5*H*-5λ⁴-thieno[3,2-*d*]pyrimidin-4-yl]-amin
1.14. (R)-2-{2-[4-(4-Methoxy-1-methyl-1*H*-benzimidazol-2-yl)-piperazin-1-yl]-5-oxo-6,7-dihydro-5*H*-5λ⁴-thieno[3,2-*d*]pyrimidin-4-ylamino}-3-methylbutan-1-ol
1.15. (R)-2-{2-[4-(7-Ethyl-6,7,8,9-tetrahydro-5*H*-pyrazino[2,3-*d*]azepin-2-yl)-piperazin-1-yl]-5-oxo-6,7-dihydro-5*H*-5λ⁴-thieno[3,2-*d*]pyrimidin-4-ylamino}-3-methylbutan-1-ol
1.16. (R)-3-Methyl-2-[5-oxo-2-(4-pyrimidin-4-yl-piperazin-1-yl)-6,7-dihydro-5*H*-5λ⁴-thieno[3,2-*d*]pyrimidin-4-ylamino]-butan-1-ol
1.17. 4-{4-[4-((R)-1-Hydroxymethyl-2-methylpropylamino)-5-oxo-6,7-dihydro-5H-5λ4-thieno[3,2-*d*]pyrimidin-2-yl]-piperazin-1-yl}-pyridin-2-ol
1.18. (R)-3-Methyl-2-[5-oxo-2-(4-pyridin-4-yl-piperazin-1-yl)-6,7-dihydro-5*H*-5λ⁴-thieno[3,2-*d*]pyrimidin-4-ylamino]-butan-1-ol
1.19. (R)-2-{2-[4-(3-Dimethylaminopyridazin-4-yl)-piperazin-1-yl]-5-oxo-6,7-dihydro-5*H*-5λ⁴-thieno[3,2-*d*]pyrimidin-4-ylamino}-3-methylbutan-1-ol
1.20. 6-Chlor-4-{4-[4-((R)-1-hydroxymethyl-2-methylpropylamino)-5-oxo-6,7-dihydro-5*H*-5λ⁴-thieno[3,2-*d*]pyrimidin-2-yl]-piperazin-1-yl}-pyridazin-3-ol
1.21. (R)-2-(2-{4-[6-(2-Ethoxyethoxy)-pyridazin-3-yl]-piperazin-1-yl}-5-oxo-6,7-dihydro-5*H-*5λ⁴-thieno[3,2-*d*]pyrimidin-4-ylamino)-3-methylbutan-1-ol
1.22. (R)-3-Methyl-2-[5-oxo-2-(4-pyridazin-4-yl-piperazin-1-yl)-6,7-dihydro-5H-5λ⁴-thieno[3,2-*d*]pyrimidin-4-ylamino]-butan-1-ol
1.23. {1-[5-Oxo-2-(4-pyrimidin-4-yl-piperazin-1-yl)-6,7-dihydro-5*H*-5λ⁴-thieno[3,2-*d*]pyrimidin-4-ylamino]-cyclopropyl}-methanol
1.24. {1-[5-Oxo-2-(4-pyridin-4-yl-piperazin-1-yl)-6,7-dihydro-5*H*-5λ⁴-thieno[3,2-*d*]pyrimidin-4-ylamino]-cyclopropyl}-methanol
1.25 (S)-1-Methyl-5-[5-oxo-2-(4-pyrimidin-4-yl-piperazin-1-yl)-6,7-dihydro-5H-5λ4-thieno[3,2-d]pyrimidin-4-ylamino]-piperidin-2-on
1.26 {2-[4-(5-Fluor-1-methyl-1H-benzoimidazol-2-yl)-piperazin-1-yl]-5-oxo-6,7-dihydro-5H-5λ4-thieno[3,2-d]pyrimidin-4-yl}-(tetrahydropyran-4-yl)-amin
1.27 [5-Oxo-2-(4-pyridin-4-yl-piperazin-1-yl)-6,7-dihydro-5H-5λ4-thieno[3,2-d]pyrimidin-4-yl]-(tetrahydropyran-4-yl)-amin
1.28 (3-Fluorphenyl)-{2-[4-(4-methoxy-1-methyl-1H-benzimidazol-2-yl)-piperazin-1-yl]-5-oxo-6,7-dihydro-5H-5λ4-thieno[3,2-d]pyrimidin-4-yl}-amin
1.29 {2-[4-(7-Ethyl-6,7,8,9-tetrahydro-5H-pyrazino[2,3-d]azepin-2-yl)-piperazin-1-yl]-5-oxo-6,7-dihydro-5H-5λ4-thieno[3,2-d]pyrimidin-4-yl}-(3-fluorphenyl)-amin
1.30 (3-Fluorphenyl)-[5-oxo-2-(4-pyrimidin-4-yl-piperazin-1-yl)-6,7-dihydro-5H-5λ4-thieno[3,2-d]pyrimidin-4-yl]-amin
1.31 4-{4-[4-(3-Fluorphenylamino)-5-oxo-6,7-dihydro-5*H*-5λ⁴-thieno[3,2-d]pyrimidin-2-yl]-piperazin-1-yl}-pyridin-2-ol
1.32 (3-Fluorphenyl)-[5-oxo-2-(4-pyridin-4-yl-piperazin-1-yl)-6,7-dihydro-5*H*-5λ⁴-thieno[3,2-d]pyrimidin-4-yl]-amin
1.33 (3-Fluorphenyl)-(2-{4-[4-(4-fluorphenyl)-thiazol-2-yl]-piperazin-1-yl}-5-oxo-6,7-dihydro-5*H*-5λ4-thieno[3,2-d]pyrimidin-4-yl)-amin
1.34 [2-(4-Benzo[d]isoxazol-3-yl-piperazin-1-yl)-5-oxo-6,7-dihydro-5H-5λ4-thieno[3,2-d]pyrimidin-4-yl]-(3-fluorphenyl)-amin
1.35 (R)-2-(2-{4-[4-(4-Fluorphenyl)-thiazol-2-yl]-piperazin-1-yl}-5-oxo-6,7-dihydro-5H-5λ4-thieno[3,2-d]pyrimidin-4-ylamino)-3-methylbutan-1-ol
1.36 (R)-2-[2-(4-Benzo[d]isoxazol-3-yl-piperazin-1-yl)-5-oxo-6,7-dihydro-5H-5λ4-thieno[3,2-d]pyrimidin-4-ylamino]-3-methylbutan-1-ol
wenigstens ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125), Acetylsalicylsäure (2.3), Meloxicam (2.77), Naproxen (2.82) und Piroxicam (2.93) umfasst.

15. Arzneimittelkombination nach einem der Ansprüche 1 bis 14, **dadurch gekennzeichnet, dass** sie neben dem PDE4-Inhibitor der allgemeinen Formel **1** ein NSAID (**2**) ausgewählt aus der Gruppe bestehend aus Celecoxib (2.25), Etoricoxib (2.41), Lumiracoxib (2.74), Parecoxib (2.88), Rofecoxib (2.101) und Valdecoxib (2.125) umfasst.

16. Arzneimittelkombination
nach einem der Ansprüche 1 bis 14
zur Behandlung einer Erkrankung ausgewählt aus der Gruppe bestehend aus Atemwegserkrankungen, Lungenerkrankungen, gastrointestinalen Beschwerden, Erkrankungen wie auch entzündliche Erkrankungen der Gelenke, der Haut oder der Augen, Krebserkrankungen und Erkrankungen des periphären oder zentralen Nervensystems.

17. Arzneimittelkombination nach Anspruch 16, zur Behandlung einer Erkrankung ausgewählt aus COPD, chronischer Sinusitis, Asthma, Morbus Crohn, idiopathischer Lungenfibrose und Colitis ulcerosa.

## Claims

1. Drug combination, **characterised in that**, in addition to a PDE4 inhibitor of general formula **1**, 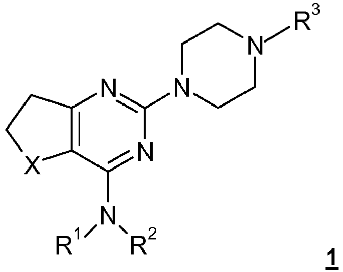 in which
**X** denotes SO,
**R**¹ denotes H,
**R**² is C₁₋₆-alkyl, which may optionally be substituted by one or more groups selected from F, CF₃, CHF₂ or CH₂F or which may optionally be substituted by one or more groups selected from among OR^{2.1}, COOR^{2.1}, CONR^{2.2}R^{2.3}, SR^{2.1}, SO-R^{2.1}, SO₂-R^{2.1}, phenyl, a het, a hetaryl, a monocyclic C₃₋₇-cycloalkyl, CH₂-NR^{2.2}R^{2.3} and NR^{2.2}R^{2.3}, which in turn may optionally be substituted by one or more groups selected from among OH, F, Cl, Br, CF₃, CHF₂, CH₂F, OR^{2.1}, oxo, methyl, ethyl, propyl, isopropyl, C₁₋₂-alkanol, phenyl, COOR^{2.1}, CH₂-NR^{2.2}R^{2.3} and NR^{2.2}R^{2.3},
**R**^{2.1} denotes H or a group selected from among methyl, ethyl, propyl, isopropyl, monocyclic C₃₋₇-cycloalkyl, phenyl-C₁₋₂-alkylene, hetaryl-C₁₋₂-alkylene, het-C₁₋₂-alkylene, C₃₋₇-cycloalkyl-C₁₋₂-alkylene, phenyl, a hetaryl and a het,
which may optionally be substituted by one or more groups selected from among OH, halogen, methyl, ethyl, propyl, isopropyl, O-methyl, O-ethyl, O-propyl, O-isopropyl and phenyl,
**R**^{2.2} and **R^{2.3}** denote, independently of one another, H or a group selected from among methyl, ethyl, propyl, isopropyl, monocyclic C₃₋₇-cycloalkyl, phenyl-C₁₋₃-alkylene, hetaryl-C₁₋₃-alkylene, phenyl, het, hetaryl, CO-NH₂, CO-NHCH₃, CON(CH₃)₂, SO₂-(C₁-C₂-alkyl), CO-R^{2.1} and COOR^{2.1}, which may optionally be substituted by one or more groups selected from among OH, F, Cl, Br, methyl, ethyl, propyl, isopropyl, phenyl and COOR^{2.1},
**het** is a three- to seven-membered, monocyclic, saturated or partly saturated heterocycle which contains 1, 2 or 3 heteroatoms selected independently of one another from among N, S or O,
and
**hetaryl** is a five- to six-membered, monocyclic, aromatic heteroaryl which contains 1, 2 or 3 heteroatoms selected independently of one another from among N, S or O,
and
**cycloalkyl** may be saturated or partly saturated,
or
**R²** denotes a monocyclic C₃₋₇-cycloalkyl, which may optionally be substituted by one group selected from among branched or unbranched C₁₋₂-alkanol, C₁₋₃-fluoroalkyl, C₁₋₃-alkylene-OR^{2.1}, OR^{2.1}, COOR^{2.1}, SO₂-NR^{2.2}R^{2.3}, het, methyl, ethyl, propyl, isopropyl, phenyl, phenyl-C₁₋₂-alkylene, hetaryl-C₁₋₂-alkylene, monocyclic C₃₋₇-cycloalkyl and NR^{2.2}R^{2.3},
which may optionally be substituted by one or more groups selected from among OH, OR^{2.1}, oxo, halogen, CF₃, CHF₂, CH₂F, methyl, ethyl, propyl, isopropyl, phenyl and NR^{2.2}R^{2.3},
or
**R²** denotes a phenyl, which may optionally be substituted by OH, SH, F, Cl or Br or by one or more groups selected from among OR^{2.1}, COOR^{2.1}, NR^{2.2}R^{2.3}, CH₂-NR^{2.2}R^{2.3}, C₃₋₇-cycloalkyl, het, methyl, ethyl, propyl, isopropyl, CF₃, CHF₃, CH₂F, phenyl-C₁₋₂-alkylene, het-C₁₋₂-alkylene, hetaryl-C₁₋₂-alkylene, phenyl, SO₂-CH₃, SO₂-CH₂CH₃ and SO₂-NR^{2.2}R^{2.3},
which in turn may optionally be substituted by one or more groups selected from among OH, OR^{2.1}, oxo, F, Cl, Br, CF₃, CHF₂, CH₂F, methyl, ethyl, propyl, isopropyl, phenyl and NR^{2.2}R^{2.3},
or
**R²** denotes a group selected from among a het and a hetaryl, which may optionally be substituted by one or more groups selected from among F, Cl, Br, OH, oxo, CF₃, CHF₂, CH₂F and SH or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, C₁₋₂-alkanol, C₃₋₁₀-cycloalkyl, phenyl, methyl, ethyl, propyl, isopropyl, phenyl-C₁₋₂-alkylene, hetaryl-C₁₋₂-alkylene, het, hetaryl, C₁₋₂-alkanol and NR^{2.2}R^{2.3},
which in turn may optionally be substituted by one or more groups selected from among OH, OR^{2.1}, oxo, F, Cl, Br, CF₃, CHF₂, CH₂F, C₁₋₆-alkyl, phenyl and NR^{2.2}R^{2.3},
and in which
**R**³ is a group selected from among a saturated or partly saturated, monocyclic, three- to seven-membered heterocyle, a saturated or partly saturated, bicyclic, five- to eleven-membered heterocycle, a monocyclic, five- to six-membered heteroaryl and a bicyclic, seven- to eleven-membered heteroaryl,
which contains in each case 1, 2, 3 or 4 heteroatoms selected independently of one another from among N, O and S
and which may optionally be substituted in each case by one or more groups selected from among
halogen, C₁₋₃-fluoroalkyl, CN, OH, oxo, -C₁₋₆-alkyl, -O-R^{2.1}, -COOR^{2.1}, SO-R^{2.1}, SO₂-R^{2.1}, C₆₋₁₀-aryl, C₁₋₃-alkylene-C₆₋₁₀-aryl, -C₁₋₃-alkylene-NR^{2.2}R^{2.3}, -NR^{2.2}R^{2.3}, a C₃₋₁₀-cycloalkyl, a C₁₋₃-alkylene-C₃₋₁₀-Cycloalkyl, het, a hetaryl, C₁₋₃-alkylene-hetaryl and C₁₋₃-alkylene-het,
which in turn may optionally be substituted by one or more groups selected from among OH, halogen, -C₁₋₃-fluoroalkyl, C₁₋₆-alkyl, C₆₋₁₀-aryl, -COO(C₁₋₃-alkyl) and O-(C₁₋₃-alkyl),
it contains at least one NSAID (= non-steroidal anti-inflammatory drug) (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

2. Drug combination according to claim 1, **characterised in that**, in addition to the PDE4 inhibitor of general formula **1,** in which
**R²** is a group according to formula **3** 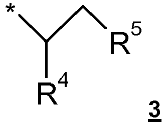
in which **R⁵** is OH or NH₂ and
in which **R⁴** denotes a group selected from among C₁₋₄-alkyl, hetaryl and phenyl, which may optionally be substituted by one or more groups selected from among OH, F, Br, OR^{2.1}, oxo, methyl, ethyl, C₁₋₂-alkanol, phenyl, COOR^{2.1}, CH₂-NR^{2.2}R^{2.3} and NR^{2.2}R^{2.3},
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

3. Drug combination according to claim 2, **characterised in that**, in addition to the PDE4 inhibitor of general formula **1**, in which
**R²** is a group according to formula **3** 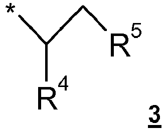
in which **R⁵** is OH or NH₂ and
in which **R⁴** denotes methyl, ethyl, propyl, isopropyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

4. Drug combination according to claim 1, **characterised in that**, in addition to the PDE4 inhibitor of general formula **1**, in which
**R²** is a monocyclic, three-, four-, five-, six- or seven-membered cycloalkyl ring which may optionally be substituted in the spiro position by a group selected from among -CH₂-OR^{2.1}, branched or unbranched C₂₋₆-alkylene-OR^{2.1}, methyl, ethyl, propyl, isopropyl, butyl, isobutyl, cyclopropyl, -CF₃, CHF₂, CH₂F and C₂₋₄-fluoroalkyl, in which
**R^{2.1}** is selected from among methyl, ethyl, propyl, isopropyl, butyl, isobutyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

5. Drug combination according to claim 1, **characterised in that**, in addition to the PDE4 inhibitor of general formula **1**, in which
**R²** is phenyl, which is optionally substituted in one or both meta positions by one or more groups selected from among methyl, ethyl, propyl, isopropyl, cyclopropyl, F, Cl, Br, OH, OR^{2.1}, COOR^{2.1}, CF₃, CHF₂, CH₂F, NH₂ and N(CH₃)₂, in which R^{2.1} may be H, methyl or ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

6. Drug combination according to claim 1, **characterised in that**, in addition to the PDE4 inhibitor of general formula **1**, in which
**R²** denotes a monocyclic, saturated, three-, four-, five-, six- or seven-membered heterocycle having 1, 2 or 3 heteroatoms in each case selected from among N, O and S, which may optionally be substituted by one or more groups selected from among fluorine, chlorine, bromine, CF₃, CHF₂, CH₂F, OH, oxo and SH or by one or more groups selected from among OR^{2.1}, C₁₋₃-alkylene-OR^{2.1}, SR^{2.1}, SO-R^{2.1}, SO₂-R^{2.1}, COOR^{2.1}, COR^{2.1}, C₁₋₆-alkanol, C₃₋₁₀-cycloalkyl, phenyl, C₁₋₆-alkyl, phenyl-C₁₋₆-alkylene, hetaryl-C₁₋₆-alkylene, het, hetaryl and NR^{2.2}R^{2.3}, which in turn may optionally be substituted by one or more groups selected from among OH, OR^{2.1}, oxo, F, Cl, CF₃, CHF₂, CH₂F, C₁₋₆-alkyl, phenyl and NR^{2.2}R^{2.3},
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

7. Drug combination according to claim 6, **characterised in that**, in addition to the PDE4 inhibitor of general formula **1**, in which
**R²** is a monocyclic, saturated, six-membered heterocycle having a heteroatom selected from among N, O and S, which may optionally be substituted by one or more groups selected from among F, Cl, Br, CF₃, CHF₂, CH₂F, OH, oxo, NH₂, NHCH₃, N(CH₃)₂, methyl, ethyl, propyl, isopropyl, cyclopropyl, methoxy and ethoxy,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

8. Drug combination according to one of claims 6 or 7, **characterised in that**, in addition to the PDE4 inhibitor of general formula **1**, in which
**R²** denotes a group selected from among piperidine or tetrahydropyran, which may optionally be substituted by one or more groups selected from among F, Cl, Br, OH, CF₃, CHF₂, CH₂F, NH₂, NHCH₃, N(CH₃)₂, oxo, methyl and methoxy,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

9. Drug combination according to one of claims 1 to 8, **characterised in that**, in addition to the PDE4 inhibitor of general formula **1**, in which
**R³** is a monocyclic, five- or six-membered heteroaryl ring which may optionally be substituted by one or more groups selected from among
F, Cl, Br, CF₃, CHF₂, CH₂F, CN, OH, -methyl, ethyl, propyl, isopropyl, -O-methyl, O-ethyl, -COOmethyl, -COOethyl, SO₂-(CH₃), SO-(CH₃), SO₂-(CH₂CH₃), SO-(CH₂CH₃), phenyl, -methylene-phenyl, -ethylene-phenyl, -NH₂, -NH(CH₃), N(CH₃)₂, -methylene-NH₂, -methylene-NH(CH₃), -methylene-N(CH₃)₂, a C₃₋₆-cycloalkyl, a methylene-C₃₋₆-cycloalkyl, a saturated or partly saturated, five- to six-membered heterocycle, a five- or six-membered heteroaryl, -methylene-hetaryl, and -methylene-het, which in turn may optionally be substituted by one or more groups selected from among OH, F, Cl, Br, CF₃, CHF₂, CH₂F, methyl, ethyl, propyl, isopropyl, phenyl, -COO(CH₃), -O-methyl and -O-ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

10. Drug combination according to one of claims 1 to 8, **characterised in that**, in addition to the inhibitor of general formula 1, in which
**R³** is a bicyclic, 9- to 11-membered, saturated, unsaturated or partly saturated heterocycle, which may optionally be substituted by one or more groups selected from among
F, Cl, Br, CF₃, CHF₂, CH₂F, CN, OH, -methyl, ethyl, propyl, isopropyl, -O-methyl, O-ethyl, -COOmethyl, -COOethyl, SO₂-(CH₃), SO-(CH₃), SO₂-(CH₂CH₃), SO-(CH₂CH₃), phenyl, -methylene-phenyl, -ethylene-phenyl, -NH₂, -NH(CH₃), N(CH₃)₂, -methylene-NH₂, -methylene-NH(CH₃), -methylene-N(CH₃)₂, a -C₃₋₆-cycloalkyl, a -methylene-C₃₋₆-cycloalkyl, a saturated, partly unsaturated or unsaturated, 5- to 6-membered heterocycle, a 5- to 6-membered heteroaryl, -methylene-hetaryl, and -methylene-het, which in turn may optionally be substituted by one or more groups selected from among OH, F, Cl, Br, CF₃, CHF₂, CH₂F, methyl, ethyl, propyl, isopropyl, phenyl, -COO(CH₃), -O-methyl and -O-ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

11. Drug combination according to claim 9, **characterised in that**, in addition to the PDE4 inhibitor of general formula **1**, in which
**R³** is a monocyclic, five- or six-membered heteroaryl ring selected from among pyrrole, pyrazole, furan, thiophene, thiazole, imidazole, oxazole, pyridazine, pyrimidine, pyrazine, thiadiazole, oxadiazole, triazine, isooxazole, isothiazole and pyridine,
which may optionally be substituted by one or more groups selected from among F, Cl, Br, CF₃, CHF₂, CH₂F, CN, OH, -methyl, ethyl, propyl, isopropyl, -O-methyl, O-ethyl, -COOmethyl, -COOethyl, SO₂-(CH₃), SO₂-(CH₂CH₃), phenyl, -methylene-phenyl, -ethylene-phenyl, -NH₂, -NH(CH₃), N(CH₃)₂, -methylene-NH₂, -methylene-NH(CH₃), -methylene-N(CH₃)₂, a C₃₋₆-cycloalkyl, a methylene-C₃₋₆-cycloalkyl, a het, a hetaryl, -methylene-hetaryl, and -methylene-het,
which in turn may optionally be substituted by one or more groups selected from among OH, F, Cl, Br, CF₃, CHF₂, CH₂F, methyl, ethyl, propyl, isopropyl, phenyl, -COO(CH₃), -O-methyl and -O-ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

12. Drug combination according to claim 10, **characterised in that**, in addition to the PDE4 inhibitor of general formula **1**, in which
**R³** denotes a bicyclic 9- to 11-membered heterocycle selected from among benzoxazole, benzodioxole, dihydrobenzodioxin, benzodioxin, benzisoxazole, benzothiazole, benzisothiazole, thienopyrimidine, furopyrimidine, thienopyridine, furopyridine, indole, isoindole, quinoxaline, naphthyridine, pyridopyrazine, pyridopyrimidine, quinoline, isoquinoline, benzoimidazole, 6,7,8,9-tetrahydro-5H-pyrazino[2,3-d]azepine, benzothiophene, benzofuran, quinazoline, indazole, isobenzofuran and pteridine,
which may optionally be substituted by one or more groups selected from among F, Cl, Br, CF₃, CHF₂, CH₂F, CN, OH, -methyl, ethyl, propyl, isopropyl, -O-methyl, O-ethyl, -COOmethyl, -COOethyl, SO₂-(CH₃), SO₂-(CH₂CH₃), phenyl, -methylene-phenyl, -ethylene-phenyl, -NH₂, -NH(CH₃), N(CH₃)₂, -methylene-NH₂, -methylene-NH(CH₃), -methylene-N(CH₃)₂, a C₃₋₆-cycloalkyl, a methylene-C₃₋₆-cycloalkyl, a het, a hetaryl, -methylene-hetaryl, and -methylene-het,
which in turn may optionally be substituted by one or more groups selected from among OH, F, Cl, Br, CF₃, CHF₂, CH₂F, methyl, ethyl, propyl, isopropyl, phenyl, -COO(CH₃), -O-methyl and -O-ethyl,
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

13. Drug combination according to claim 1, **characterised in that**, in addition to the PDE4 inhibitor of general formula **1**,
in which
**R¹** is H,
**R²** is a group selected from among 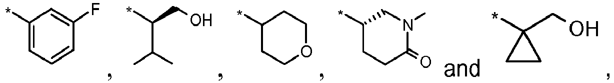
**R³** is a group selected from among 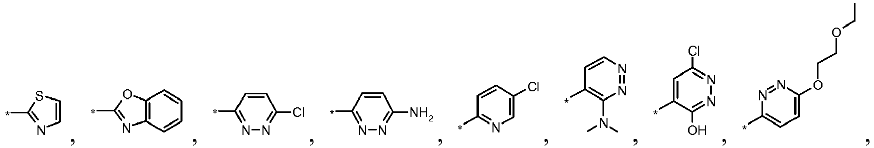 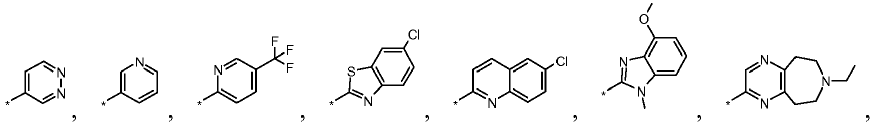 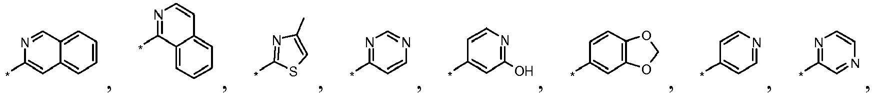 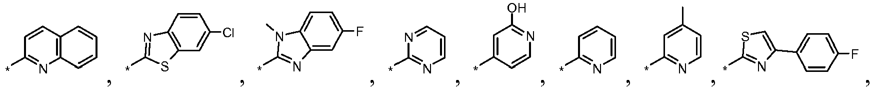 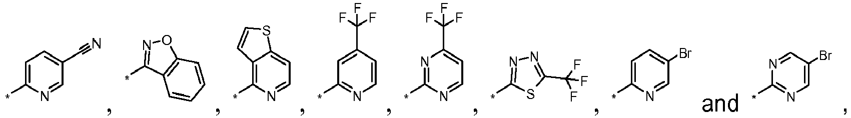
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

14. Drug combinations according to claim 1, **characterised in that**, in addition to the PDE4 inhibitor of general formula **1** selected from among:
1.1 (3-fluorophenyl)-[5-oxo-2-(4-thiazol-2-yl-piperazin-1-yl)-6,7-dihydro-5*H*-5λ⁴-thieno[3,2-*d*]pyrimidin-4-yl]-amine
1.2 (R)-3-methyl-2-[5-oxo-2-(4-thiazol-2-yl-piperazin-1-yl)-6,7-dihydro-5*H*-5λ⁴-thieno[3,2-*d*]pyrimidin-4-ylamino]-butan-1-ol
1.3 [2-(4-benzoxazol-2-yl-piperazin-1-yl)-5-oxo-6,7-dihydro-5*H*-5λ⁴-thieno[3,2-*d*]pyrimidin-4-yl]-(3-fluorophenyl)-amine
1.4 [2-(4-benzoxazol-2-yl-piperazin-1-yl)-5-oxo-6,7-dihydro-5*H*-5λ⁴-thieno[3,2-*d*]pyrimidin-4-yl]-(tetrahydropyran-4-yl)-amine
1.5 (R)-2-{2-[4-(6-chloropyridazin-3-yl)-piperazin-1-yl]-5-oxo-6,7-dihydro-5*H*-5λ⁴-thieno[3,2-*d*]pyrimidin-4-ylamino}-3-methylbutan-1-ol
1.6 {2-[4-(6-chloropyridazin-3-yl)-piperazin-1-yl]-5-oxo-6,7-dihydro-5*H*-5λ⁴-thieno[3,2-*d*]pyrimidin-4-yl}-(3-fluorophenyl)-amine
1.7 (R)-2-[2-(4-benzoxazol-2-yl-piperazin-1-yl)-5-oxo-6,7-dihydro-5*H*-5λ⁴-thieno[3,2-*d*]pyrimidin-4-ylamino]-3-methylbutan-1-ol
1.8 (1-{2-[4-(5-chloropyridin-2-yl)-piperazin-1-yl]-5-oxo-6,7-dihydro-5*H*-5λ⁴-thieno[3,2-*d*]pyrimidin-4-ylamino}-cyclopropyl)-methanol
1.9 {2-[4-(5-chloropyridin-2-yl)-piperazin-1-yl]-5-oxo-6,7-dihydro-5*H*-5λ⁴-thieno[3,2-*d*]pyrimidin-4-yl}-(tetrahydropyran-4-yl)-amine
1.10 {2-[4-(3-dimethylaminopyridazin-4-yl)-piperazin-1-yl]-5-oxo-6,7-dihydro-5*H*-5λ⁴-thieno[3,2-*d*]pyrimidin-4-yl}-(3-fluorophenyl)-amine
1.11 6-chloro-4-{4-[4-(3-fluorophenylamino)-5-oxo-6,7-dihydro-5*H*-5λ⁴-thieno[3,2-*d*]pyrimidin-2-yl]-piperazin-1-yl}-pyridazin-3-ol
1.12 2-{4-[6-(2-ethoxyethoxy)-pyridazin-3-yl]-piperazin-1-yl}-5-oxo-6,7-dihydro-5*H*-5λ⁴-thieno[3,2-*d*]pyrimidin-4-yl)-(3-fluorophenyl)-amine
1.13 (3-fluorophenyl)-[5-oxo-2-(4-pyridazin-4-yl-piperazin-1-yl)-6,7-dihydro-5*H*-5λ⁴-thieno[3,2-*d*]pyrimidin-4-yl]-amine
1.14 (R)-2-{2-[4-(4-methoxy-1-methyl-1*H*-benzimidazol-2-yl)-piperazin-1-yl]-5-oxo-6,7-dihydro-5*H*-5λ⁴-thieno[3,2-*d*]pyrimidin-4-ylamino}-3-methylbutan-1-ol
1.15 (R)-2-{2-[4-(7-ethyl-6,7,8,9-tetrahydro-5*H*-pyrazino[2,3-*d*]azepin-2-yl)-piperazin-1-yl]-5-oxo-6,7-dihydro-5*H*-5λ⁴-thieno[3,2-*d*]pyrimidin-4-ylamino}-3-methylbutan-1-ol
1.16 (R)-3-methyl-2-[5-oxo-2-(4-pyrimidin-4-yl-piperazin-1-yl)-6,7-dihydro-5*H*-5λ⁴-thieno[3,2-*d*]pyrimidin-4-ylamino]-butan-1-ol
1.17 4-{4-[4-((R)-1-hydroxymethyl-2-methylpropylamino)-5-oxo-6,7-dihydro-5H-5λ⁴-thieno[3,2-*d*]pyrimidin-2-yl]-piperazin-1-yl}-pyridin-2-ol
1.18 (R)-3-methyl-2-[5-oxo-2-(4-pyridin-4-yl-piperazin-1-yl)-6,7-dihydro-5*H*-5λ⁴-thieno[3,2-*d*]pyrimidin-4-ylamino]-butan-1-ol
1.19 (R)-2-{2-[4-(3-dimethylaminopyridazin-4-yl)-piperazin-1-yl]-5-oxo-6, 7-dihydro-5H-5^{A}4-thieno[3,2-*d*]pyrimidin-4-ylamino}-3-methylbutan-1-ol
1.20 6-chloro-4-{4-[4-((R)-1-hydroxymethyl-2-methylpropylamino)-5-oxo-6,7-dihydro-5*H*-5λ⁴-thieno[3,2-*d*]pyrimidin-2-yl]-piperazin-1-yl}-pyridazin-3-ol
1.21 (R)-2-(2-{4-[6-(2-ethoxyethoxy)-pyridazin-3-yl]-piperazin-1-yl}-5-oxo-6,7-dihydro-5*H-*5λ⁴-thieno[3,2-*d*]pyrimidin-4-ylamino)-3-methylbutan-1-ol
1.22 (R)-3-methyl-2-[5-oxo-2-(4-pyridazin-4-yl-piperazin-1-yl)-6,7-dihydro-5*H*-5λ⁴-thieno[3,2-*d*]pyrimidin-4-ylamino]-butan-1-ol
1.23 {1-[5-oxo-2-(4-pyrimidin-4-yl-piperazin-1-yl)-6,7-dihydro-5*H*-5λ⁴-thieno[3,2-*d*]pyrimidin-4-ylamino]-cyclopropyl}-methanol
1.24 {1-[5-oxo-2-(4-pyridin-4-yl-piperazin-1-yl)-6,7-dihydro-5*H*-5λ⁴-thieno[3,2-*d*]pyrimidin-4-ylamino]-cyclopropyl}-methanol
1.25 (S)-1-methyl-5-[5-oxo-2-(4-pyrimidin-4-yl-piperazin-1-yl)-6,7-dihydro-5H-5λ4-thieno[3,2-d]pyrimidin-4-ylamino]-piperidin-2-one
1.26 {2-[4-(5-fluoro-1-methyl-1H-benzoimidazol-2-yl)-piperazin-1-yl]-5-oxo-6,7-dihydro-5H-5λ4-thieno[3,2-d]pyrimidin-4-yl}-(tetrahydropyran-4-yl)-amine
1.27 [5-oxo-2-(4-pyridin-4-yl-piperazin-1-yl)-6,7-dihydro-5H-5λ4-thieno[3,2-d]pyrimidin-4-yl]-(tetrahydropyran-4-yl)-amine
1.28 (3-fluorophenyl)-{2-[4-(4-methoxy-1-methyl-1H-benzimidazol-2-yl)-piperazin-1-yl]-5-oxo-6,7-dihydro-5H-5A4-thieno[3,2-d]pyrimidin-4-yl}-amine
1.29 {2-[4-(7-ethyl-6,7,8,9-tetrahydro-5H-pyrazino[2,3-d]azepin-2-yl)-piperazin-1-yl]-5-oxo-6,7-dihydro-5H-5λ4-thieno[3,2-d]pyrimidin-4-yl}-(3-fluorophenyl)-amine
1.30 (3-fluorophenyl)-[5-oxo-2-(4-pyrimidin-4-yl-piperazin-1-yl)-6,7-dihydro-5H-5λ4-thieno[3,2-d]pyrimidin-4-yl]-amine
1.31 4-{4-[4-(3-fluorophenylamino)-5-oxo-6,7-dihydro-5H-5λ4-thieno[3,2-d]pyrimidin-2-yl]-piperazin-1-yl}-pyridin-2-ol
1.32 (3-fluorophenyl)-[5-oxo-2-(4-pyridin-4-yl-piperazin-1-yl)-6,7-dihydro-5H-5λ⁴-thieno[3,2-d]pyrimidin-4-yl]-amine
1.33 (3-fluorophenyl)-(2-{4-[4-(4-fluorophenyl)-thiazol-2-yl]-piperazin-1-yl}-5-oxo-6,7-dihydro-5H-5λ4-thieno[3,2-d]pyrimidin-4-yl)-amine
1.34 [2-(4-benzo[d]isoxazol-3-yl-piperazin-1-yl)-5-oxo-6,7-dihydro-5H-5λ4-thieno[3,2-d]pyrimidin-4-yl]-(3-fluorophenyl)-amine
1.35 (R)-2-(2-{4-[4-(4-fluorophenyl)-thiazol-2-yl]-piperazin-1-yl}-5-oxo-6,7-dihydro-5H-5λ4-thieno[3,2-d]pyrimidin-4-ylamino)-3-methylbutan-1-ol
1.36 (R)-2-[2-(4-benzo[d]isoxazol-3-yl-piperazin-1-yl)-5-oxo-6,7-dihydro-5H-5λ4-thieno[3,2-d]pyrimidin-4-ylamino]-3-methylbutan-1-ol
it contains at least one NSAID (**2**) selected from among celecoxib (2.25), etoricoxib (2.41), lumiracoxib (2.74), parecoxib (2.88), rofecoxib (2.101) and valdecoxib (2.125), acetylsalicylic acid (2.3), meloxicam (2.77), naproxen (2.82) and piroxicam (2.93).

15. Drug combination according to one of claims 1 to 14, **characterised in that**, in addition to the PDE4 inhibitor of general formula **1**, it contains at least one NSAID (**2**) selected from among celecoxib (2.25), etoricoxib (2.41), lumiracoxib (2.74), parecoxib (2.88), rofecoxib (2.101) and valdecoxib (2.125).

16. Drug combination according to one of claims 1 to 14 for the treatment of a disease selected from among respiratory diseases, pulmonary diseases, gastrointestinal ailments and diseases and also inflammatory diseases of the joints, skin or eyes, cancers, and diseases of the peripheral or central nervous system.

17. Drug combination according to claim 16 for the treatment of a disease selected from COPD, chronic sinusitis, asthma, Crohn's disease, idiopathic pulmonary fibrosis and ulcerative colitis.

## Revendications

1. Combinaison de médicaments, **caractérisée en ce qu'**elle comprend, en plus d'un inhibiteur PDE4 de formule générale 1, 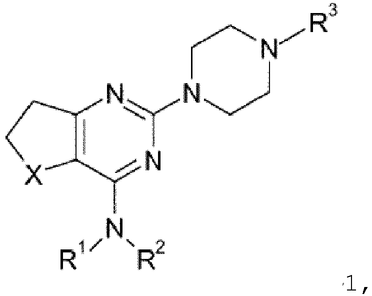 dans laquelle
X est SO,
R¹ est H,
R² est C₁₋₆ alkyle, qui peut être substitué le cas échéant par un ou plusieurs résidus choisis parmi F, CF₃, CHF₂ ou CH₂F ou qui peut être substitué le cas échéant par un ou plusieurs résidus choisis dans le groupe constitué deconstitué de OR^{2.1}, COOR^{2.1} CONR^{2.2}R^{2.3}, SR^{2.1}, SO-R^{2.1}, SO₂-R^{2.1}, phényle, un hét, un hétaryle, un C₃₋₇ cycloalkyle monocyclique, CH₂-NR^{2.2}R^{2.3} et NR^{2.2}R^{2.3},
qui peut de nouveau être substitué le cas échéant par un ou plusieurs résidus choisis dans le groupe constitué deconstitué de OH, F, Cl, Br, CF₃, CHF₂, CH₂F, OR^{2.1}, oxo, méthyle, éthyle, propyle, isopropyle, C₁₋₂ alcanol, phényle, COOR^{2.1}, CH₂-NR^{2.2}R^{2.3} et NR^{2.2}R^{2.3},
dans laquelle R^{2.1} est H ou un résidu choisi dans le groupe constitué deconstitué de méthyle, éthyle, propyle, isopropyle, C₃₋₇ cycloalkyle monocyclique, phényl-C₁₋₂ alkylène, hétaryl-C₁₋₂ alkylène, hét-C₁₋₂ alkylène, C₃₋₇ cycloalkyle-C₁₋₂ alkylène, phényle, un hétaryle et un hét,
qui peut être le cas échéant substitué par un ou plusieurs résidus choisis dans le groupe constitué deconstitué de OH, halogène, méthyle, éthyle, propyle, isopropyle, 0-méthyle, 0-éthyle, O-propyle, 0-isopropyle et phényle,
dans laquelle R^{2.2} et R^{2.3} sont, indépendamment l'un de l'autre, H ou un résidu choisi dans le groupe constitué deconstitué de méthyle, éthyle, propyle, isopropyle, C₃₋₇ cycloalkyle monocyclique, phényl-C₁₋₃ alkylène, hétaryl-C₁₋₃ alkylène, phényle, hét, hétaryle, CO-NH₂, CO-NHCH₃, CON(CH₃)₂, SO₂-(C₁-C₂ alkyle), CO-R^{2.1} et COOR^{2.1},
qui peut être le cas échéant substitué par un ou plusieurs résidus choisis dans le groupe constitué deconstitué de OH, F, Cl, Br, méthyle, éthyle, propyle, isopropyle, phényle et COOR^{2.1},
dans laquelle
hét est un hétérocycle saturé ou partiellement saturé, monocyclique, de trois à sept chaînons, qui contient 1, 2 ou 3 hétéroatomes choisis, indépendamment les uns des autres, dans le groupe constitué deconstitué de N, S, ou 0,
et dans laquelle
hétaryle est un hétéroaryle aromatique, monocyclique, de cinq à six chaînons, qui contient 1, 2 ou 3 hétéroatomes choisis, indépendamment les uns des autres, dans le groupe constitué deconstitué de N, S ou O,
et dans laquelle
cycloalkyle peut être saturé ou partiellement saturé,
ou
R² est un C₃₋₇ cycloalkyle monocyclique, qui peut être substitué le cas échéant par un résidu choisi dans le groupe constitué de C₁₋₂ alcanol ramifié ou non ramifié, C₁₋₃ fluoroalkyle, C₁₋₃ alkylène-OR^{2.1}, OR^{2.1}, COOR^{2.1}, SO₂-NR^{2.2}R^{2.3}, hét, méthyle, éthyle, propyle, isopropyle, phényle, phényl-C₁₋₂ alkylène, hétaryl-C₁₋₂ alkylène, C₃₋₇ cycloalkyle monocyclique et NR^{2.2}R^{0.3},
qui peut être substitué le cas échéant par un résidu choisi dans le groupe constitué de OH, OR^{2.1}, oxo, halogène, CF₃, CHF₂, CH₂F, méthyle, éthyle, propyle, isopropyle, phényle, et NR^{2.2}R^{2.3},
ou
R² est un phényle, qui peut être substitué le cas échéant par OH, SH, F, Cl ou Br ou par un ou plusieurs résidus choisis dans le groupe constitué de OR^{2.1}, COOR^{2.1}, NR^{2.2}R^{2.3}, CH₂-NR^{2.2}R^{2.3}, C₃₋₇ cycloalkyle, hét, méthyle, éthyle, propyle, isopropyle, CF₃, CHF₂, CH₂F, phényl-C₁₋₂ alkylène, hét-C₁₋₂ alkylène, hétaryl-C₁₋₂ phényle, SO₂-CH₃, SO₂-CH₂CH₃ et SO₂-NR^{2.2}R^{2.3},
qui peut de nouveau être substitué le cas échéant par un ou plusieurs résidus choisis dans le groupe constitué de OH, OR^{2.1}, oxo, F, Cl, Br, CF₃, CHF₂, CH₂F, méthyle, éthyle, propyle, isopropyle, phényle, et NR^{2.2}R^{2.3},
ou
R² est un résidu choisi dans un groupe constitué d'un hét et d'un hétaryle, qui peut être substitué le cas échéant par un ou plusieurs résidus choisis dans le groupe F, Cl, Br, OH, oxo, CF₃, CHF₂, CH₂F, et SH ou par un ou plusieurs résidus choisis dans le groupe OR^{2.1}, C₁₋₃ alkylène-OR^{2.1}, SR^{2.1}, SO-R^{2.1}, SO₂-R^{2.1}, COOR^{2.1}, COR^{2.1}, C₁₋₂ alcanol, C₃₋₁₀ cycloalkyle, phényle, méthyle, éthyle, propyle, isopropyle, phényl-C₁₋₂ hétaryl-C₁₋₂ hét, hétaryle, C₁₋₂ alcanol, et NR^{2.2}R^{2.3},
qui peut de nouveau être substitué le cas échéant par un ou plusieurs résidus choisis dans le groupe constitué de OH, OR^{2.1}, oxo, F, Cl, Br, CF₃, CHF₂, CH₂F, C₁₋₆ alkyle, phényle, et NR^{2.2}R^{2.3},
et dans laquelle
R³ est un résidu choisi dans le groupe constitué d'un hétérocycle saturé ou partiellement saturé, monocyclique, de trois à sept chaînons, d'un hétérocycle saturé ou partiellement saturé, bicyclique de cinq à onze chaînons, d'un hétéroaryle monocyclique, de cinq à six chaînons et d'un hétéroaryle bicyclique de sept à onze chaînons,
qui contient respectivement 1, 2, 3 ou 4 hétéroatomes choisis, indépendamment les uns des autres, dans le groupe constitué de N, 0 et S
et qui peut être substitué respectivement le cas échéant par un ou plusieurs résidus choisis dans le groupe constitué de halogène, C₁₋₃ fluoroalkyle, CN, OH, oxo, -C₁₋₆ alkyle, -O-R^{2.1}, -COOR^{2.1}, SO-R^{2.1}, SO₂-R^{2.1}, C₆₋₁₀ aryle, -C₁₋₃ alkylène-C₆₋₁₀ aryle, -C₁₋₃ alkylène-NR^{2.2}R^{2.3}, -NR^{2.2}R^{2.3}, un C₃₋₁₀ cycloalkyle, un C₁₋₃ alkylène-C₃₋₁₀ cycloalkyle, hét, un hétaryle, C₁₋₃ alkylène-hétaryle, et un C₁₋₃ alkylène-hét,
qui peut de nouveau être substitué le cas échéant par un ou plusieurs résidus choisis dans le groupe OH, halogène, C₁₋₃ fluoroalkyle, C₁₋₆ alkyle, C₆₋₁₀ aryle, - COO(C₁₋₃ alkyle) et O-(C₁₋₃ alkyle),
au moins un AINS (= anti-inflammatoire non stéroïdien) (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

2. Combinaisons de médicaments selon la revendication 1, **caractérisées en ce qu'**elles comprennent en plus de l'inhibiteur PDE4 de formule générale 1, dans laquelle
R² est un résidu de formule 3 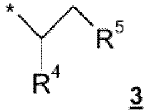 dans laquelle R⁵ est OH ou NH₂, et
dans laquelle R⁴ est un résidu choisi dans le groupe constitué de C₁₋₄ alkyle, hétaryle et phényle,
qui peut être substitué le cas échéant par un ou plusieurs résidus choisis dans le groupe constitué de OH, F, Br, OR^{2.1}, oxo, méthyle, éthyle, C₁₋₂ alcanol, phényle, COOR^{2.1}, CH₂-NR^{2.2}R^{2.3} et NR^{2.2}R^{2.3},
au moins un AINS (= anti-inflammatoire non stéroïdien) (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

3. Combinaison de médicaments selon la revendication 2, **caractérisée en ce qu'**elle comprend en plus de l'inhibiteur PDE4 de formule générale 1, dans laquelle
R² est un résidu de formule 3 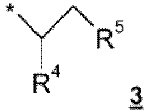
dans laquelle R⁵ est OH ou NH₂, et
dans laquelle R⁴ est méthyle, éthyle, propyle, isopropyle,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

4. Combinaison de médicaments selon la revendication 1, **caractérisée en ce qu'**elle comprend en plus de l'inhibiteur PDE4 de formule générale 1, dans laquelle
R² est un cycle cycloalkyle monocyclique de trois, quatre, cinq, six ou sept chaînons, qui peut être le cas échéant substitué en position spiro par un résidu choisi dans le groupe constitué de -CH₂-OR^{2.1}, C₂₋₆ alkylène linéaire ou ramifié -OR^{2.1}, méthyle, éthyle, propyle, isopropyle, butyle, isobutyle, cyclopropyle, - CF₃, CHF₂, CH₂F, et C₂₋₄ fluoroalkyle, dans laquelle
R^{2.1} est choisi dans le groupe constitué de méthyle, éthyle, propyle, isopropyle, butyle, isobutyle,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

5. Combinaison de médicaments selon la revendication 1, **caractérisée en ce qu'**elle comprend en plus de l'inhibiteur PDE4 de formule générale 1, dans laquelle
R² est un phényle, qui peut être le cas échéant substitué en une ou en deux positions méta par un ou plusieurs résidus choisis dans le groupe constitué de méthyle, éthyle, propyle, isopropyle, cyclopropyle, F, Cl, Br, OH, OR^{2.1}, COOR^{2.1}, CF₃, CHF₂, CH₂F, NH₂ et N(CH₃)₂, dans laquelle R^{2.1} peut être H, méthyle ou éthyle,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

6. Combinaison de médicaments selon la revendication 1, **caractérisée en ce qu'**elle comprend en plus de l'inhibiteur PDE4 de formule générale 1, dans laquelle
R² est un hétérocycle monocyclique saturé, de trois, quatre, cinq, six ou sept chaînons ayant 1, 2 ou 3 hétéroatomes respectivement choisis dans le groupe constitué de N, 0 et S, qui peut être le cas échéant substitué par un ou plusieurs résidus choisis dans le groupe constitué de fluor, chlore, brome, CF₃, CHF₂, CH₂F, OH, oxo et SH ou par un ou plusieurs résidus choisis dans le groupe constitué de OR^{2.1}, C₁₋₃ alkylène-OR^{2.1}, SR^{2.1}, SO-R^{2.1}, SO₂-R^{2.1}, COOR^{2.1}, COR^{2.1}, C₁₋₆ alcanol, C₃₋₁₀ cycloalkyle, phényle, C₁₋₆ alkyle, phényl-C₁₋₆ hétaryl-C₁₋₆ hét, hétaryle, et NR^{2.2}R^{2.3}, qui peut de nouveau être le cas échéant substitué par un ou plusieurs résidus choisis dans le groupe constitué de OH, OR^{2.1}, oxo, F, Cl, CF₃, CHF₂, CH₂F, C₁₋₆ alkyle, phényle, et NR^{2.2}R^{2.3},
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

7. Combinaison de médicaments selon la revendication 6, **caractérisée en ce qu'**elle comprend en plus de l'inhibiteur PDE4 de formule générale 1, dans laquelle
R² est un hétérocycle monocyclique saturé de six chaînons ayant un hétéroatome choisi dans le groupe constitué de N, 0 et S, qui peut être le cas échéant substitué par un ou plusieurs résidus choisis dans le groupe constitué de F, Cl, Br, CF₃, CHF₂, CH₂F, OH, oxo, NH₂, NHCH₃, N(CH₃)₂, méthyle, éthyle, propyle, isopropyle, cyclopropyle, méthoxy, et éthoxy,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

8. Combinaison de médicaments selon l'une des revendications 6 ou 7, **caractérisée en ce qu'**elle comprend en plus de l'inhibiteur PDE4 de formule générale 1, dans laquelle
R² est un résidu choisi dans un groupe constitué de pipéridine ou de tétrahydropyrane, qui peut être le cas échéant substitué par un ou plusieurs résidus choisis dans le groupe constitué de F, Cl, Br, OH, CF₃, CHF₂, CH₂F, NH₂, NHCH₃, N(CH₃)₂, oxo, méthyle, et méthoxy,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

9. Combinaison de médicaments selon l'une des revendications 1 à 8, **caractérisée en ce qu'**elle comprend en plus de l'inhibiteur PDE4 de formule générale 1, dans laquelle
R³ est un cycle hétéroaryle monocyclique de cinq ou six chaînons, qui peut être substitué le cas échéant par un ou plusieurs résidus choisis dans le groupe constitué de F, Cl, Br, CF₃, CHF₂, CH₂F, CN, OH, - méthyle, éthyle, propyle, isopropyle, -0-méthyle, -0-éthyle, -COO-méthyle, -COO-éthyle, SO₂-(CH₃), SO-(CH₃), SO₂-(CH₂CH₃), SO-(CH₂CH₃), phényle, -méthylène-phényle, -éthylène-phényle, -NH₂, -NH(C_{H}3), N(CH₃)₂, -méthylène-NH₂, -méthylène-NH (CH₃), -méthylène-N (CH₃)₂, un C₃₋₆ cycloalkyle, un méthylène-C₃₋₆ cycloalkyle, un hétérocycle saturé ou partiellement saturé, de cinq à six chaînons, un hétéroaryle de cinq ou six chaînons, - méthylène-hétaryle, et -méthylène-hét, qui peut de nouveau être substitué le cas échéant par un ou plusieurs résidus choisis dans le groupe OH, F, Cl, Br, CF₃, CHF₂, CH₂F, méthyle, éthyle, propyle, isopropyle, phényle, -COO(CH₃), -0-méthyle et 0-éthyle,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

10. Combinaison de médicaments selon l'une des revendications 1 à 8, **caractérisée en ce qu'**elle comprend en plus de l'inhibiteur de formule générale 1, dans laquelle
R³ est un hétérocycle bicyclique de 9 à 11 chaînons, saturé, insaturé ou partiellement saturé, qui peut être substitué le cas échéant par un ou plusieurs résidus choisis dans le groupe constitué de F, Cl, Br, CF₃, CHF₂, CH₂F, CN, OH, -méthyle, éthyle, propyle, isopropyle, -0-méthyle, 0-éthyle, -COO-méthyle, -COO-éthyle, SO₂-(CH₃), SO-(CH₃), SO₂-(CH₂CH₃), SO-(CH₂CH₃) , phényle, -méthylène-phényle, -éthylène-phényle, -NH₂, - NH(CH₃), N(CH₃)₂, -méthylène-NH₂, -méthylène-NH(CH₃), - méthylène-N (CH₃)₂, un C₃₋₆ cycloalkyle, un méthylène-C₃₋₆ cycloalkyle, un hétérocycle saturé, partiellement insaturé ou insaturé, de 5 à 6 chaînons, un hétéroaryle de 5 à 6 chaînons, -méthylène-hétaryle, et -méthylène-hét, qui peut de nouveau être substitué le cas échéant par un ou plusieurs résidus choisis dans le groupe OH, F, Cl, Br, CF₃, CHF₂, CH₂F, méthyle, éthyle, propyle, isopropyle, phényle, -COO(CH₃), -0-méthyle et -O-éthyle,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

11. Combinaison de médicaments selon la revendication 9, **caractérisée en ce qu'**elle comprend en plus de l'inhibiteur PDE4 de formule générale 1, dans laquelle
R³ est un cycle hétéroaryle monocyclique de cinq ou six chaînons, choisi dans le groupe constitué de pyrrole, pyrazole, furane, thiophène, thiazole, imidazole, oxazole, pyridazine, pyrimidine, pyrazine, thiadiazole, oxadiazole, triazine, isooxazole, isothiazole et pyridine,
qui peut être substitué le cas échéant par un ou plusieurs résidus choisis dans le groupe constitué de F, Cl, Br, CF₃, CHF₂, CH₂F, CN, OH, -méthyle, éthyle, propyle, isopropyle, -O-méthyle, -O-éthyle, -COO-méthyle, -COO-éthyle, SO₂-(CH₃), SO₂-(CH₂CH₃), phényle, -méthylène-phényle, -éthylène-phényle, -NH₂, -NH(CH₃), N(CH₃)₂, -méthylène-NH₂, -méthylène-NH(CH₃), -méthylène-N(CH₃)₂, un C₃₋₆ cycloalkyle, un méthylène-C₃₋₆ cycloalkyle, un hét, un hétaryle, -méthylène-hétaryle, et -méthylène-hét,
qui peut de nouveau être substitué le cas échéant par un ou plusieurs résidus choisis dans le groupe OH, F, Cl, Br, CF₃, CHF₂, CH₂F, méthyle, éthyle, propyle, isopropyle, phényle, -COO(CH₃), -0-méthyle et O-éthyle,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

12. Combinaison de médicaments selon la revendication 10, **caractérisée en ce qu'**elle comprend en plus de l'inhibiteur PDE4 de formule générale 1, dans laquelle
R³ est un hétérocycle bicyclique de 9 à 11 chaînons, choisi dans le groupe constitué de benzoxazole, benzodioxole, dihydrobenzodioxine, benzodioxine, benzisoxazole, benzothiazole, benzisothiazole, thiénopyrimidine, furopyrimidine, thiénopyridine, furopyridine, indole, isoindole, quinoxaline, naphtyridine, pyridopyrazine, pyridopyrimidine, quinoline, isoquinoline, benzoimidazole, 6,7,8,9-tétrahydro-5H-pyrazino[2,3-d]azépine, benzothiophène, benzofurane, quinazoline, indazole, isobenzofurane et ptéridine, qui peut être substitué le cas échéant par un ou plusieurs résidus choisis dans le groupe constitué de F, Cl, Br, CF₃, CHF₂, CH₂F, CN, OH, -méthyle, éthyle, propyle, isopropyle, -0-méthyle, -0-éthyle, -COO-méthyle, -COO-éthyle, SO₂-(CH₃), SO₂-(CH₂CH₃), phényle, -méthylène-phényle, -éthylène-phényle, -NH₂, -NH(CH₃), N(CH₃)₂, - méthylène-NH₂, -méthylène-NH (CH₃), -méthylène-N (CH₃)₂, un C₃₋₆ cycloalkyle, un méthylène-C₃₋₆ cycloalkyle, un hét, un hétaryle, -méthylène-hétaryle, et -méthylène-hét, qui peut de nouveau être substitué le cas échéant par un ou plusieurs résidus choisis dans le groupe OH, F, Cl, Br, CF₃, CHF₂, CH₂F, méthyle, éthyle, propyle, isopropyle, phényle, -COO(CH₃), -0-méthyle et O-éthyle,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

13. Combinaison de médicaments selon la revendication 1, **caractérisée en ce qu'**elle comprend en plus de l'inhibiteur PDE4 de formule générale 1, dans laquelle
R¹ est H,
R² est un résidu choisi dans le groupe constitué de 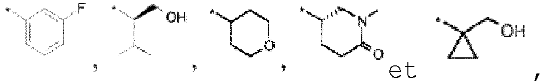 R³ est un résidu choisi dans le groupe constitué de 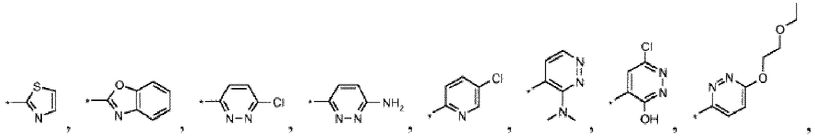 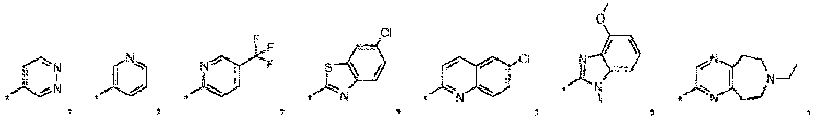 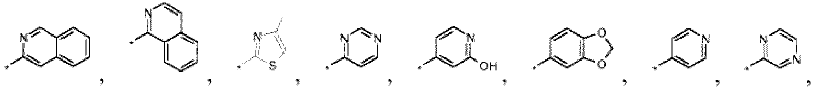 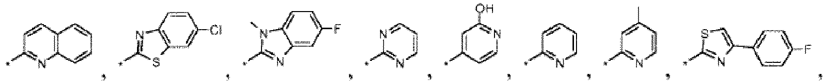 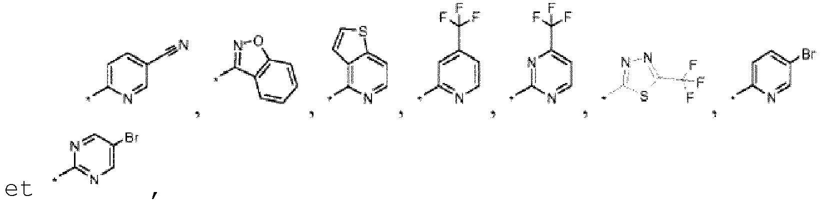 au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

14. Combinaison de médicaments selon la revendication 1, **caractérisée en ce qu'**elle comprend en plus de l'inhibiteur PDE4 de formule générale 1 choisi dans le groupe constitué de :
1.1. (3-fluorophényl)-[5-oxo-2-(4-thiazol-2-yl-pipérazin-1-yl)-6,7-dihydro-5H-5λ⁴-thiéno[3,2-*d*]pyrimidin-4-yl]-amine
1.2. (R)-3-méthyl-2-[5-oxo-2-(4-thiazol-2-yl-pipérazin-1-yl)-6,7-dihydro-5H-5A⁴-thiéno[3,2-*d*]pyrimidin-4-ylamino]-butan-l-ol
1.3. [2-(4-benzoxazol-2-yl-pipérazin-1-yl)-5-oxo-6,7-dihydro-5*H*-5λ⁴-thiéno[3,2-*d*]pyrimidin-4-yl]-(3-fluorophényl)-amine
1.4. [2-(4-benzoxazol-2-yl-pipérazin-1-yl)-5-oxo-6,7-dihydro-5*H*-5λ⁴-thiéno[3,2-*d*]pyrimidin-4-yl]-(tétrahydropyran-4-yl)-amine
1.5. (R)-2-{2-[4-(6-chloropyridazin-3-yl)-pipérazin-1-yl]-5-oxo-6,7-dihydro-5*H*-5λ⁴-thiéno[3,2-*d*]pyrimidin-4-ylamino}-3-méthylbutan-1-ol
1.6. {2-[4-(6-chloropyridazin-3-yl)-pipérazin-1 - yl]-5-oxo-6,7-dihydro-5*H*-5λ⁴-thiéno[3,2-d]pyrimidin-4-yl}-(3-fluorophényl)-amine
1.7. (R)-2-[2-(4-benzoxazol-2-yl-pipérazin-1-yl)-5-oxo-6,7-dihydro-5*H*-5λ⁴-thiéno[3,2-*d*]pyrimidin-4-ylamino]-3-méthylbutan-1-ol
1.8. (1-{2-[4-(5-chloropyridin-2-yl)-pipérazin-1-yl]-5-oxo-6,7-dihydro-5*H*-5λ⁴-thiéno[3,2-*d*]pyrimidin-4-ylamino}-cyclopropyl)-méthanol
1.9. {2-[4-(5-chloropyridin-2-yl)-pipérazin-1-yl]-5-oxo-6,7-dihydro-5*H*-5λ⁴-thiéno[3,2-*d*]pyrimidin-4-yl}-(tétrahydropyran-4-yl)-amine
1.10. {2-[4-(3-diméthylaminopyridazin-4-yl)-pipérazin-1-yl]-5-oxo-6,7-dihydro-5*H*-5λ⁴-thiéno[3,2-*d*]pyrimidin-4-yl}-(3-fluorophényl)-amine
1.11. 6-chloro-4-{4-[4-(3-fluorophénylamino)-5-oxo-6,7-dihydro-5*H*-5λ⁴-thiéno[3,2-*d*]pyrimidin-2-yl]-pipérazin-1-yll-pyridazin-3-ol
1.12. 2-{4-[6-(2-éthoxyéthoxy)-pyridazin-3-yl]-pipérazin-1-yl}-5-oxo-6,7-dihydro-5*H*-5λ⁴-thiéno[3,2-*d*]pyrimidin-4-yl)-(3-fluorophényl)-amine
1.13. (3-fluorophényl)-[5-oxo-2-(4-pyridazin-4-yl-pipérazin-1-yl)-6,7-dihydro-5*H*-5λ⁴-thiéno[3,2-*d*]pyrimidin-4-yl]-aminé
1.14. (R)-2-{2-[4-(4-méthoxy-1-méthyl-1H-benzimidazol-2-yl)-pipérazin-1-yl]-5-oxo-6,7-dihydro-5*H*-5λ⁴-thiéno[3,2-*d*]pyrimidin-4-ylamino}-3-méthylbutan-1-ol
1.15. (R)-2-{2-[4-(7-éthyl-6,7,8,9-tétrahydro-5*H-*pyrazino[2,3-*d*]azépin-2-yl)-pipérazin-1-yl]-5-oxo-6,7-dihydro-5*H*-5λ⁴-thiéno[3,2-d]pyrimidin-4-ylamino}-3-méthylbutan-1-ol
1.16. (R)-3-méthyl-2-[5-oxo-2-(4-pyrimidin-4-yl-pipérazin-1-yl)-6,7-dihydro-5*H*-5λ⁴-thiéno[3,2-*d*]pyrimidin-4-ylamino]-butan-l-ol
1.17. 4-{4-[4-((R)-1-hydroxyméthyl-2-méthylpropylamino)-5-oxo-6,7-dihydro-5*H*-5λ⁴-thiéno[3,2-*d*]pyrimidin-2-yl]-pipérazin-1-yl}-pyridin-2-ol
1.18. (R)-3-méthyl-2-[5-oxo-2-(4-pyridin-4-yl-pipérazin-1-yl)-6,7-dihydro-5*H*-5λ⁴-thiéno[3,2-*d*]pyrimidin-4-ylamino]-butan-l-ol
1.19. (R)-2-{2-[4-(3-diméthylaminopyridazin-4-yl)-pipérazin-1-yl]-5-oxo-6,7-dihydro-5*H*-5λ⁴-thiéno[3,2-*d*]pyrimidin-4-ylamino}-3-méthylbutan-1-ol
1.20. 6-chloro-4-{4-[4-((R)-1-hydroxyméthyl-2-méthylpropylamino)-5-oxo-6,7-dihydro-5*H*-5λ⁴-thiéno[3,2-*d*]pyrimidin-2-yl]-pipérazin-1-yl}-pyridazin-3-ol
1.21. (R)-2-(2-{4-[6-(2-éthoxyéthoxy)-pyridazin-3-yl]-pipérazin-1-yl}-5-oxo-6,7-dihydro-5*H*-5λ⁴-thiéno[3,2-*d*]pyrimidin-4-ylamino)-3-méthylbutan-1-ol
1.22. (R)-3-méthyl-2-[5-oxo-2-(4-pyridazin-4-yl-pipérazin-1-yl)-6,7-dihydro-5*H*-5λ⁴-thiéno[3,2-d]pyrimidin-4-ylamino]-butan-1-ol
1.23. {1-[5-oxo-2-(4-pyrimidin-4-yl-pipérazin-1-yl)-6,7-dihydro-5*H*-5λ⁴-thiéno[3,2-d]pyrimidin-4-ylamino]-cyclopropyl}-méthanol
1.24. {1-[5-oxo-2-(4-pyridin-4-yl-pipérazin-1-yl)-6,7-dihydro-5*H*-5λ⁴-thiéno[3,2-*d*]pyrimidin-4-ylamino]-cyclopropyl}-méthanol
1.25. (S)-1-méthyl-5-[5-oxo-2-(4-pyrimidin-4-yl-pipérazin-1-yl)-6,7-dihydro-5*H*-5λ4-thiéno[3,2-d]pyrimidin-4-ylamino]-pipéridin-2-one
1.26. {2-[4-(5-fluoro-1-méthyl-1H-benzoimidazol-2-yl)-pipérazin-1-yl]-5-oxo-6,7-dihydro-5*H*-5λ4-thiéno[3,2-d]pyrimidin-4-yl}-(tétrahydropyran-4-yl)-amine
1.27. [5-oxo-2-(4-pyridin-4-yl-pipérazin-1-yl)-6,7-dihydro-5*H*-5λ4-thiéno[3,2-*d*]pyrimidin-4-yl]-(tétrahydropyran-4-yl)-amine
1.28. (3-fluorophényl)-{2-[4-(4-méthoxy-1-méthyl-1H-benzimidazol-2-yl)-pipérazin-1-yl]-5-oxo-6,7-dihydro-5*H*-5λ4-thiéno[3,2-*d*]pyrimidin-4-yl}-amine
1.29. {2-[4-(7-éthyl-6,7,8,9-tétrahydro-5H-pyrazino[2,3-d]azépin-2-yl)-pipérazin-1-yl]-5-oxo-6,7-dihydro-5*H*-5λ4-thiéno[3,2-*d*]pyrimidin-4-yl}-(3-fluorophényl)-amine
1.30. (3-fluorophényl)-[5-oxo-2-(4-pyrimidin-4-yl-pipérazin-1-yl)-6,7-dihydro-5*H*-5λ4-thiéno[3,2-*d*]pyrimidin-4-yl]-amine
1.31. 4-{4-[4-(3-fluorophénylamino)-5-oxo-6,7-dihydro-5*H*-5λ4-thiéno[3,2-*d*]pyrimidin-2-yl]-pipérazin-1-yl}-pyridin-2-ol
1.32. (3-fluorophényl)-[5-oxo-2-(4-pyridin-4-yl-pipérazin-1-yl)-6,7-dihydro-5*H*-5λ4-thiéno[3,2-d]pyrimidin-4-yl]-amine
1.33. (3-fluorophényl)-(2-{4-[4-(4-fluorphényl)-thiazol-2-yl]-pipérazin-1-yl}-5-oxo-6,7-dihydro-5*H*-5λ4-thiéno[3,2-d]pyrimidin-4-yl)-amine
1.34. [2-(4-benzo[d]isoxazol-3-yl-pipérazin-1-yl)-5-oxo-6,7-dihydro-5*H*-5λ4-thiéno[3,2-d]pyrimidin-4-yl]-(3-fluorophényl)-amine
1.35. (R)-2-(2-{4-[4-(4-fluorophényl)-thiazol-2-yl]-pipérazin-1-yl}-5-oxo-6,7-dihydro-5*H*-5λ4-thiéno[3,2-*d*]pyrimidin-4-ylamino)-3-méthylbutan-1-ol
1.36. (R)-2-[2-(4-benzo[d]isoxazol-3-yl-pipérazin-1-yl)-5-oxo-6,7-dihydro-5*H*-5λ4-thiéno[3,2-*d*]pyrimidin-4-ylamino]-3-méthylbutan-1-ol,
au moins un AINS (2) choisi dans le groupe constitué de célécoxib (2.25), étoricoxib (2.41), lumiracoxib (2.74), parécoxib (2.88), rofécoxib (2.101) et valdécoxib (2.125), acide acétylsalicylique (2.3), méloxicam (2.77), naproxène (2.82) et piroxicam (2.93).

15. Combinaison de médicaments selon l'une des revendications 1 à 14, **caractérisée en ce qu'**elle comprend en plus de l'inhibiteur PDE4 de formule générale 1, un AINS (2) choisi dans le groupe constitué de célécoxib (2.25), étoricoxib (2.41), lumiracoxib (2.74), parécoxib (2.88), rofécoxib (2.101) et valdécoxib (2.125).

16. Combinaison de médicaments selon l'une des revendications 1 à 14 pour le traitement d'une maladie choisie dans le groupe constitué des affections des voies respiratoires, des affections pulmonaires, des troubles gastro-intestinaux, des maladies telles que les maladies inflammatoires des articulations, de la peau ou des yeux, des maladies cancéreuses et des maladies du système nerveux périphérique ou central.

17. Combinaison de médicaments selon la revendication 16 pour le traitement d'une maladie choisie parmi la BPCO, la sinusite chronique, l'asthme, la maladie de Crohn, la fibrose pulmonaire idiopathique et la colite ulcéreuse.
